(19) Europäisches Patentamt European Patent Office Office européen des brevets

(11) **EP 1 007 653 B1**

(12) **EUROPEAN PATENT SPECIFICATION**

(45) Date of publication and mention
of the grant of the patent:
**29.08.2007 Bulletin 2007/35**

(51) Int Cl.:
*C12N 15/09* (2006.01)   *C07K 14/00* (2006.01)
*C12Q 1/00* (2006.01)   *G01N 33/543* (2006.01)

(21) Application number: **98940391.0**

(22) Date of filing: **01.09.1998**

(86) International application number:
**PCT/GB1998/002565**

(87) International publication number:
**WO 1999/011774 (11.03.1999 Gazette 1999/10)**

(54) **DETECTION METHODS COMPRISING THE USE OF POLYPEPTIDES COMPRISING A COILED-COIL AND AN ADDITION SITE**

DETEKTIONSVERFAHREN UMFASSEND DIE VERWENDUNG VON POLYPEPTIDEN MIT EINEM COILED-COIL UND EINER ADDITIONSSTELLE

PROCEDES DE DETECTION COMPRENANT L'UTILISATION DE POLYPEPTIDES A STRUCTURE BISPIRALEE COMPORTANT UN SITE D'ADDITION

(84) Designated Contracting States:
**AT BE CH CY DE DK ES FI FR GB GR IE IT LI LU MC NL PT SE**

(30) Priority: **30.08.1997 GB 9718358**

(43) Date of publication of application:
**14.06.2000 Bulletin 2000/24**

(73) Proprietor: **Cyclacel Limited**
**London SW1Y 4RB (GB)**

(72) Inventors:
• **COLYER, John**
**Bardsey LS17 9AN (GB)**
• **LIGHTOWLER, Joanne**
**York, YO24 2HS (GB)**
• **WOOLFSON, Derek,**
**Univer. of Sussex**
**Design &Drug Dev. ,**
**Falmer BN19 9QG (GB)**

(74) Representative: **Maschio, Antonio et al**
**D Young & Co**
**120 Holborn**
**London EC1N 2DY (GB)**

(56) References cited:
**WO-A-92/00388**   **WO-A-93/15210**
**WO-A-97/00267**   **WO-A-97/12988**
**WO-A-97/41424**

• **LUPAS, A.: "Coiled coils: new structures and new functions" TIBS, vol. 21, October 1996, pages 375-382, XP002089892**

<110> Fluorescience Limited

<120> Chemical modification

<130> Application number: pct/gb98/02565

<140>
<141>

<160> 9

<170> PatentIn Ver. 2.0

<210> 1
<211> 36

<212> PRT
<213> Artificial Sequence

<220>
<223> Description of Artificial Sequence:Synthetic Peptide

<400> 1

```
Arg Met Lys Gln Leu Glu Asp Lys Val Glu Glu Leu Leu Ser Lys Thr
 1               5                  10                  15

Tyr His Leu Glu Asn Glu Val Ala Cys Leu Lys Lys Leu Val Gly Glu
            20                  25                  30

Arg Ala Ala Lys
            35
```

<210> 2
<211> 36
<212> PRT
<213> Artificial Sequence

<220>
<223> Description of Artificial Sequence:Synthetic Peptide

<400> 2

```
Arg Met Lys Gln Leu Glu Asp Gln Val Arg Arg Leu Arg Arg Lys Ser
 1               5                  10                  15

Tyr His Leu Glu Asn Glu Val Ala Cys Leu Lys Lys Leu Val Gly Glu
            20                  25                  30

Arg Ala Ala Lys
            35
```

<210> 3
<211> 36
<212> PRT
<213> Artificial Sequence

<220>
<223> Description of Artificial Sequence:Synthetic Peptide

<400> 3

```
Arg Met Lys Gln Leu Glu Asp Gln Val Arg Arg Leu Arg Arg Lys Thr
  1               5                   10                  15

Tyr His Leu Glu Asn Glu Val Ala Cys Leu Lys Lys Leu Val Gly Glu
              20                  25                  30

Arg Ala Ala Lys
          35
```

<210> 4
<211> 34
<212> PRT
<213> Artificial Sequence

<220>
<223> Description of Artificial Sequence:Synthetic Peptide

<400> 4

```
His Met Lys Gln Leu Glu Asp Lys Val Glu Glu Leu Leu Ser Lys Asn
  1               5                   10                  15

Tyr Cys Leu Glu Asn Glu Val Arg Arg Leu Arg Arg Ala Ser Phe Ser
              20                  25                  30

Leu Gln
```

<210> 5
<211> 35
<212> PRT
<213> Artificial Sequence
<220>
<223> Description of Artificial Sequence:Synthetic Peptide

<400> 5

```
Arg Arg Ile Arg Arg Ala Ser Ile Asp Lys Val Glu Glu Leu Lys Ser
  1               5                   10                  15

Lys Asn Tyr Cys Leu Glu Asn Glu Val Ala Arg Leu Lys Lys Leu Val
              20                  25                  30

Gly Glu Arg
          35
```

<210> 6
<211> 35
<212> PRT
<213> Artificial Sequence

<220>
<223> Description of Artificial Sequence:Synthetic Peptide

<400> 6

```
Ile Ala Ala Leu Glu Gln Lys Ile Ala Ala Leu Ser Ala Val Ser Ser
1               5                  10                  15

Asp Leu Gly Thr Val Leu Lys Cys Leu Gln Gln Lys Ile Ala Ala Leu
                20                  25                  30

Glu Gln Lys
            35
```

<210> 7
<211> 36
<212> PRT
<213> Artificial Sequence

<220>
<223> Description of Artificial Sequence:Synthetic Peptide

<400> 7

```
Arg Met Lys Gln Leu Glu Asp Lys Val Glu Glu Leu Leu Ser Lys Asn
1               5                  10                  15

Tyr Ser Leu Glu Asn Glu Val Ala Cys Leu Lys Lys Leu Val Gly Glu
                20                  25                  30

Leu Glu Gln Lys
            35
```

<210> 8
<211> 36
<212> PRT
<213> Artificial Sequence

<220>
<223> Description of Artificial Sequence:Synthetic Peptide

<400> 8

```
Arg Met Lys Gln Leu Glu Asp Lys Val Glu Glu Leu Leu Ser Lys Asn
 1               5               10                  15

Tyr Thr Leu Glu Asn Glu Val Ala Cys Leu Lys Lys Leu Val Gly Glu
                20              25                  30

Leu Glu Gln Lys
            35
```

<210> 9
<211> 35
<212> PRT
<213> Artificial Sequence

<220>
<223> Description of Artificial Sequence:Synthetic Peptide

<400> 9

```
Leu Tyr Asn Leu Lys Phe Ser Ile Ile Cys Leu Lys Asn Gln His Lys
 1               5               10                  15

Glu Leu Arg Lys Arg Ile Glu Asn Leu Gly Gly Lys Phe Glu Val Lys
                20              25                  30

Ile Ser Glu
            35
```

**Description**

<u>FIELD</u>

This invention relates to the use of a polypeptide reporter molecule for monitoring the interaction of peptides as a function of the addition or subtraction of a chemical moiety to one of the reporter molecule by a protein modifying enzyme; and/or chemical modification resulting in the dissociation of reporter molecules that would naturally associate.

<u>BACKGROUND</u>

The post-translational modification of proteins has been known for over 40 years and since then has become a ubiquitous feature of protein structure. The addition of biochemical groups to translated polypeptides has wide-ranging effects on protein stability, protein secondary/tertiary structure, enzyme activity and in more general terms on the regulated homeostasis of cells. Such additions include, but are not limited to, phosphorylation, glycosylation, ADP-ribosylation and ubiquitination.

Phosphorylation is a well-studied example of a post-translational modification of protein. There are many cases in which polypeptides form higher order tertiary structures with like polypeptides (homo-oligomers) or with unalike polypeptides (hetero-oligomers). In the simplest scenario, two identical polypeptides associate to form an active homodimer. An example of this type of association is the natural association of myosin II molecules in the assembly of myosin into filaments.

The dimerization of myosin II monomers is the initial step in seeding myosin filaments. The initial dimerization is regulated by phosphorylation the effect of which is to induce a conformational change in myosin II secondary structure resulting in the folded 10S monomer subunit extending to a 6S molecule. This active molecule is able to dimerize and subsequently to form filaments. The involvement of phosphorylation of myosin II in this priming event is somewhat controversial. Although in higher eukaryotes the conformational change is dependant on phosphorylation, in *Ancanthoamoeba,* a lower eukaryote, the post-translational addition of phosphate is not required to effect the initial dimerization step. It is of note that the dimerization domains in myosin II of higher eukaryotes contain the sites for phosphorylation and it is probable that phosphorylation in this region is responsible for enabling myosin II to dimerize and subsequently form filaments. In *Dictyostelium* this situation is reversed in that the phosphorylation sites are outside the dimerization domain and phosphorylation at these sites is required to effect the disassembly of myosin filaments. In contrast to both these examples, *Acanthoamoeba* myosin II is phosphorylated in the dimerization domain but this modification is not necessary to enable myosin II monomers to dimerize in this species.

By far the most frequent example of post-translational modification is the addition of phosphate to polypeptides by specific enzymes known as protein kinases. These enzymes have been identified as important regulators of the state of phosphorylation of target proteins and have been implicated as major players in regulating cellular physiology. For example, the cell-division-cycle of the eukaryotic cell is primarily regulated by the state of phosphorylation of specific proteins the functional state of which is determined by whether or not the protein is phosphorylated. This is determined by the relative activity of protein kinases which add phosphate and protein phosphatases which remove the phosphate moiety from these proteins. Clearly dysfunction of either the kinases or phosphatases may lead to a diseased state. This is best exemplified by the uncontrolled cellular division shown by tumor cells. The regulatory pathway is composed of a large number of genes that interact *in vivo* to regulate the phosphorylation cascade that ultimately determines if a cell is to divide or arrest cell division.

Currently there are several approaches to analysing the state of modification of target proteins *in vivo:*

1. *In vivo* incorporation of labeled (for example, radiolabeled) moieties (e.g., phosphate, ubiquitin or ADP-ribosyls, which are added to target proteins.

2. Back-labeling. The incorporation of a labeled moiety into a protein *in vitro* to estimate the state of modification *in vivo.*

3. The use of cell-membrane-permeable protein-modifying enzyme inhibitors (*e.g.*, Wortmannin, staurosporine) to block modification of target proteins and comparable inhibitors of the enzymes involved in other forms of protein modification (above) .

4. Western blotting, of either 1- or 2-dimensional gels bearing test protein samples, in which modification is detected using antibodies specific for modified forms of target proteins.

5. The exploitation of eukaryotic microbial systems to identify mutations in protein-modifying enzymes.

These strategies have certain limitations. Monitoring states of modification by pulse or steady-state labelling is merely a descriptive strategy to show which proteins are modified when samples are separated by gel electrophoresis and visualized by autoradiography. This is unsatisfactory, due to the inability to identify many of the proteins that are modified. A degree of specificity is afforded to this technique if it is combined with immunoprecipitation; however, this is of course limited by the availability of antibodies to target proteins. Moreover, only highly-expressed proteins are readily detectable using this technique, which may fail to identify many low-abundance proteins, which are potentially important regulators of cellular functions.

The use of enzyme inhibitors to block activity is also problematic. For example, very few kinase inhibitors have adequate specificity to allow for the unequivocal correlation of a given kinase with a specific kinase reaction. Indeed, many inhibitors have a broad inhibitory range. For example, staurosporine is a potent inhibitor of phospholipid/$Ca^{+2}$ dependant kinases. Wortmannin is some what more specific, being limited to the phosphatidylinositol-3 kinase family. This is clearly unsatisfactory because more than one biochemical pathway may be affected during treatment making the assignment of the effects almost impossible.

Finally, yeast *(Saccharomyces cervisiae* and *Schizosaccharomyces pombe)* has been exploited as a model organism for the identification of gene function using recessive mutations. It is through research on the effects of these mutations that the functional specificities of many protein-modifying enzymes have been elucidated. However, these molecular genetic techniques are not easily transferable to higher eukaryotes, which are diploid and therefore not as genetically tractable as these lower eukaryotes.

An example of heterodimer association is described in patent application number WO92/00388. It describes an adenosine 3: 5 cyclic monophosphate (cAMP) dependent protein kinase which is a four-subunit enzyme being composed of two catalytic polypeptides (C) and two regulatory polypeptides (R). In nature the polypeptides associate in a stoichiometry of $R_2C_2$. In the absence of cAMP the R and C subunits associate and the enzyme complex is inactive. In the presence of cAMP the R subunit functions as a ligand for cAMP resulting in dissociation of the complex and the release of active protein kinase. The invention described in WO92/00388 exploits this association by adding fluorochromes to the R and C subunits.

The polypeptides are labeled (or 'tagged') with fluorophores having different excitation/emission wavelengths. The excitation and emission of one such fluorophore effects a second excitation/emission event in the second fluorophore. By monitoring the fluorescence emission of each fluorophore, which reflects the presence or absence of fluorescence energy transfer between the two, it is possible to derive the level of association between the R and C subunits as a function of cAMP concentration. Therefore, the natural affinity of the C subunit for the R subunit has been exploited to monitor the concentration of a specific metabolite, namely cAMP.

The prior art teaches that intact, fluorophore-labeled proteins can function as reporter molecules for monitoring the formation of multi-subunit complexes from protein monomers; however, in each case, the technique relies on the natural ability of the protein monomers to associate.

Tsien et al. (WO97/28261) teach that fluorescent proteins having the proper emission and excitation spectra that are brought into physically close proximity with one another can exhibit fluorescence resonance energy transfer ("FRET"). The invention of WO97/28261 takes advantage of that discovery to provide tandem fluorescent protein constructs in which two fluorescent protein moieties capable of exhibiting FRET are coupled through a linker to form a tandem construct. In the assays of the Tsien application, protease activity is monitored using FRET to determine the distance between fluorophores controlled by a peptide linker and subsequent hydrolysis thereof. Other applications rely on a change in the intrinsic fluorescence of the protein as in the kinase assays of WO98/06737.

The present invention instead encompasses the use of FRET to monitor the association of polypeptides, as described herein, which are labeled with fluorescent moieties (protein and chemical); in the method described, FRET indicates the proximity of two labeled polypeptide binding partners, which labeled partners associate either in the presence or absence of a given post-translational modification to an engineered site which has been introduced into at least one of the partners, but not into the fluorophore, reflecting the modification state of one or both of the binding partners and, consequently, the level of activity of a protein-modifying enzyme.

There is a need in the art for efficient means of monitoring and/or modulating post-translational protein modification. Further, there is a need to develop a technique whereby the addition/removal of a modifying group can be monitored continuously during real time to provide a dynamic assay system that also has the ability to resolve spatial information.

## SUMMARY

According to a first aspect of the present invention, we provide a method of monitoring the activity of an enzyme comprising the steps of: (a) providing a polypeptide comprising a coiled-coil and introducing a modification site into the coiled-coil of the polypeptide, the modification site being sufficient for the addition of a moiety at the site selected from the group consisting of: a phosphate ($PO_4$), ubiquitin, glycosyl or ADP-ribosyl moiety, such that the polypeptide comprising the introduced modification site is capable of modification dependent binding to a binding partner via said coiled-coil in which: (i) addition of a moiety at the site permits association of the modified polypeptide with the binding partner, or (ii) addition of a moiety at the site prevents association of the modified polypeptide with the binding partner; and (b) detecting association between the polypeptide and the binding partner as an indication of enzyme activity.

The invention makes use of an isolated polypeptide, or a fragment thereof, comprising at least one engineered site sufficient for the addition of at least one chemical or biological "moiety", i.e., a group, that is one of a phosphate, ubiquitin, glycosyl or ADP-ribosyl moiety, wherein the polypeptide binds to at least one binding partner in at least one of the following manners: phosphorylation-, ubiquitination-, glycosylation- or ADP-ribosylation-dependent manner.

Reference herein to the term "isolated polypeptide" comprises reference to a polypeptide that forms a coiled-coil structure. Coiled-coil structures are well known to those skilled in the art but a description is also provided hereinafter.

As used herein, the term "isolated polypeptide" refers to a synthetic polypeptide containing or consisting of at least one coiled-coil or a natural polypeptide comprising at least one coiled-coil structure, so long as the polypeptide has a binding partner and so long as binding of the polypeptide to its binding partner is dependent upon the presence or absence of a "moiety" at an engineered site, which site is present in one or both of the isolated polypeptide and its binding partner.

The method may also use a synthetic polypeptide containing at least one coiled-coil and containing at least one amino acid already modified by a moiety that is one of a phosphate, ubiquitin, glycosyl or ADP-ribosyl moiety, wherein the synthetic polypeptide binds to a binding partner in a phosphorylation-, ubiquitination-, glycosylation- or ADP-ribosylation-dependent manner, such that the moiety may be removed by an enzyme.

According to the method, binding of an isolated or synthetic polypeptide and its binding partner(s) is dependent upon addition or removal of at least one moiety, which addition or removal may occur on one or both of the isolated polypeptide and its binding partner.

An "engineered site" suitable for addition or removal of a "moiety" is placed within an isolated polypeptide or binding partner thereof of the invention at a position such that formation of a dimer between the isolated polypeptide and its binding partner is dependent upon the presence or absence of the "moiety"; and preferably does not overlap with an amino acid which is part of a fluorescent tag.

Similarly, the amino acid that includes a "moiety" as described herein may be positioned anywhere within the synthetic polypeptide such that formation of a dimer between the synthetic polypeptide and its binding partner is dependent upon the presence or absence of the moiety.

As used herein, the term "binding partner" refers to a polypeptide or fragment thereof (a peptide) that binds to (associates with) a polypeptide comprising a coiled-coil. A binding partner usually will contain a coiled-coil and an engineered site, if these are required for binding, but does not necessarily have to contain these elements if they are not required for binding.

It is contemplated that the position at which an engineered site or an amino acid containing a moiety is to reside is initially determined by random placement of the site within the polypeptide or binding partner, followed by testing by methods described herein of the ability of the isolated or synthetic polypeptide and its binding partner to associate or not, depending upon the presence of absence of a moiety. A pair of binding partners, of which at least the isolated polypeptide comprises a site so placed and which is found to display modification-dependent association, is of use in the assays described here.

As used herein, the terms "polypeptide"and "peptide" refer to a polymer in which the monomers are amino acids and are joined together through peptide or disulfide bonds. "Polypeptide" refers to either a full-length naturally-occurring amino acid chain or a "fragment thereof" or "peptide", such as a selected region of the polypeptide that is of interest in a binding assay and for which a binding partner is known or determinable. "Fragment thereof" thus refers to an amino acid sequence that is a portion of a full-length polypeptide, between about 8 and about 500 amino acids in length, preferably about 8 to about 300, more preferably about 8 to about 200 amino acids, and even more preferably about 10 to about 50 or 100 amino acids in length. "Peptide" refers to a short amino acid sequence that is 10-40 amino acids long, preferably 10-35 amino acids. Additionally, unnatural amino acids, for example, β-alanine, phenyl glycine and homoarginine may be included. Commonly-encountered amino acids which are not gene-encoded may also be used. All of the amino acids used may be either the D- or L- optical isomer. The L-isomers are preferred. In addition, other peptidomimetics are also useful, e.g. in linker sequences of polypeptides (see Spatola, 1983, in Chemistry and Biochemistry of Amino Acids, Peptides and Proteins, Weinstein, ed., Marcel Dekker, New York, p. 267).

"Naturally-occurring" as used herein, as applied to a polypeptide or polynucleotide, refers to the fact that the polypeptide or polynucleotide can be found in nature and naturally contains a coiled-coil structure. One such example is a polypeptide or polynucleotide sequence that is present in an organism (including a virus) that can be isolated form a source in nature. Once the polypeptide is engineered as described herein it is no longer naturally ocurring but is derived from a naturally ocurring polypeptide.

"Polynucleotide" refers to a polymeric form of nucleotides of at least 10 bases in length and up to 1,000 bases or even more, either ribonucleotides or deoxyribonucleotides or a modified form of either type of nucleotide. The term includes single and double stranded forms of DNA.

As used herein, the term "associates" or "binds" refers to a polypeptide as described herein and its binding partner having a binding constant sufficiently strong to allow detection of binding by FRET or other detection means, which are in physical contact with each other and have a dissociation constant (Kd) of about 10iM or lower. The contact region may include all or parts of the two molecules. Therefore, the terms "substantially dissociated" and "dissociated" or "substantially unbound" or "unbound" refer to the absence or loss of contact between such regions, such that the binding constant is reduced by an amount which produces a discernable change in a signal compared to the bound state, including a total absence or loss of contact, such that the proteins are completely separated, as well as a partial absence or loss of contact, so that the body of the proteins are no longer in close proximity to each other but may still be tethered together or otherwise loosely attached, and thus have a dissociation constant greater than 10iM (Kd). In many cases, the Kd will be in the mM range. The terms "complex" and, particularly, "dimer", "multimer" and "oligomer"as used herein, refer to the polypeptide, peptide, protein, domain or subunit and its binding partner in the associated or bound state. More than one molecule of each of the two or more proteins may be present in a complex, dimer, multimer or oligomer.

As used herein, "post-translational modification" of a polypeptide refers to the addition or removal of a "moiety" as described herein and does not refer to other post-translational events which do not involve addition or removal of such a moiety as described herein, and thus does not include simple cleavage of the reporter molecule polypeptide backbone by hydrolysis of a peptide bond, but does include hydrolysis of an isopeptide bond (e.g., in the removal of ubiquitin).

In an assay as described here, post-translational modification is reversible, such that a repeating cycles of addition and removal of a modifying moiety may be observed, although such cycles may not occur in a living cell found in nature.

The term "site sufficient for the addition of " refers to an amino acid sequence which is recognized by (i.e., a recognition site for) a post-translational modifying enzyme, at which sequence modification (e.g., addition or removal of a phosphate, ubiquitin, glycosyl or ADP-ribosyl moiety) occurs. It is contemplated that a site comprises a small number of amino acids, typically from 2 to 10, less often up to 30 amino acids, and further that a site comprises fewer than the total number of amino acids present in the polypeptide.

The invention encompasses assays which measure the activity of a protein-modifying enzyme as indicated by the presence or absence of or the addition or removal of a chemical or biological moiety (e.g., addition or subtraction of a ubiquitin or ADP-ribosyl group), and does not encompass methods to detect post-translational cleavage of the reporter molecule polypeptide backbone.

As used interchangeably herein, the terms "moiety" and "group" refer to one of the post-translational added or removed groups referred to herein: i.e., one of a phosphate, ubiquitin, glycosyl or ADP-ribosyl moiety. A "fluorescent tag" or "fluorescent group" refers to either a fluorophore or a fluorescent protein or fluorescent fragment thereof.

"Fluorescent protein" refers to any protein which fluoresces when excited with appropriate electromagnetic radiation. This includes proteins whose amino acid sequences are either natural or engineered. A "fluorescent protein moiety" is a fluorescent protein or fluorescent fragment thereof . By the same token, the term "linker moiety" refers to the radical of a molecular linker that is coupled to both the donor and acceptor protein molecules, such as an amino acid sequence joining two engineered sites or two Coiled-coils or a disulfide bond between two polypeptides.

Preferably, addition of at least one of the following moieties: phosphate, ubiquitin, glycosyl or ADP-ribosyl moiety permits association of the corresponding phosphorylated-, ubiquitin-, glycosyl- or ADP-ribosyl-containing polypeptide with the binding partner.

Alternatively, it is preferred that addition of at least one of the following moieties: phosphate, ubiquitin, glycosyl or ADP-ribosyl moiety prevents association of the corresponding phosphorylated-, ubiquitin-, glycosyl- or ADP-ribosyl-containing polypeptide with the binding partner.

As used herein the term "prevents association" refers to the ability of at least one of the following: ubiquitin, glycosyl or ADP-ribosyl group to inhibit the association, as defined above, of at least two isolated polypeptides, an isolated polypeptide and a binding partner thereof or at least an isolated pair of polypeptides, as defined above, by at least 10%, preferably by 25-50%, highly preferably by 75-90% and, most preferably, by 95-100% relative the association observed in the absence of such a modification under the same experimental conditions.

It is additionally preferred that the isolated or synthetic polypeptide comprises at least one "contact site" which physically contacts or binds to said binding partner, and at least one of the contact sites of the polypeptide comprises either the engineered site sufficient for the addition of at least one of the following moieties: phosphate, ubiquitin, glycosyl or ADP-ribosyl moiety, or contains the amino acid that contains a moiety as defined herein.

Preferably, the polypeptide further comprises a detection means, the polypeptide comprising the detection means being a reporter molecule, the detection means ideally comprises a light emitting detection means, and the light emitting detection means ideally emits light of at least a fluorescent wavelength emission.

It is preferred that the light emitting detection means comprises two different fluorophores.

It is additionally preferred that the fluorophores comprise fluorescein and tetramethylrhodamine or another suitable pair.

Preferably, the polypeptide comprises a cysteine amino acid through which the light emitting means is attached via a covalent bond.

In another preferred embodiment, the light emitting detection means comprises two different fluorescent proteins.

It is preferred that the two different fluorescent proteins comprise green fluoresecent protein and red fluorescent protein.

It is additionally preferred that the two different fluorescent proteins comprise green fluorescent protein and blue fluorescent protein.

Preferably, the polypeptide comprises a coiled-coil containing a site sufficient for the addition of at least one of the following moieties: phosphate ($PO_4$), ubiquitin, glycosyl or ADP-ribosyl moiety.

It is preferred that the polypeptide associates via the coiled-coil with another coiled-coil containing polypeptide and more preferred that the polypeptide contains two coiled-coils and is therefore capable of self association via the two coiled-coils.

In another preferred embodiment, addition of a least one of the following moieties: phosphate, ubiquitin, glycosyl or ADP-ribosyl moiety permits association of the corresponding phosphate-, ubiquitin-, glycosyl- or ADP-ribosyl-containing polypeptide with another coiled-coil containing polypeptide to form a dimer.

In another preferred embodiment, addition of a least one of the following moieties: phosphate, ubiquitin, glycosyl or ADP-ribosyl moiety prevents association of the corresponding phosphate-, ubiquitin-, glycosyl- or ADP-ribosyl-containing polypeptide with another coiled-coil containing polypeptide to form a dimer.

We further describe a kit for determining the enzyme activity of a selected kinase, phosphatase, UDP-N-Acetylglucosamine-Dolichyl-phosphate-N-acetylsglucosamine phosphotransferase, O-GlcNAc transferase, ubiquitin activating enzyme E1, ubiquitin conjugating enzyme E2, ubiquitin protein ligase E3, poly (ADP-ribose) polymerase or NAD:Arginine ADP ribosyltransferase in real time comprising an isolated polypeptide, or a fragment thereof, comprising an engineered site sufficient for the addition of at least one of the following moieties: phosphate ($PO_4$), ubiquitin, glycosyl or ADP-ribosyl moiety, wherein the polypeptide binds to at least one binding partner in at least one of the following manners: phosphorylation-, ubiquitination-, glycosylation- or ADP-ribosylation-dependent manner, and packaging materials therefore.

It is additionally preferred that the polypeptide assembles to form a coiled-coil structure and which has in its assembly region a site sufficient for at least one of the following: phosphorylation, ubiquitination, glycosylation or ADP-ribosylation.

We further describe the use of an isolated polypeptide, or a fragment thereof, comprising an engineered site sufficient for the addition of at least one of the following moieties: phosphate ($PO_4$), ubiquitin, glycosyl or ADP-ribosyl moiety, wherein the polypeptide binds to at least one binding partner in at least one of the following manners: phosphorylation-, ubiquitination-, glycosylation- or ADP-ribosylation-dependent manner to monitor the addition of said phosphate, ubiquitin, glycosyl or ADP-ribosyl moiety to said polypeptide.

We describe a method to monitor the activity of an enzyme comprising the step of monitoring the addition of at least one of the following moieties: phosphate, ubiquitin, glycosyl or ADP-ribosyl moiety to at least one polypeptide, wherein the polypeptide is an isolated polypeptide, or a fragment thereof, comprising an engineered site sufficient for the addition of at least one of the following moieties: phosphate ($PO_4$), ubiquitin, glycosyl or ADP-ribosyl moiety, wherein the polypeptide binds to at least one binding partner in at least one of the following manners: phosphorylation-, ubiquitination-, glycosylation- or ADP-ribosylation-dependent manner and wherein the polypeptide further comprises a detection means, the polypeptide comprising the detection means being a reporter molecule.

We further describe a method to monitor the activity of an enzyme comprising the step of monitoring the removal of at least one of a phosphate, ubiquitin, glycosyl or ADP-ribosyl moiety from at least one polypeptide, wherein the polypeptide is an isolated polypeptide, or a fragment thereof, comprising an engineered site sufficient for the addition of at least one of the following moieties: phosphate ($PO_4$), ubiquitin, glycosyl or ADP-ribosyl moiety, wherein the polypeptide binds to at least one binding partner in at least one of the following manners: phosphorylation-, ubiquitination-, glycosylation- or ADP-ribosylation-dependent manner and wherein the polypeptide further comprises a detection means, the polypeptide comprising the detection means being a reporter molecule.

Preferably, the methods further comprise, prior to the step of monitoring, the step of mixing in an appropriate buffer and an appropriate polypeptide concentration, the polypeptide and its binding partner labeled with an appropriate combination of fluorescence emitting means to monitor association between the polypeptide and its binding partner.

As used herein, the term "appropriate buffer" refers to a medium which permits activity of the protein-modifying enzyme used in an assay described here, and is typically a low-ionic-strength buffer or other biocompatible solution (e.g., water, containing one or more of physiological salt, such as simple saline, and/or a weak buffer, such as Tris or phosphate, or others as described hereinbelow), a cell culture medium, of which many are known in the art, or a whole or fractionated cell lysate. An "appropriate buffer" permits dimerization of non-phosphorylated and/or non-ubiquitinated and/or non-ADP-ribosylated and/or non-glycosylated engineered sites on isolated polypeptides of the invention and, preferably, inhibits degradation and maintains biological activity or the reaction components. Inhibitors of degradation, such as protease inhibitors (e.g., pepstatin, leupeptin, etc.) and nuclease inhibitors (e.g., DEPC) are well known in the art. Lastly, an appropriate buffer may comprise a stabilizing substance such as glycerol, sucrose or polyethylene glycol.

As used herein, the term "appropriate reporter molecule concentration" refers to an amount of labeled reporter molecule (that is, a labeled polypeptide) which emits a signal within the detection limits of a measuring device used in an assay as described. Such an amount is great enough to permit detection of a signal, yet small enough that a change in signal emission is detectable (e.g., such that a signal is below the upper limit of the device).

As used herein with regard to fluorescent labels, the term "appropriate combination" refers to a choice of reporter labels such that the emission wavelength spectrum of one (the "donor" moiety) is within the excitation wavelength spectrum of the other (the "acceptor" moiety).

It is preferred that the methods further comprise incubating the polypeptide and said binding partner with an appropriate modifying enzyme and measuring the change in energy transfer between the polypeptide and its binding partner.

Preferably, the measuring is performed by fluorescent resonance energy transfer (FRET).

Preferably, the method further comprises exciting the reporter molecules and monitoring fluorescence emission.

It is preferred that the modifying enzyme is selected from the group that includes a kinase, a phosphatase, a UDP-N-Acetylglucosamine-Dolichyl-phosphate-N-acetylsglucosamine phosphotransferase, an O-GlcNAc transferase, a ubiquitin activating enzyme E1, a ubiquitin conjugating enzyme E2, a ubiquitin protein ligase E3, poly (ADP-ribose) polymerase and an NAD:Arginine ADP ribosyltransferase.

In a preferred embodiment, the method further comprises the addition to the buffer of an agent which modulates the activity of the modifying enzyme.

As used herein with regard to a biological or chemical agent, the term "modulate" refers to enhancing or inhibiting the activity of a protein-modifying enzyme in an assay as described; such modulation may be direct (e.g. including, but not limited to, cleavage of- or competitive binding of another substance to the enzyme) or indirect (e.g. by blocking the initial production or, if required, activation of the modifying enzyme).

"Modulation" refers to the capacity to either increase or decease a measurable functional property of biological activity or process (e.g., enzyme activity or receptor binding) by at least 10%, 15%, 20%, 25%, 50%, 100% or more; such increase or decrease may be contingent on the occurrence of a specific event, such as activation of a signal transduction pathway, and/or may be manifest only in particular cell types.

The term "modulator" refers to a chemical compound (naturally occurring or non-naturally occurring), such as a biological macromolecule (e.g., nucleic acid, protein, non-peptide, or organic molecule), or an extract made from biological materials such as bacteria, plants, fungi, or animal (particularly mammalian) cells or tissues, or even an inorganic element or molecule. Modulators are evaluated for potential activity as inhibitors or activators (directly or indirectly) of a biological process or processes (e.g., agonist, partial antagonist, partial agonist, antagonist, antineoplastic agents, cytotoxic agents, inhibitors of neoplastic transformation or cell proliferation, cell proliferation-promoting agents, and the like) by inclusion in screening assays described herein. The activities (or activity) of a modulator may be known, unknown or partially-known. Such modulators can be screened using the methods described herein.

The term "candidate modulator" refers to a compound to be tested by one or more screening method(s) described here as a putative modulator. Usually, various predetermined concentrations are used for screening such as 0.01 iM, 0.1iM, 1.0 iM, and 10.0 iM, as described more fully hereinbelow. Test compound controls can include the measurement of a signal in the absence of the test compound or comparison to a compound known to modulate the target.

Preferably, the method further comprises the addition to the buffer of an agent which modulates fluorescence emission of the reporter molecules.

We further describe a kit comprising a fluorophore-labeled polypeptide, wherein the polypeptide is an isolated polypeptide, or a fragment thereof, comprising an engineered site sufficient for the addition of at least one of the following moieties: phosphate ($PO_4$), ubiquitin, glycosyl or ADP-ribosyl moiety, wherein the polypeptide binds to at least one binding partner in at least one of the following manners: phosphorylation-, ubiquitination-, glycosylation- or ADP-ribosylation-dependent manner, an enzyme selected from the group that includes a kinase, a phosphatase, a UDP-N-Acetylglucosamine-Dolichyl-phosphate-N-acetylsglucosamine phosphotransferase, an O-GlcNAc transferase, a ubiquitin activating enzyme E1, a ubiquitin conjugating enzyme E2, a ubiquitin protein ligase E3, a poly (ADP-ribose) polymerase, an NAD:Arginine ADP ribosyltransferase and packaging therefor.

It is preferred that the kit further comprises a reaction buffer for the enzyme.

It is additionally preferred that the kit further comprises a substrate for the enzyme.

Preferably, the substrate is selected from the group that includes MgATP, cAMP, ubiquitin, nicotinamide adenine dinucleotide ($NAD^+$), uridine-diphosphate-N-acetylglucosamine-dolichyl-phosphate (UDP-N-acetylglucosamine-dolichyl-phosphate) and UDP-N-acetylglucosamine.

It is contemplated that at least a part of a substrate of an enzyme of use in an assay described here is transferred to an engineered site on an isolated polypeptide. As used herein, the term "at least a part of a substrate" refers to a portion (e.g., a fragment of an amino acid sequence, a moiety or a group, as defined above) which comprises less than the whole of the substrate for the enzyme, the transfer of which portion to an engineered site on an isolated polypeptide, both as defined above, is catalyzed by the enzyme.

It is preferred that the kit further comprises a cofactor for the enzyme.

We describe an isolated pair of polypeptides which associate to form a dimer in at least one of the following manners: phosphorylation-, ubiquitination-, glycosylation- or ADP-ribosylation-dependent manner, the pair comprising a first polypeptide comprising at least one binding domain, at least one engineered phosphorylation, ubiquitination, glycosylation or ADP-ribosylation site, and a detection means whereby the addition/removal of phosphate, ubiquitin, glycosyl or ADP-ribosyl moiety to the corresponding phosphorylation, ubiquitination, glycosylation or ADP-ribosylation site is detectable via binding of the binding domain with a binding partner; and a second polypeptide which is a binding partner of the first polypeptide, wherein dimer formation is detectable via the detection means.

There is provided, according to a second aspect of the present invention, a method of screening for a candidate modulator of enzymatic activity of a kinase, a phosphatase, a UDP-N-Acetylglucosamine-Dolichyl-phosphate-N-acetylsglucosamine phosphotransferase, an O-GlcNAc transferase, a ubiquitin activating enzyme E1, a ubiquitin conjugating enzyme E2, a ubiquitin protein ligase E3, a poly (ADP-ribose) polymerase and an NAD:Arginine ADP ribosyltransferase, the method comprising: (a) providing a polypeptide comprising a coiled-coil and introducing a modification site into the coiled-coil of the polypeptide, the modification site being sufficient for the addition of a moiety at the site selected from the group consisting of: a phosphate ($PO_4$), ubiquitin, glycosyl or ADP-ribosyl moiety, such that the polypeptide comprising the introduced modification site is capable of modification dependent binding to a binding partner via said coiled-coil in which (i) addition of a moiety at the site permits association of the modified polypeptide with the binding partner, or (ii) addition of a moiety at the site prevents association of the modified polypeptide with the binding partner; (b) mixing in an appropriate buffer and an appropriate amount of the polypeptide together with the binding partner; in which each of the polypeptide and the binding partner is suitably labelled with detection means for monitoring association/disassociation between same; and a sample of material whose enzymatic activity is to be tested; and (c) monitoring the addition or removal of at least one of the following groups: phosphate, ubiquitin, glycosyl or ADP-ribosyl group to the polypeptide, in which the addition or removal of the phosphate, ubiquitin, glycosyl or ADP-ribosyl group is indicative of modulation by the candidate modulator of the enzymatic activity.

As used herein, the term "sample" refers to a collection of inorganic, organic or biochemical molecules which is either found in nature (e.g., in a biological- or other specimen) or in an artificially-constructed grouping, such as agents which might be found and/or mixed in a laboratory. Such a sample may be either heterogeneous or homogeneous.

As used herein, the interchangeable terms "biological specimen" and "biological sample" refer to a whole organism or a subset of its tissues, cells or component parts (e.g. body fluids, including but not limited to blood, mucus, lymphatic fluid, synovial fluid, cerebrospinal fluid, saliva, amniotic fluid, amniotic cord blood, urine, vaginal fluid and semen). "Biological sample" further refers to a homogenate, lysate or extract prepared from a whole organism or a subset of its tissues, cells or component parts, or a fraction or portion thereof. Lastly, "biological sample" refers to a medium, such as a nutrient broth or gel in which an organism has been propagated, which contains cellular components, such as proteins or nucleic acid molecules.

As used herein, the term "organism" refers to all cellular life-forms, such as prokaryotes and eukaryotes, as well as non-cellular, nucleic acid-containing entities, such as bacteriophage and viruses.

We provide, according to a third aspect of the present invention, a method to monitor the activity of an enzyme comprising the step of monitoring the removal of at least one of the following moieties: phosphate ubiquitin, glycosyl or ADP-ribosyl moiety from a synthetic polypeptide comprising a coiled-coil, in which the synthetic polypeptide is synthesized such that it comprises at least one amino acid in the coiled-coil which is covalently linked to at least one of the following groups: phosphate, ubiquitin, glycosyl or ADP-ribosyl group, in which the synthetic polypeptide has at least one binding partner to which it is capable of finding via the coiled-coil, in which the binding partner : (i) associates with the synthetic polypeptide in the absence of the phosphate, ubiquitin, glycosyl or ADP-ribosyl moiety, or (ii) dissociates from the synthetic polypeptide in the absence of the phosphate, ubiquitin, glycosyl or ADP-ribosyl moiety; and in which the synthetic polypeptide and the binding partner therefor each comprise at least one light emitting detection means.

It is highly preferred that a method of the methods described above comprises real-time observation of association of an isolated polypeptide and its binding partner or of an isolated pair of polypeptides.

As used herein in reference to monitoring, measurements or observations in assays described here, the term "real-time" refers to that which is performed contemporaneously with the monitored, measured or observed events and which yields a result of the monitoring, measurement or observation to one who performs it simultaneously, or effectively so, with the occurrence of a monitored, measured or observed event. Thus, a "real time" assay or measurement contains not only the measured and quantitated result, such as fluorescence, but expresses this in real time, that is, in hours, minutes, seconds, milliseconds, nanoseconds, picoseconds, etc. Shorter times exceed the instrumentation capability; further, resolution is also limited by the folding and binding kinetics of polypeptides.

Further features and advantages of the invention will become more fully apparent in the following description of the embodiments and drawings thereof, and from the claims.

BRIEF DESCRIPTIONS OF THE DRAWINGS

Figure 1 diagrams the structure of single and dimerized coiled-coil peptide motifs.

Figure 2 presents a schematic overview of FRET in an assay.

Figure 3 presents monomer:excimer fluorescence.

Figure 4 presents the circular dichroism (CD) spectra of Zip3 and Zip3P at 20˚C. Ellipticity in mdeg is shown on the y-axis, while wavelength in nm is shown on the x-axis. Closed diamonds, Zip3; closed squares, Zip4.

Figure 5 shows thermal melting of Zip3 and Zip3P. Ellipticity at 222nm is shown in mdeg on the y-axis, while temperature in ˚C is shown on the x-axis.

Figure 6 shows the effect of the addition of 0.48 iM Zip3R to 0.08 iM Zip3F. Fluorescence at 516 nm is shown on the y-axis; time in seconds is charted on the x-axis.

Figure 7 shows the effect of the addition of 0.48 iM Zip3PR to 0.08 iM Zip3PF. Fluorescence at 516 nm is shown on the y-axis; time in seconds is charted on the x-axis.

Figure 8 presents the phosphorylation of Zip4S and Zip4T by PKA (diamonds. Zip4S; +'s, Zip4T; x's, PL919Y). Counts per minute are indicated on the y-axis; time in minutes is shown on the x-axis.

Figure 9 shows data demonstrating the specificity of Zip4S phosphorylation. 1: Autocamtide II phosphorylated by CaMKII in the presence of $Ca^{2+}$ and calmodulin. 2: Zip4S phosphorylated by PKA. 3: Zip4S phosphorylated by CaMKII in the presence of Calmodulin and $Ca^{2+}$. 4: Autocamtide II phosphorylated by CaMKII in the presence of CaM, but absence of $Ca^{2+}$.

Figure 10 shows thermal denaturation of Zip4S and Zip4T. Molar ellipticity is at 222 nm is indicated on the y-axis; temperature in ˚C is indicated on the x-axis.

Figure 11 presents thermal denaturation of Zip4S and Zip4SP. Molar ellipticity is indicated on the y-axis and temperature in ˚C on the x-axis.

Figure 12 presents CD spectra of Zip4S and Zip4SP at 1˚C. Molar ellipticity is charted on the y-axis and wavelength in nm is shown on the x-axis (closed diamonds, Zip4S; closed squares, Zip4SP).

Figure 13 presents FRET between Zip4SF and Zip4SR, reversed by phosphorylation of the polypeptide reporter group (fluorescence on y-axis; emission ë on x-axis).

Figure 14 presents FRET between Zip4SF and Zip4SR, lost upon phosphorylation of polypeptides by PKA. Time course of phosphorylation measured in real time. Fluorescence at 516 nm on y-axis (excitation ë, 450 nm), time in seconds on x-axis. Arrow (filled head): addition of Zip4SR. Arrow (open head): addition of PKA to Zip4SF.

DESCRIPTION

The invention is based upon the discovery that a polypeptide comprising a coiled-coil associates with a binding partner to form oligomers or dissociates from a binding partner in a manner that is dependent upon the presence or absence of a "moiety", as described herein, and that is detectable and measurable in a highly sensitive manner that may be in real time;

Cooled-Coils

The coiled-coil domain is structurally conserved among many proteins that interact to form homo- or heterodimeric oligomers. The leucine zipper provides an example of one such protein structural motif. It is found in, among other examples, a nuclear protein that functions as a transcriptional activator of a family of genes involved in the General Control of Nitrogen (GCN4) metabolism in *S. cerevisiae.* The protein is able to dimerize and bind promoter sequences containing the recognition sequence for GCN4, thereby activating transcription in times of nitrogen deprivation.

Coiled-coils are á-helical oligomers or bundles with between 1 and 5 polypeptide strands with the following characteristics: (i) a sequence hallmark of a predominance of hydrophobic residues (in particular alanine, isoleucine, leucine, methionine or valine) spaced 3 and 4 residues apart in the primary sequence which is repeated three or more times in near or exact succession (canonical heptad coiled-coil repeat, abbreviated to $(3,4)_n$ where n=3 or greater). The hydrophobic residues are present at the 'a' and 'd' positions within a heptad when the amino acids are identified as positions a,b,c,d,e,f and g by order of sequence. In addition, spacing of hydrophobic residues in patterns of 3,4,4 and 3,4,3 (hendecad repeat) have recently been reported (Hicks et al., 1997, Folding and Design, 2: 149-158) and are compatible with the coiled-coil structure. (ii) In structural terms coiled-coil helical bundles have between 2 and 5 helices which are offset at roughly 20˚ to adjacent strands with the hydrophobic sidechains interdigitating in the interface between helices in what is termed the "knobs into holes" packing (Crick, 1953, Acta. Crystallogr., 6: 689-697). Natural and non-natural coiled-coils can have parallel and/or antiparallel helices. Both homotypic (multiple strands of identical sequence) and heterotypic bundles have been described.

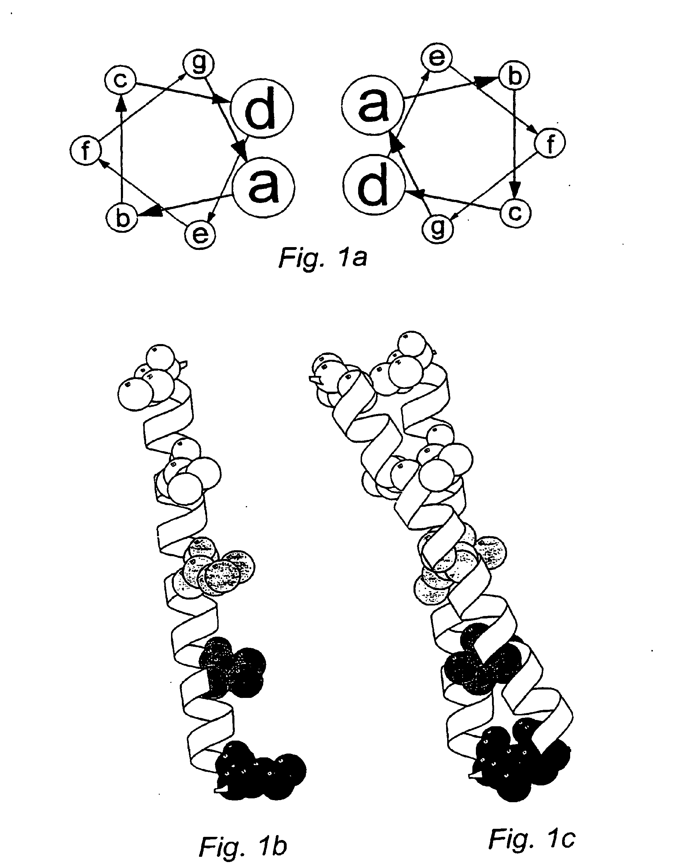

Leucine zipper sequences conform to the coiled- coil rules above and typically have leucine residues at the 'd' position of the canonical heptad repeat (Fig. 1A). As shown in Fig. 1B, these leucine residues represent a single face of the helix. Interdigitating with these leucine residues are valine residues. The combination of these residues forms a continuous hydrophobic face which associates with an equivalent region in an associating subunit (Fig. 1C). Alternatively the hydophobic face can be discontinuous due to interruptions in the heptad repeat sequence. This, however, does not interfere with the ability of these coiled-coils to interact. The stability of the dimer thus formed is conferred by the hydrophobic interactions between the leucine and valine residues and hydrogen bonds that form between residues present on the two interacting helices. Interestingly, the coiled-coil domain of GCN4 has been shown to dimerize as an isolated peptide (Gonzalez et al., 1996, Nature Structural Biology, 3: 1011-1018).

Examples of naturally-occurring coiled-coils are as follows:

Coiled-coil class and example:

fgabcdefgabcdefgabcdefgabcdefgabcdefgabcdeg

Parallel two-stranded

TMPA_RABIT, 10-279 (270)

tropomyosin

(J.BIOL.CHEM. 253, 1137-1148, 1978)

dystrophin      ILISLESEERGELERILADLEEENRNLQAEYDRLKQQHEHK

SWISS PROT:P11532 (HUMAN)

(Trends Biol. Sci., 20,133-135, 1995)

GCN4*      MKQLEDKVEELLSKNYHLENEVARLKKLVGER

GCN4_YEAST, 250-281 (32)

(Proc. Natl. Acad. Sci. U.S.A., 81, 6442-6446, 1982)

cFOS*      TDTLQAETDQLEDERSALQTEIANLLKEKEKLEFILAAH

FOS_HUMAN, 162-199 (39) (Proc. Natl. Acad. Sci.
U.S.A., 80: 3183-3187, 198)

cJUN*      IARLEEKVKTLKAQNSELASTANMLREQVAQLKQKVMNH

AP1_ HUMAN, 277-315 (39)

(Proc. Natl. Acad. Sci. U.S.A., 85: 9148-9152, 1988

antiparallel two-stranded

Seryl-tRNA synthetase, E. coli*      VDKLGALEERRKVLQVKTENLQAERNSRSKSIGQAKAR

SYS_ECOLI, 27-64 (38)      EPLRLEVNKLGEELDAAKAELDALQAEIRDIA

NUCLEIC ACIDS RES., 15, 1005-1017,1987

SYS_ECOLI, 69-100 (32)

Seryl-tRNA synthetase,

DLEALLALDREVQELKKRLQEVQTERNQVAKRV

Thermus thermophilus*      EALIARGKALGEEAKRLEEALREKEARLEALL

SYS_THERM, 26-58 (33)

(Science, 263: 1404-141)

SYS_THERM, 67-98 (32)

Transcript cleavage factor GreA*      LRGAEKLREELDFLKSvFRPEIIAAIAEAR

GREA_ECOLI, 8-37 (30)      AEYHAAREQQGFCEGRIKDIEAKLSN

(Nature, 373: 636-640, 1995)

(continued)

GREA_ECOLI, 46-71 (26)

Parallel three-stranded
GCN4 Zip mutant pll*
GCN4 Zip mutant pll*     MKQIEDKIEEILSKIYHIENEIARIKKLIGER
(Nature, 371: 80-83)

Antiparallel three-stranded
synthetic peptide coil-Ser*     EWEALEKKLAALESKLQALEKKLEALEHG
(Science, 259: 1288-1293)

Parallel four-stranded

GCN4 Zip mutant pLI*     MKQIEDKLEEILSKLYHIENELARIKKLLGER
(Nature, 371: 80-83)

Antiparallel four-stranded

Repressor of primer ROP*     QEKTALNMARFIRSQTLTLLEKLNE
ROP_ECOLI, 4-28 (25)     DEQADICESLHDHADELYRSCLAR
(Proc. Natl. Acad. Sci. U.S.A., 79: 6313-6317 1982)
ROP_ECOLI, 32-55 (24)

Parallel five-stranded

phospholamban     LILICLLLICIIVMLL
PPLA_HUMAN, 37-52 (16)
(JBC 271, 5941-5946, 1996)

Dimerization of coiled-coils is not disrupted by modifications occurring at specific amino acids; however, as disclosed herein, phosphorylation of the "a" amino acid of the central heptad repeat is not tolerated, in that it destabilizes the coiled-coil structure. We describe the use of polypeptides comprising coiled-coils in order to assay the activity of a protein modifying enzyme which influences the state of post-translational protein modification.

General guidelines (assuming, in this instance, a 4.5-heptad coiled-coil structure) for placement of a protein modification site within a polypeptide comprising a coiled-coil for use in an assay as described are as follows:

1. It is preferable to insert a protein modification site into the interface between coiled-coil strands (i.e., positions a, d, e or g of the canonical heptad repeat structure or corresponding positions in non-canonical coiled-coil structures; Hicks et al., 1997, Folding & Design, 2: 149-158).

2. Modification at the 'a' site is preferable to that which occurs at the 'd' site, as the 'a site tolerates the presence of a polar amino acid better than does the 'd' site (Woolfson and Alber, 1995, Protein Sci., 4: 1596-1607).

3. Positions 'a' and 'd' of the heptad repeat usually contain residues V, I, L, M, or A (canonical residues); however certain other residues can be tolerated at these positions and often modulate oligomeric assembly. The stability of the coiled-coil oligomer reduces with each substitution of a non-canonical residue at positions 'a' or 'd', measured as a reduction in Tm (mid-point of the thermal unfolding transition of approximately 40°C per non-canonical residue at the 'a' or 'd' position in the GCN4 sequence background (Harbury, P.B., Zhang, T., Kim, P.S., and Alter, T., (1993) Science 262, 1401-1407).

4. It is preferable to insert the polar residue for modification within the central 3 heptads of the 4.5-heptad coiled-coil structure for maximum impact on the stability of the oligomer upon modification of the polar residue.

5. The covalent linkage of two coiled-coil strands increases the stability of the interaction between strands considerably. This can be used to facilitate the incorporation of more extreme changes from the canonical sequence pattern, and/or to reduce the number of repeating structures (heptad or other non-canonical repeats) needed for a stable interface.

6. Modification elsewhere in the coiled-coil (away from the hydrophobic interface) might affect the kinetics of folding of secondary structure and thereby inhibit coiled-coil oligomer formation. This will be chemical-moiety-dependent, having the greatest effect if the moiety in question is large and highly solvated.

Methods by which assays of the invention are performed are described in detail in the following sections and in the Examples.

Unless defined otherwise, all technical and scientific terms used herein have the same meaning as commonly understood by one of ordinary skill in the art to which this invention belongs. Generally, the nomenclature used herein and the laboratory procedures in cell culture, molecular genetics, and nucleic acid chemistry and hybridization described below are those well known and commonly employed in the art. Standard techniques are used for recombinant nucleic acid methods, polynucleotide synthesis, and microbial culture and transformation (e.g., electroporation, lipofection). Generally, enzymatic reactions and purification steps are performed according to the manufacturer's specifications. The techniques and procedures are generally performed according to conventional methods in the art and various general references (see generally, Sambrook et al., Molecular Cloning: A Laboratory Manual. 2d ed. (1989) Cold Spring Harbor Laboratory Press, Cold Spring Harbor, N.Y., which is incorporated herein by reference) which are provided throughout this document. The nomenclature used herein and the laboratory procedures in analytical chemistry, organic synthetic chemistry, and pharmaceutical formulation described herein are those well known and commonly employed in the art. Standard techniques are used for chemical syntheses, chemical analyses, pharmaceutical formulation and delivery, and treatment of patients. As employed throughout the disclosure, terms, unless otherwise indicated, shall be understood to have commonly understood meanings.

Fluorescence energy resonance transfer (FRET)

A tool with which to assess the distance between one molecule and another (whether protein or nucleic acid) or between two positions on the same molecule is provided by the technique of fluorescence resonance energy transfer (FRET), which is now widely known in the art (for a review, see Matyus, 1992, J. Photochem. Photobiol. B: Biol., 12: 323-337, which is herein incorporated by reference). FRET is a radiationless process in which energy is transferred from an excited donor molecule to an acceptor molecule; the efficiency of this transfer is dependent upon the distance between the donor an acceptor molecules, as described below. Since the rate of energy transfer is inversely proportional to the sixth power of the distance between the donor and acceptor, the energy transfer efficiency is extremely sensitive to distance changes. Energy transfer is said to occur with detectable efficiency in the 1-10 nm distance range, but is typically 4-6 nm for favorable pairs of donor and acceptor.

Radiationless energy transfer is based on the biophysical properties of fluorophores. These principles are reviewed elsewhere (Lakowicz, 1983, Principles of Fluorescence Spectroscopy. Plenum Press, New York; Jovin and Jovin, 1989, Cell Structure and Function by Microspectrofluorometry, eds. E. Kohen and J.G. Hirschberg, Academic Press, both of which are incorporated herein by reference). Briefly, a fluorophore absorbs light energy at a characteristic wavelength. This wavelength is also known as the excitation wavelength. The energy absorbed by a flurochrome is subsequently released through various pathways, one being emission of photons to produce fluorescence. The wavelength of light being emitted is known as the emission wavelength and is an inherent characteristic of a particular fluorophore. Radiationless energy transfer is the quantum-mechanical process by which the energy of the excited state of one fluorophore is transferred without actual photon emission to a second fluorophore. That energy may then be subsequently released at the emission wavelength of the second fluorophore. The first fluorophore is generally termed the donor (D) and has an excited state of higher energy than that of the second fluorophore, termed the acceptor (A). The essential features of the process are that the emission specturm of the donor overlap with the excitation spectrum of the acceptor, and that the donor and acceptor be sufficiently close. The distance over which radiationless energy transfer is effective depends on many factors including the fluorescence quantum efficiency of the donor, the extinction coefficient of the acceptor, the degree of overlap of their respective spectra, the refractive index of the medium, and the relative orientation of the transition moments of the two fluorophores. In addition to having an optimum emission range overlapping the excitation wavelength of the other fluorophore, the distance between D and A must be sufficiently small to allow the radiationless transfer of energy between the fluorophores.

FRET may be performed either *in vivo* or *in vitro*. Proteins are labeled either *in vivo* or *in vitro* by methods known in the art. Two coiled-coil domains comprised either by the same or by different polypeptide molecules may be differentially labeled, one with a donor and the other with an acceptor moiety, and differences in fluorescence between a test assay, comprising a protein modifying enzyme, and a control, in which the modifying enzyme is absent, are measured using a fluorimeter or laser-scanning microscope. It will be apparent to those skilled in the art that excitation/detection means can be augmented by the incorporation of photomultiplier means to enhance detection sensitivity. The differential labels may comprise either two different fluorescent moieties (e.g., fluorescent proteins as described below or the fluorophores rhodamine, fluorescein, SPQ, and others as are known in the art) or a fluorescent moiety and a molecule known to quench its signal; differences in the proximity of the coiled-coil domains with and without the protein-modifying enzyme can be gauged based upon a difference in the fluorescence spectrum or intensity observed.

This combination of protein-labelling methods and devices confers a distinct advantage over prior art methods for determining the activity of protein-modifying enzymes, as described above, in that results of all measurements are observed in real time (i.e., as a reaction progresses). This is significantly advantageous, as it allows both for rapid data collection and yields information regarding reaction kinetics under various conditions.

A sample, whether *in vitro* or *in vivo,* which is assayed therefore comprises a mixture at equilibrium of polypeptides comprising labeled coiled-coil domains which, when disassociated from one another, fluoresce at one frequency and, when complexed together, fluoresce at another frequency or, alternatively, of molecules which either do or do not fluoresce depending upon whether or not they are associated.

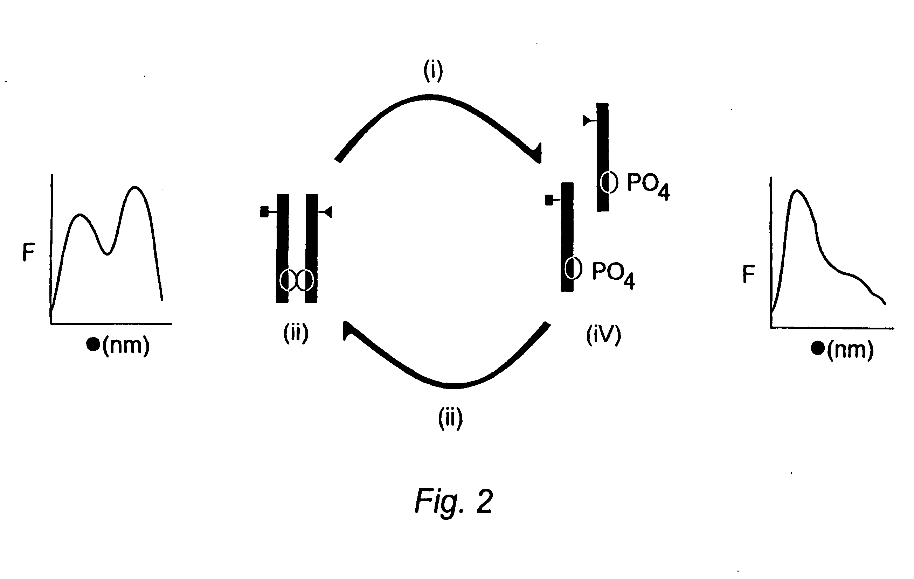

The coiled-coil portion of a polypeptide comprising a coiled-coil is modified to allow the attachment of a fluorescent moiety to the surface of the a-helix or is fused in-frame with a fluorescent protein, as described below. The choice of fluorescent moiety will be such that upon excitation with light, labeled peptides which are associated will show optimal energy transfer between fluorophores. In the presence of a protein modifying enzyme (e.g., a ubiquitinating-, ADP-ribosylating- or glycosylating enzyme), the polypeptides comprising coiled-coils dissociate due to disruption of the coiled-coil structure which occurs as a consequence of modification of the enzyme recognition site, thereby leading to a decrease in energy transfer and increased emission of light by the donor fluorophore. In this way, the state of polypeptide modification can be monitored and quantitated in real-time. This scheme is shown in Figure 2.

As used herein, the terms "fluorophore" and "fluorochrome" refer interchangeably to a molecule which is capable of absorbing energy at a wavelength range and releasing energy at a wavelength range other than the absorbance range. The term "excitation wavelength" refers to the range of wavelengths at which a fluorophore absorbs energy. The term "emission wavelength" refers to the range of wavelength that the fluorophore releases energy or fluoresces.

A non-limiting list of chemical fluorophores, along with their excitation and emission wavelengths, is presented in Table 1.

Table 1

| Fluorophore | Excitation (nm) | Emission (nm) | Color |
|---|---|---|---|
| PKH2 | 490 | 504 | green |
| PKH67 | 490 | 502 | green |
| Fluorescein (FITC) | 495 | 525 | green |
| Hoechst 33258 | 360 | 470 | blue |
| R-Phycoerythrin (PE) | 488 | 578 | orange-red |
| Rhodamine (TRITC) | 552 | 570 | red |
| Quantum Red™ | 488 | 670 | red |
| PKH26 | 551 | 567 | red |
| Texas Red | 596 | 620 | red |
| Cy3 | 552 | 570 | red |

Examples of fluorescent proteins which vary among themselves in excitation and emission maxima are listed in Table 1 of WO 97/28261 (Tsien et al., 1997, supra). These (each followed by [excitation max./emission max.] wavelengths expressed in nanometers) include wild-type Green Fluorescent Protein [395(475)/508] and the cloned mutant of Green Fluorescent Protein variants P4 [383/447], P4-3 **[381/445],** W7 [433(453)/475(501)], W2 [432(453)/480], S65T [489/511], P4-1 [504(396)/480], S65A [471/504], S65C [479/507], S65L [484/510], Y66F [360/442], Y66W [458/480], I0c [513/527], W1B [432(453)/476(503)], Emerald [487/508] and Sapphire [395/511]. This list is not exhaustive of fluorescent proteins known in the art; additional examples are found in the Genbank and SwissProt public databases.

A number of parameters of fluorescence output are envisaged including

1) measuring fluoresence emitted at the emission wavelength of the acceptor (A) and donor (D) and determining the extent of energy transfer by the ratio of their emission amplitudes;
2) measuring the fluoresence lifetime of D;
3) measuring the rate of photobleaching of D;
4) measuring the anistropy of D and/or A; or

5) measuring the Stokes shift monomer; eximer fluorescence.

Fluorescent protein moieties

In a FRET assay, the fluorescent protein moieties are chosen such that the excitation spectrum of one of the moieties (the acceptor moiety) overlaps with the emission spectrum of the excited fluorescent moiety (the donor moiety). The donor moiety is excited by light of appropriate intensity within the donor's excitation spectrum. The donor then emits some of the absorbed energy as fluorescent light and dissipates some of the energy by FRET to the acceptor fluorescent moiety. The fluorescent energy it produces is quenched by the acceptor fluorescent protein moiety. FRET can be manifested as a reduction in the intensity of the fluorescent signal from the donor, reduction in the lifetime of its excited state, and re-emission of fluorescent light at the longer wavelengths (lower energies) characteristic of the acceptor. When the donor and acceptor moieties become spatially separated, FRET is diminished or eliminated.

One can take advantage of the FRET exhibited by two polypeptides comprising coiled-coils labeled with different fluorescent protein moieties, wherein one coiled-coil of a polypeptide comprising a coiled-coil is linked to a donor and another to an acceptor moiety, in monitoring protein modification A single polypeptide may comprises a blue fluorescent protein donor moiety and a green fluorescent protein acceptor moiety, wherein each is fused to a different coiled-coil within a polypeptide comprising a coiled-coil; such a construct is herein referred to as a "tandem" fusion protein. Alternatively, two distinct polypeptides ("single" fusion proteins) each comprising a coiled-coil may be differentially labeled with the donor and acceptor fluorescent protein moieties, respectively. The construction and use of tandem fusion proteins can reduce significantly the molar concentration of peptides necessary to effect an association between differentially-labeled coiled-coils within one of more polypeptides comprising a coiled-coil relative to that required when single fusion proteins are instead used. The labeled coiled-coils comprised by polypeptides comprising a coiled-coil may be produced via the expression of recombinant nucleic acid molecules comprising an in-frame fusion of sequences encoding a coiled-coil within a polypeptide comprising a coiled-coil and a fluorescent protein moiety either *in vitro* (e.g., using a cell-free transcription/translation system, as described below, or instead using cultured cells transformed or transfected using methods well known in the art) or *in vivo,* for example in a trangenic animal including, but not limited to, insects, amphibians and mammals. A recombinant nucleic acid molecule may be constructed and expressed by molecular methods well known in the art, and may additionally comprise sequences including, but not limited to, those which encode a tag (e.g., a histidine tag) to enable easy purification, a secretion signal, a nuclear localization signal or other primary sequence signal capable of targeting the construct to a particular cellular location, if it is so desired.

The means by which two polypeptides comprising coiled-coils are assayed for association using fluorescent protein moiety labels according to the methods described here may be briefly summarized as follows:

Whether or not the two coiled-coils are present on a single polypeptide molecule, one is labeled with a green fluorescent protein, while the other is preferably labeled with a red or, alternatively, a blue fluorescent protein. Useful donor:acceptor pairs of flurescent proteins (see Tsien et al., 1997, supra) include, but are not limited to:

Donor: S72A, K79R, Y145F, M153A and T2031 (excitation 6 395nm; emission ë 511)
Acceptor: ë S659, S72A, K79R and T203Y (wavelengths not noted), or T203Y/S65G, V68L, Q69K or S72A (excitation e 515nm; emission 527nm).

An example of a blue:green pairing is P4-3 (shown in Table 1 of Tsien et al., 1997, supra) as the donor moiety and S65C (also of Table 1 of Tsien et al., 1997, supra) as the acceptor moiety. The polypeptides comprising coiled-coils are exposed to light at, for example, 368 nm, a wavelength that is near the excitation maximum of P4-3. This wavelength excites S65C only minimally. Upon excitation, some portion of the energy absorbed by the blue fluorescent protein moiety is transferred to the acceptor moiety through FRET if the two polypeptides comprising coiled-coils are in close association. As a result of this quenching, the blue fluorescent light emitted by the blue fluorescent protein is less bright than would be expected if the blue fluorescent protein existed in isolation. The acceptor moiety (S65C) may re-emit the energy at longer wavelength, in this case, green fluorescent light.

After modification (e.g., phosphorylation, ADP-ribosylation, ubiquitination or glycosylation, all as described below) of one or both of the coiled-coils of a polypeptide comprising a coiled-coil by an enzyme, the two polypeptides comprising coiled-coils (and, hence, the green and red or, less preferably, green and blue fluorescent proteins) physically separate or associate, accordingly inhibiting or promoting FRET. For example, if activity of the modifying enzyme results in dissociation of a protein:protein dimer, the intensity of visible blue fluorescent light emitted by the blue fluorescent protein increases, while the intensity of visible green light emitted by the green fluorescent protein as a result of FRET, decreases.

Such a system is useful to monitor the activity of enzymes that modify the coiled-coils of one or more polypeptides comprising a coiled-coil to which the fluorescent protein moieties are fused as well as the activity of modulators or candidate modulators of those enzymes.

In particular, our methods include assays in which the amount- or activity of a modifying enzyme in a sample is determined by contacting the sample with a pair of polypeptides comprising coiled-coil motifs differentially labeled with fluorescent proteins, as described above, and measuring changes in fluorescence of the donor moiety, the acceptor moiety or the relative fluorescence of both. Fusion proteins, as described above, which comprise either one or both labeled polypeptides comprising coiled-coils can be used for, among other things, monitoring the activity of a modifying enzyme inside the cell that expresses the recombinant tandem construct or two different recombinant constructs.

Advantages of single- and tandem fluorescent protein/polypeptide comprising a coiled-coil fusion constructs include the greater extinction coefficient and quantum yield of many of these proteins compared with those of the Edans fluorophore. Also, the acceptor in such a construct or pair of constructs is, itself, a fluorophore rather than a non-fluorescent quencher like Dabcyl. Thus, the enzyme's substrate, i.e., the unmodified coiled-coils of the construct(s) and products (i.e., the coiled-coils after modification) are both fluorescent but with different fluorescent characteristics.

In particular, the substrate and modified products exhibit different ratios between the amount of light emitted by the donor and acceptor moieties. Therefore, the ratio between the two fluorescences measures the degree of conversion of substrate to products, independent of the absolute amount of either, the optical thickness of the sample, the brightness of the excitation lamp, the sensitivity of the detector, etc. Furthermore, *Aequorea*-derived or -related fluorescent protein moieties tend to be protease resistant. Therefore, they are likely to retain their fluorescent properties throughout the course of an experiment.

Polypeptide comprising a coiled-coil/fluorescent protein fusion constructs

As stated above, recombinant nucleic acid constructs of particular use in the methods described here are those which comprise in-frame fusions of sequences encoding a polypeptide comprising a coiled-coil motifs and a fluorescent protein. If two coiled-coils are to be expressed as part of a single polypeptide, the nucleic acid molecule additionally encodes, at a minimum, a donor fluorescent protein moiety fused to one coiled-coil, an acceptor fluorescent protein moiety fused to a second coiled-coil, a linker moiety that couples the two coiled-coils and is of sufficient length and flexibility to allow for folding of the polypeptide and pairing of the two coiled-coils and gene regulatory sequences operatively linked to the fusion coding sequence. If single fusion proteins are instead encoded (whether by one or more nucleic acid molecules), each nucleic acid molecule need only encode a polypeptide comprising a coiled-coil fused either to a donor or acceptor fluorescent protein moiety and operatively linked to gene regulatory sequences.

"Operatively-linked" refers to polynucleotide sequences which are necessary to effect the expression of coding and non-coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, components whose presence can influence expression, and can also include additional components whose presence is advantageous, for example, leader sequences and fusion partner sequences.

As described above, the donor fluorescent protein moiety is capable of absorbing a photon and transferring energy to another fluorescent moiety. The acceptor fluorescent protein moiety is capable of absorbing energy and emitting a photon. If needed, the linker moiety connects the two polypeptides comprising coiled-coils either directly or indirectly, through an intermediary linkage with one or both of the donor and acceptor fluorescent protein moieties. Regardless of the relative order of the first and second polypeptides comprising coiled-coils and the donor and acceptor fluorescent protein moieties on a polypeptide molecule, it is essential that sufficient distance be placed between the donor and acceptor by the linker and/or the polypeptides comprising coiled-coils to ensure that FRET does not occur unless the two coiled-coils dimerize. It is desirable, as described in greater detail in WO97/28261, to select a donor fluorescent protein moiety with an emission spectrum that overlaps with the excitation spectrum of an acceptor fluorescent protein moiety. In some embodiments the overlap in emission and excitation spectra will facilitate FRET. Such an overlap is not necessary, however, if intrinsic fluorescence is measured instead of FRET. A fluorescent protein of use in the method includes, in addition to those with intrinsic fluorescent properties, proteins that fluoresce due intramolecular rearrangements or the addition of cofactors that promote fluorescence.

For example, green fluorescent proteins ("GFPs") of cnidarians, which act as their energy-transfer acceptors in bioluminescence, can be used in the methods. A green fluorescent protein, as used herein, is a protein that fluoresces green light, and a blue fluorescent protein is a protein that fluoresces blue light. GFPs have been isolated from the Pacific Northwest jellyfish, *Aequorea victoria,* from the sea pansy, *Renilla reniformis,* and from *Phialidium gregarium.* (Ward et al., 1982, Photochem. Photobiol., 35: 803-808; Levine et al., 1982, Comp. Biochem. Physiol.,72B: 77-85).

A variety of *Aequorea*-related GFPs having useful excitation and emission spectra have been engineered by modifying the amino acid sequence of a naturally occurring GFP from Aequorea victoria. (Prasher et al., 1992, Gene, 111: 229-233; Heim et al., 1994, Proc. Natl. Acad. Sci. U.S.A., 91: 12501-12504; PCT/US95/14692). As used herein, a fluorescent protein is an Aequorea-related fluorescent protein if any contiguous sequence of 150 amino acids of the fluorescent protein has at least 85% sequence identity with an amino acid sequence, either contiguous or non-contiguous, from the wild-type Aequorea green fluorescent protein (SwissProt Accession No. P42212). More preferably, a fluorescent protein is an Aequorea-related fluorescent protein if any contiguous sequence of 200 amino acids of the fluorescent protein has at least 95% sequence identity with an amino acid sequence, either contiguous or non-contiguous, from the wild type *Aequorea* green fluorescent protein of SwissProt Accession No. P42212. Similarly, the fluorescent protein may be related to *Renilla* or *Phialidium* wild-type fluorescent proteins using the same standards.

*Aequorea*-related fluorescent proteins include, for example, wild-type (native) *Aequorea victoria* GFP, whose nucleotide and deduced amino acid sequences are presented in Genbank Accession Nos. L29345, M62654, M62653 and others *Aequorea*-related engineered versions of Green Fluorescent Protein, of which some are listed above. Several of these, i.e., P4, P4-3, W7 and W2 fluoresce at a distinctly shorter wavelength than wild type.

Recombinant nucleic acid molecules encoding single- or tandem fluorescent protein/polypeptide comprising a coiled-coil fusion proteins useful in the methods may be expressed either for *in vivo* assay of the activity of a modifying enzyme on the encoded products. Alternatively, the encoded fusion protiens may be isolated prior to assay, and instead assayed in a cell-free *in vitro* assay system, as described elsewhere herein.

As used herein, the terms "protein", "subunit" and "domain" refer to a linear sequence of amino acids which exhibits biological function. This linear sequence includes full-length amino acid sequences (e.g. those encoded by a full-length gene), or a portion or fragment thereof, provided the biological function is maintained by that portion or fragment. The terms subunit and domain also may refer to polypeptides and peptides having biological function. A peptide useful in the methods will at least have a binding capability, i,e, with respect to binding as or to a binding partner, and also may have another biological function that is a biological function of a protein or domain from which the peptide sequence is derived.

Protein modifications in assays

ADP-ribosylation

Mono-ADP-ribosylation is a post-translational modification of proteins which is currently thought to play a fundamental role in cellular signalling. A number of mono-ADP-ribosyl-transferases have been identified, including endogenous enzymes from both bacterial and eukaryotic sources and bacterial toxins. A mono-ADP-riboylating enzyme, using as substrates the protein to be modified and nicotinamide adenine dinucleotide (NAD$^+$), is NAD:Arginine ADP ribosyltransferase (Zolkiewska et al., 1992, Proc Natl. Acad. Sci. U.S.A., 89: 11352-11356). The reactions catalysed by bacterial toxins such as cholera and pertussis toxin are well understood, the activity of these toxins result in the permanent modification of heterotrimeric G proteins. Endogenous transferases are also thought to modify G proteins and therefore to play a role in signal transduction in the cell (de Murcia et al., 1995, Trends Cell Biol., 5: 78-81). The extent of the effects that ADP-ribosylation can mediate in the cell is illustrated by the example of brefeldin A, a fungal toxin metabolite of palmitic acid. This toxin induces the mono-ADP-ribosylation of BARS-50 (a G protein involved in membrane transport) and glyceraldehyde-3-phosphate dehydrogenase. The cellular effects of brefeldin A include the blocking of constitutive protein secretion and the extensive disruption of the Golgi apparatus. Inhibitors of the brefeldin A mono-ADP-ribosyltransferase reaction have been shown to antagonise the disassembly of the Golgi apparatus induced by the toxin (Weigert et al., 1997, J. Biol. Chem., 272: 14200-14207). A number of amino acid residues within proteins have been shown to function as ADP-ribose acceptors. Bacterial transferases have been identified which modify arginine, asparagine, cysteine and diphthamide residues in target proteins. Endogenous eukaryotic transferases are known which also modify these amino acids, in addition there is evidence that serine, threonine, tyrosine, hydroxyproline and histidine residues may act as ADP-ribose acceptors but the relevant transferases have not yet been identified (Cervantes-Laurean et al., 1997, Methods Enzymol., 280: 275-287 and references therein).

Poly-ADP-ribosylation is thought to play an important role in events such as DNA repair, replication, recombination and packaging and also in chromosome decondensation. The enzyme responsible for the poly-ADP-ribosylation of proteins involved in these processes is poly (ADP-ribose) polymerase (PARP; for *Drosophila melanogaster* PARP, see Genbank Accession Nos. D13806, D13807 and D13808). The discovery of a leucine zipper in the self-poly(ADP-ribosyl)ation domain of the mammalian PARP (Uchida et al., 1993, Proc. Natl. Acad. Sci. U.S.A., 90: 3481-3485) suggested that this region may be important for the dimerisation of PARP and also its interaction with other proteins (Poly (ADP-ribose) polymerase is a catalytic dimer and the automodification reaction is intermolecular, Mendoza-Alvarez et al., 1993. J. Biol, Chem., 268: 22575-22580).

Specific examples of ADP ADP-ribosylation sites are those found at Cys$_3$ and Cys$_4$ (underlined) of the B-50 protein (Coggins et al., 1993, J. Neurochem., 60: 368-371; SwissProt Accession No. P06836):
ML<u>CC</u>MRRTKQVEKNDDD
and Pã (the a subunit of cyclic CMP phophodiesterase; Bondarenko et al., 1997, J. Biol. Chem., 272: 15856-15864;

Genbank Accession No. X04270):
FKQRQTRQFK.

A survey of the literature suggests that ADP-ribosylation is a very important post-translational modification whose significance has only relatively recently been appreciated and that as the field develops, the scope for applying this concept to the study ADP-ribosylation will increase.

Ubiquitination

Ubiquitination of a protein targets the protein for destruction by the proteosome. This process of destruction is very rapid ($t_{1/2} \sim 60$ seconds), and many proteins with rapid turnover kinetics are destroyed via this route. These include cyclins, p53, transcription factors and transcription regulatory factors, among others. Thus, ubiquitination is important in processes such as cell cycle control, cell growth, inflammation, signal transduction; in addition, failure to ubiquitinate proteins in an appropriate manner is implicated in malignant transformation. Ubiquitin is a 76-aminoacid protein which is covalently attached to a target protein by an isopeptide bond, between the a-amino group of a lysine residue and the C-terminal glycine residue of ubiquitin. Such modification is known as mono-ubiquitination, and this can occur on multiple Lys residues within a target protein. Once attached, the ubiquitin can itself be ubiquitinated, thus forming extended branched chains of polyubiquitin. It is this latter state which signals destruction of the target protein by the proteosome. In the process of destruction, it appears that the polyubiquitinated protein is taken to the proteosome via a molecular chaperone protein, the ubiquitin molecules are removed undamaged (and recycled) and the target is degraded.

Ubiquitination is a complex process, which requires the action of three enzymes: Ubiquitin activating enzyme E1 (for human, Genbank Accession No. X56976), ubiquitin conjugating enzyme E2, also referred to as the ubiquitin carrier protein, (for human 17kDa form, Genbank Accession No. X78140) and Ubiquitin protein ligase E3á (UBR1; human, Genbank Accession No. AF061556). There are multiple forms of each of these enzymes in the cell, and the above examples are, therefore, non-limiting.

The signals contained within a protein which determine whether the protein is subject to the process of ubiquitination and destruction are two-fold: first, the identity of the N-terminal amino acid (so called N-end rule, Varshavsky, 1996, Proc. Natl. Acad. Sci. U.S.A., 93: 12142-12149), and secondly the presence of a suitably positioned Lys residue in the protein (Varshavsky, 1996, supra). This Lys can be up to $\sim 30$ amino acids away from the N-terminus in experimental examples studied where the N-terminus is a flexible, poorly-structured element of the protein (Varshavsky, 1996, supra) or could potentially be anywhere in the sequence where this presents it at an appropriate location relative to the N-terminus. An appropriate location is one which allows interaction of both the N-terminal residue and this integral lysine with the enzyme(s) responsible for ubiquitination, presumably simultaneously. The Lys residue becomes ubiquitinated, and the process of destruction is initiated. N-terminal residues can be classed as stabilizing (s) or destabilizing (d), and the inclusion of an amino acid in one of these broad classes is species-dependent (prokaryotes differ from yeast, which differs from mammals; Varshavsky, 1996, supra).

In a dimeric (or other oligomeric protein) the destabilizing N-terminal residue and the internal Lys can be in *cis* (on a single peptide), but may also be in *trans* (on two different polypeptides). The *trans*-recognition event will only take place while the complex is physically associated. Only the ubiquitinated subunit is proteolyzed (Varsharsky, 1996, supra).

Two examples of ubiquitination sites from natural proteins, IeB (Dai et al., 1998, J. Biol. Chem, 273: 3562-3573; Genbank Accession No. M69043) and â-galactosidase (Johnson et al., 1990, Nature. 346: 287-291) are as follows:

IêB               NH$_3$-MFQAAERPQEWAMEGPRDGLKKERLLDDRH-COOH
â-galactosidase   NH$_3$-HGSGAWLLPVSLVKRKTTLAP-COOH

where the ubiquitinated lysine residue is underlined for each (e.g., Lys$_{15}$ and Lys$_{17}$ for β-galactosidase).

In the methods described here, a ubiquitination assay measures the addition of ubiquitin to-, rather than the destruction of-, a polypeptide comprising a coiled-coil.

Glycosylation

N-linked glycosylation is a post-translational modification of proteins which occurs in the endoplasmic reticulum and golgi apparatus and is utilized with some proteins *en route* for secretion or destined for expression on the cell surface or in another organelle. The carbohydrate moiety is attached to Asn residues in the non-cytoplasmic domains of the target proteins, and the consensus sequence (Shakineshleman, 1996, Trends Glycosci. Glycotech., 8: 115-130) for a glycosylation site is:

## NxS/T,

where x cannot be proline or aspartic acid. An enzyme known to mediate N-glycosylation at the initial step of synthesis of dolichol-P-P-oligosaccharides is UDP-N-Acetylglucosamine-Dolichyl-phosphate-N-acetylsglucosamine phosphotransferase (for mouse, Genbank Accession Nos. X65603 and S41875).

Oxygen-linked glycosylation also occurs in nature with the attachment of various sugar moieties to Ser or Thr residues (Hansen et al., 1995, Biochem. J., 308: 801-813). Intracellular proteins are among the targets for O-glycosylation through the dynamic attachment and removal of O-N-Acetyl-D-glucosamine (O-GlcNAc; reviewed by Hart, 1997, Ann. Rev. Biochem., 66: 315-335). Proteins known to be O-glycosylated include cytoskeletal proteins, transcription factors, the nuclear pore protein complex, and tumor-suppressor proteins (Hart, 1997, supra). Frequently these proteins are also phosphoproteins, and there is a suggestion that O-GlcNAc and phosphorylation of a protein play reciprocal roles. Furthermore, it has been proposed that the glycosylation of an individual protein regulates protein:protein interactions in which it is involved.

Specific sites for the addition of O-GlcNAc are found, for example, at $Ser_{277}$, $Ser_{316}$ and $Ser_{383}$ of $p67^{SRF}$ (Reason et al., 1992, J. Biol. Chem., 267: 16911-16921; Genbank Accession No. J03161). The recognition sequences encompassing these residues are shown below:

$^{274}$GTTSTIQTAP
$^{313}$SAVSSADGTVLK
$^{374}$DSSTDLTQTSSSGTVTLP

The identity of sites of O-GlcNAc is additionally known for a small number of proteins including c-myc ($Thr_{58}$, also a phosphorylation site; Chou et al., 1995, J. Biol. Chem., 270: 18961-18965), the nucleopore protein p62 (see Reason et al., 1992, supra):

MAGGPADTSDPL

and band 4.1 of the erythrocyte (see Reason et al., 1992, supra):

AQTITSETPSSTT.

The site at which modification occurs is, in each case, underlined. The reaction is mediated by O-GlcNAc transferase (Kreppel et al., 1997, J. Biol. Chem., 272: 9308-9315). These sequences are rich in helix breaking residues (e.g., G and P) and may therefore be difficult to incorporate into the coiled-coil framework, as it is an α-helical structure.

Phosphorylation

Protein phosphorylation is described at some length above and in the sections following.

Methods for detection of protein modification in real time

A. *In vitro* protein modification and detection thereof

Modifying enzymes

The invention detects the presence of a modifying enzyme which catalyzes either the addition or removal of a modifying group. A range of kinases, phosphatases and other modifying enzymes are available commercially (e.g. from Sigma, St. Louis, MO; Promega, Madison, WI; Boehringer Mannheim Biochemicals, Indianapolis, IN; New England Biolabs, Beverly, MA; and others). Alternatively, such enzymes may be prepared in the laboratory by methods well known in the art.

The catalytic sub-unit of protein kinase A (c-PKA) can be purified from natural sources (e.g. bovine heart) or from cells/organisms engineered to heterologously express the enzyme. Other isoforms of this enzyme may be obtained by these procedures. Purification is performed as previously described from bovine heart (Peters et al.,1977, Biochemistry. 16: 5691-5697) or from a heterologous source (Tsien et al., WO92/00388), and is in each case briefly summarized as follows:

Bovine ventricular cardiac muscle (2kg) is homogenized and then centrifuged. The supernatant is applied to a strong anion exchange resin (e.g. Q resin, Bio-Rad) equilibrated in a buffer containing 50mM Tris-HCl, 10mM NaCl, 4mM EDTA pH 7.6 and 0.2mM 2-mercaptoethanol. The protein is eluted from the resin in a second buffer containing 50mM Tris-HCl, 4mM EDTA pH 7.6, 0.2mM 2-mercaptoethanol, 0.5M NaCl. Fractions containing c-PKA are pooled and ammonium sulphate added to 30% saturation. Proteins precipitated by this are removed by centrifugation and the ammonium sulphate concentration of the supernatant was increased to 75% saturation. Insoluble proteins are collected by centrifugation (included c-PKA) and are dissolved in 30mM phosphate buffer pH 7.0, 1mM EDTA, 0.2mM 2-mercaptoethanol. These proteins are then dialysed against the same buffer (500 volume excess) at 4°C for two periods of 8 hours each. The pH of the sample is reduced to 6.1 by addition of phosphoric acid, and the sample is mixed sequentially with 5 batches of CM-Sepharose (Pharmacia, ~ 80 ml resin each) equilibrated in 30mM phosphate pH 6.1, 1mM EDTA, 0.2 mM 2-mercaptoethanol. Cyclic AMP (10 $\mu$M) is added to the material which fails to bind to the CM-Sepharose, and the sample-cAMP mix is incubated with a fresh resin of CM-Sepharose (~ 100 ml) equilibrated as before. c-PKA is eluted from this column following extensive washing in equilibration buffer by addition of 30mM phosphate pH 6.1, 1 mM EDTA, 1M KCl, 0.2 mM 2-mercaptoethanol. Fractions containing c-PKA are pooled and concentrated by filtration through a PM-30 membrane (or similar). The c-PKA sample is then subjected to gel-filtration chromatography on a resin such as Sephacryl 200HR (Pharmacia).

The purification of recombinant c-PKA is as described in WO 92/00388. General methods of preparing pure and partially-purified recombinant proteins, as well as crude cellular extracts comprising such proteins, are well known in the art. Molecular methods useful in the production of recombinant proteins, whether such proteins are the enzymes to be assayed or the labeled reporter polypeptides comprising a coiled-coil, are well known in the art (for methods of cloning, expression of cloned genes and protein purification, see Sambrook et al., 1989, Molecular Cloning. A Laboratory Manual., 2nd Edition, Cold Spring Harbor Laboratory Press, Cold Spring Harbor, NY; Ausubel et al., Current Protocols in Molecular Biology, copyright 1987-1994, Current Protocols, copyright 1994-1998, John Wiley & Sons, Inc.). The sequences of the catalytic subunit of several PKA molecules are found in the Genbank database (see PKA Ca, bovine, Genbank Accession Nos. X67154 and S49260; PKA C$\beta$1, bovine, Genbank Accession No. J02647; PKA C$\beta$2, bovine, M60482, the form most likely purified from bovine heart by the protocol described above).

Assays of the activity of protein-modifying enzymes may be performed using crude cellular extracts, whether to test the activity of a recombinant protein or one which is found in nature, such as in a biological sample obtained from a test cell line or animal or from a clinical patient. In the former case, use of a crude cell extract enables rapid screening of many samples, which potentially finds special application in high-throughput screening methods, e.g. of candidate modulators of protein-modifying enzyme activity. In the latter case, use of a crude extract with the labeled reporter polypeptide comprising a coiled-coil facilitates easy and rapid assessment of the activity of an enzyme of interest in a diagnostic procedure, e.g., one which is directed at determining whether a protein-modifying enzyme is active at an a physiologically-appropriate level, or in a procedure designed to assess the efficacy of a therapy aimed at modulating the activity of a particular enzyme.

Synthesis of polypeptides comprising a coiled-coil

Polypeptides comprising a coiled-coil may be synthesised by Fmoc or Tboc chemistry according to methods known in the art (e.g., see Atherton et al., 1981, J. Chem. Soc. Perkin I, 1981(2): 538-546; Merrifield, 1963, J. Am. Chem. Soc., 85: 2149-2154, respectively). Following deprotection and cleavage from the resin, peptides are desalted by gel filtration chromatography and analysed by mass spectroscopy, HPLC, Edman degradation and/or other methods as are known in the art for protein sequencing using standard methodologies.

Alternatively, nucleic acid sequences encoding such peptides may be expressed either in cells or in an *in vitro* transcription/translation system (see below) and, as with enzymes to be assayed, the proteins purified by methods well known in the art.

Labelling polypeptides comprising a coiled-coil with fluorophores

Polypeptides comprising coiled-coils are labeled with thiol reactive derivatives of fluorescein and tetramethylrhodamine (isothiocyanate or iodoacetamide derivatives, Molecular Probes, Eugene, OR, USA) using procedures described by Hermanson G.T., 1995, Bioconjugate Techniques, Academic Press, London. Alternatively, primary-amine-directed conjugation reactions can be used to label lysine sidechains or the free peptide N-terminus (Hermason, 1995, supra).

Purification of fluorescent peptides

Fluorescent peptides are separated from unreacted fluorophores by gel filtration chromatography or reverse phase HPLC.

### Phosphorylation of peptides *in vitro*

Peptides (0.01-1.0iM) are phosphorylated by purified c-PKA in 50mM Histidine buffer pH 7.0, 5mM MgSO$_4$, 1mM EGTA, 0.1-1.0 iM c-PKA, and 0.2mM [$^{32}$P] ã-ATP (specific activity ~ 2Bq/pmol) at 30-37˚C for periods of time ranging from 0 to 60 minutes. Where the chemistry of the peptide is appropriate (*i.e.* having a basic charge) the phosphopeptide is captured on a cation exchange filter paper (e.g. phosphocellulose P81 paper; Whatman), and reactants are removed by extensive washing in 1% phosphoric acid (see Casnellie, 1991, Methods Enzymol., 200: 115-120). Alternatively, phosphorylation of samples is terminated by the addition of SDS-sample buffer (Laemmli,1970, Nature, 227: 680-685) and the samples analysed by SDS-PAGE electrophoresis, autoradiography and scintillation counting of gel pieces.

### Dephosphorylation of peptides *in vitro*

The dephosphorylation of peptides phosphorylated as above is studied by removal of ATP (through the addition of 10mM glucose and 30 U/ml hexokinase; Sigma, St. Louis, MO) and addition of protein phosphatase-1 (Sigma). Dephosphorylation is followed at 30-37˚C by quantitation of the remaining phosphopeptide component at various time points, determined as above.

### Fluorescence measurements of protein modification *in vitro* in real time

Donor and acceptor fluorophore-labeled polypeptides comprising coiled-coils (molar equivalents of fluorophore-labeled polypeptide or molar excess of acceptor-labeled polypeptide) are first mixed (if the two coiled-coils are present on separate polypeptides). Samples are analyzed in a fluorimeter using excitation wavelengths relevant to the donor fluorescent moiety and emission wavelengths relevant to both the donor and acceptor moieties. A ratio of emission from the acceptor over that from the donor following excitation at a single wavelength is used to determine the efficiency of fluorescence energy transfer between fluorophores, and hence their spatial proximity. Typically, measurements are performed at 0-37˚C as a function of time following the addition of the modifying enzyme (and, optionally, a modulator or candidate modulator of function for that enzyme, as described below) to the system in 50mM histidine pH 7.0, 120 mM KCI, 5mM MgSO$_4$, 5mM NaF, 0.05mM EGTA and 0.2mM ATP. The assay may be performed at a higher temperature if that temperature is compatible with the enzyme(s) under study.

### Alternative cell-free assay systems

A cell-free assay system is required to permit dimerization of unmodified, labeled polypeptides comprising coiled-coils to occur. As indicated herein, such a system may comprise a low-ionic-strength buffer (e.g., physiological salt, such as simple saline or phosphate- and/or Tris-buffered saline or other as described above), a cell culture medium, of which many are known in the art, or a whole or fractionated cell lysate. The components of an assay of protein modification may be added into a buffer, medium or lysate or may have been expressed in cells from which a lysate is derived. Alternatively, a cell-free transcription- and/or translation system may be used to deliver one or more of these components to the assay system. Nucleic acids of use in cell-free expression systems are as described for *in vivo* assays, below.

An assay may be peformed in a standard in vitro transcription/translation system under conditions which permit expression of a recombinant or other gene. The TNT® T7 Quick Coupled Transcription/Translation System (Cat. # L1170; Promega) contains all reagents necessary for *in vitro* transcription/translation except the DNA of interest and the detection label; as discussed below, polypeptides comprising coiled-coils may be encoded by expression constructs in which their coding sequences are fused in-frame to those encoding fluorescent proteins. The TNT® Coupled Reticulocyte Lysate Systems (comprising a rabbit reticulocyte lysate) include: TNT® T3 Coupled Reticulocyte Lysate System (Cat. # L4950; Promega); TNT® T7 Coupled Reticulocyte Lysate System (Cat. # L4610; Promega); TNT® SP6 Coupled Reticulocyte Lysate System (Cat. # L4600; Promega); TNT® T7/SP6 Coupled Reticulocyte Lysate System (Cat. # L5020; Promega); TNT® T7/T3 Coupled Reticulocyte Lysate System (Cat. # L5010; Promega).

An assay involving a cell lysate or a whole cell (see below) may be performed in a cell lysate or whole cell preferably eukaryotic in nature (such as yeast, fungi, insect, e.g., *Drosophila),* mouse, or human). An assay in which a cell lysate is used is performed in a standard *in vitro* system under conditions which permit gene expression. A rabbit reticulocyte lysate alone is also available from Promega, either nuclease-treated (Cat. # L4960) or untreated (Cat. # L4151).

Candidate modulators of protein-modifying enzymes to be screened

Whether *in vitro* or in an *in vivo* system (see below), this document encompasses methods by which to screen compositions which may enhance, inhibit or not affect (e.g., in a cross-screening procedure in which the goal is to determine whether an agent intended for one purpose additionally affects general cellular functions, of which protein modification is an example) the activity of a protein-modifying enzyme.

Candidate modulator compounds from large libraries of synthetic or natural compounds can be screened. Numerous means are currently used for random and directed synthesis of saccharide, peptide, and nucleic acid based compounds. Synthetic compound libraries are commercially available from a number of companies including Maybridge Chemical Co. (Trevillet, Cornwall, UK), Comgenex (Princeton, NJ), Brandon Associates (Merrimack, NH), and Microsource (New Milford, CT). A rare chemical library is available from Aldrich (Milwaukee, WI). Combinatorial libraries are available and can be prepared. Alternatively, libraries of natural compounds in the form of bacterial, fungal, plant and animal extracts are available from e.g., Pan Laboratories (Bothell, WA) or MycoSearch (NC), or are readily produceable by methods well known in the art. Additionally, natural and synthetically produced libraries and compounds are readily modified through conventional chemical, physical, and biochemical means.

Useful compounds may be found within numerous chemical classes, though typically they are organic compounds, including small organic compounds. Small organic compounds have a molecular weight of more than 50 yet less than about 2,500 daltons, preferably less than about 750, more preferably less than about 350 daltons. Exemplary classes include heterocycles, peptides, saccharides, steroids, and the like. The compounds may be modified to enhance efficacy, stability, pharmaceutical compatibility, and the like. Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carboxylic terminus, e.g. for the amino group, acylation or alkylation, and for the carboxyl group, esterification or amidification, or the like.

Candidate modulators which may be screened according to the methods described here include receptors, enzymes, ligands, regulatory factors, and structural proteins. Candidate modulators also include nuclear proteins, cytoplasmic proteins, mitochondrial proteins, secreted proteins, plasmalemma-associated proteins, serum proteins, viral antigens, bacterial antigens, protozoal antigens and parasitic antigens. Candidate modulators additionally comprise proteins, lipoproteins, glycoproteins, phosphoproteins and nucleic acids (e.g., RNAs such as ribozymes or antisense nucleic acids). Proteins or polypeptides which can be screened using the methods of the present invention include hormones, growth factors, neurotransmitters, enzymes, clotting factors, apolipoproteins, receptors, drugs, oncogenes, tumor antigens, tumor suppressors, structural proteins, viral antigens, parasitic antigens, bacterial antigens and antibodies (see below).

Candidate modulators which may be screened also include substances for which a test cell or organism might be deficient or that might be clinically effective in higher-than-normal concentration as well as those that are designed to eliminate the translation of unwanted proteins. Nucleic acids of use not only may encode the candidate modulators described above, but may eliminate or encode products which eliminate deleterious proteins. Such nucleic acid sequences are antisense RNA and ribozymes, as well as DNA expression constructs that encode them. Note that antisense RNA molecules, ribozymes or genes encoding them may be administered to a test cell or organism by a method of nucleic acid delivery that is known in the art, as described below. Inactivating nucleic acid sequences may encode a ribozyme or antisense RNA specific for the a target mRNA. Ribozymes of the hammerhead class are the smallest known, and lend themselves both to *in vitro* synthesis and delivery to cells (summarized by Sullivan, 1994, J. Invest. Dermatol., 103: 85S-98S; Usman et al., 1996, Curr. Opin. Struct. Biol., 6: 527-533).

As stated above, antibodies are of use in the assays as modulators (specifically, as inhibitors) of protein-modifying enzymes. Methods for the preparation of antibodies are well known in the art, and are briefly summarized as follows:

Either recombinant proteins or those derived from natural sources can be used to generate antibodies using standard techniques, well known to those in the field. For example, the proteins are administered to challenge a mammal such as a monkey, goat, rabbit or mouse. The resulting antibodies can be collected as polyclonal sera, or antibody-producing cells from the challenged animal can be immortalized (e.g. by fusion with an immortalizing fusion partner) to produce monoclonal antibodies.

1. Polyclonal antibodies.

The antigen protein may be conjugated to a conventional carrier in order to increases its immunogenicity, and an antiserum to the peptide-carrier conjugate is raised. Coupling of a peptide to a carrier protein and immunizations may be performed as described (Dymecki et al., 1992, J. Biol. Chem., 267: 4815-4823). The serum is titered against protein antigen by ELISA (below) or alternatively by dot or spot blotting (Boersma and Van Leeuwen, 1994, J. Neurosci.

Methods, 51: 317). At the same time, the antiserum may be used in tissue sections prepared as described below. The serum is shown to react strongly with the appropriate peptides by ELISA, for example, following the procedures of Green et al. , 1982, Cell, 28: 477-487.

2. Monoclonal antibodies.

Techniques for preparing monoclonal antibodies are well known, and monoclonal antibodies may be prepared using a candidate antigen whose level is to be measured or which is to be either inactivated or affinity-purified, preferably bound to a carrier, as described by Arnheiter et al., Nature, 294, 278-280 (1981).

Monoclonal antibodies are typically obtained from hybridoma tissue cultures or from ascites fluid obtained from animals into which the hybridoma tissue is introduced. Nevertheless, monoclonal antibodies may be described as being "raised to" or "induced by" a protein.

Monoclonal antibody-producing hybridomas (or polyclonal sera) can be screened for antibody binding to the target protein. By antibody, we include constructions using the binding (variable) region of such an antibody, and other antibody modifications. Thus, an antibody useful in the methods may comprise a whole antibody, an antibody fragment, a polyfunctional antibody aggregate, or in general a substance comprising one or more specific binding sites from an antibody. The antibody fragment may be a fragment such as an Fv, Fab or F(ab')$_2$ fragment or a derivative thereof, such as a single chain Fv fragment. The antibody or antibody fragment may be non-recombinant, recombinant or humanized. The antibody may be of an immunoglobulin isotype, e.g., IgG, IgM, and so forth. In addition, an aggregate, polymer, derivative and conjugate of an immunoglobulin or a fragment thereof can be used where appropriate.

Determination of activity of candidate modulator of a protein-modifying enzyme

A candidate modulator of the activity of a protein-modifying enzyme may be assayed as described herein, is determined to be effective if its use results in a difference of about 10% or greater relative to controls in which it is not present (see below) in FRET resulting from the association of labeled polypeptides comprising coiled-coils in the presence of a protein-modifying enzyme.

The level of activity of a candidate modulator may be quantified using any acceptable limits, for example, via the following formula:

$$\text{Percent Modulation} = \frac{(\text{Index}_{\text{Control}} - \text{Index}_{\text{Sample}})}{(\text{Index}_{\text{Control}})} \times 100$$

where Index$_{\text{Control}}$ is the quantitative result (e.g., amount of FRET, rate of change in FRET) obtained in assays that lack the candidate inhibitor (in other words, untreated controls), and Index$_{\text{Sample}}$ represents the result of the same measurement in assays containing the candidate inhibitor. As described below, control measurements are made with differentially labeled polypeptides comprising coiled-coils only and with these molecules plus a protein-modifying enzyme which recognizes a site present on them.

Such a calculation is used in either *in vitro* or *in vivo* assays performed according to the invention.

B. *In vivo* assays of enzymatic activity

Reporter group protein modification in living cells

Differentially-labeled polypeptides comprising coiled-coils are delivered (e.g., by microinjection) to cells, such as smooth muscle cells (DDT1) or ventricular cardiac myocytes as previously described (Riabowol et al., 1988, Cold Spring Harbor Symposia on Quantitative Biology, 53: 85-90). The ratio of emission from the labeled molecule(s) is measured as described above *via* a photomultiplier tube focused on a single cell. Activation of a kinase (e.g., PKA by the addition of dibutyryl cAMP or β-adrenergic agonists) is performed, subsequent inhibition is performed by removal of stimulus and by addition of a suitable antagonist (e.g., cAMP antagonist Rp-cAMPS). As described elsewhere herein, an ADP ribosylating enzyme may be stimulated with cholera toxin (G-protein recognition feature) or with brefeldin A.

Heterologous expression of peptides

Polypepitides comprising coiled-coils can be synthesized from the heterologous expression of DNA sequences for coiled-coil motifs of interest modified to include the sequence for enzmyatic modification as appropriate, or synthetic gene of the same. Expression can be in procaryotic or eukaryotic cells using a variety of plasmid vectors capable of instructing heterologous expression. Purification of these products is achieved by destruction of the cells (e.g. French Press) and chromatographic purification of the products. This latter procedure can be simplified by the inclusion of an affinity purification tag at one extreme of the peptide, separated from the peptide by a protease cleavage site if necessary.

The use of cells or whole organisms

When performed using cells, the assays described here are broadly applicable to a host cell susceptible to transfection or transformation including, but not limited to, bacteria (both gram-positive and gram-negative), cultured- or explanted plant (including, but not limited to, tobacco, arabidopsis, carnation, rice and lentil cells or protoplasts), insect (e.g., cultured *Drosophila* or moth cell lines) or vertebrate cells (e.g., mammalian cells) and yeast.

Organisms are currently being developed for the expression of agents including DNA, RNA, proteins, non-proteinaceous compounds, and viruses. Such vector microorganisms include bacteria such as *Clostridium* (Parker et al., 1947, Proc. Soc. Exp. Biol. Med., 66: 461-465; Fox et al., 1996, Gene Therapy, 3: 173-178; Minton et al., 1995, FEMS Microbiol. Rev., 17: 357-364), *Salmonella* (Pawelek et al., 1997, Cancer Res., 57: 4537-4544; Saltzman et al., 1996, Cancer Biother. Radiopharm., 11: 145-153; Carrier et al., 1992, J. Immunol., 148: 1176-1181; Su et al., 1992, Microbiol. Pathol., 13: 465-476; Chabalgoity et al., 1996, Infect. Immunol., 65: 2402-2412), *Listeria* (Schafer et al., 1992, J. Immunol . , 149: 53-59; Pan et al., 1995, Nature Med., 1: 471-477) and *Shigella* (Sizemore et al., 1995, Science, 270: 299-302), as well as yeast, mycobacteria, slime molds (members of the taxa Dictyosteliida - such as of the genera *Polysphondylium* and *Dictystelium,* e.g. *Dictyostelium discoideum -* and Myxomycetes - e.g. of the genera *Physarum* and *Didymium)* and members of the Domain Arachaea (including, but not limited to, archaebacteria), which have begun to be used in recombinant nucleic acid work, members of the phylum Protista, or other cell of the algae, fungi, or any cell of the animal or plant kingdoms.

Plant cells useful in expressing polypeptides of use in the assays include, but are not limited to, tobacco *(Nicotiana plumbaginifolia* and *Nicotiana tabacum),* arabidopsis *(Arabidopsis thaliana), Aspergillus niger, Brassica napus, Brassica nigra, Datura innoxia, Vicia narbonensis, Vicia faba,* pea *(Pisum sativum),* cauliflower, carnation and lentil *(Lens culinaris).* Either whole plants, cells or protoplasts may be transfected with a nucleic acid of choice. Methods for plant cell transfection or stable transformation include inoculation with *Agrobacterium tumefaciens* cells carrying the construct of interest (see, among others, Turpen et al., 1993, J. Virol. Methods, 42: 227-239), administration of liposome-associated nucleic acid molecules (Maccarrone et al., 1992, Biochem. Biophys. Res. Commun., 186: 1417-1422) and microparticle injection (Johnston and Tang, 1993, Genet. Eng. (NY). 15: 225-236), among other methods. A generally useful plant transcriptional control element is the cauliflower mosaid virus (CaMV) 35S promoter (see, for example, Saalbach et al., 1994, Mol. Gen. Genet., 242: 226-236). Non-limiting examples of nucleic acid vectors useful in plants include pGSGLUC1 (Saalbach et al., 1994, supra), pGA492 (Perez et al., 1989, Plant Mol. Biol., 13: 365-373), pOCA18 (Olszewski et al., 1988, Nucleic Acids Res., 16: 10765-10782), the Ti plasmid (Roussell et al., 1988, Mol. Gen. Genet., 211: 202-209) and pKR612B1 (Balazs et al., 1985, Gene, 40: 343-348).

Mammalian cells are of use in the detection methods. Such cells include, but are not limited to, neuronal cells (those of both primary explants and of established cell culture lines) cells of the immune system (such as T-cells, B-cells and macrophages), fibroblasts, hematopoietic cells and dendritic cells. Using established technologies, stem cells (e.g. hematopoietic stem cells) may be used for gene transfer after enrichment procedures. Alternatively, unseparated hematopoietic cells and stem cell populations may be made susceptible to DNA uptake. Transfection of hematopoietic stem cells is described in Mannion-Henderson et al., 1995, Exp. Hematol., 23: 1628; Schiffmann et al., 1995, Blood, 86: 1218; Williams, 1990, Bone Marrow Transplant, 5: 141; Boggs, 1990, Int. J. Cell Cloning. 8: 80; Martensson et al., 1987, Eur. J. Immunol., 17: 1499; Okabe et al., 1992, Eur. J. Immunol., 22: 37-43; and Banerji et al., 1983, Cell. 33: 729. Such methods may advantageously be used.

ii. Nucleic acid vectors for the expression of assay components in cells or multicellular organisms

A nucleic acid of use according to the methods described here may be either double- or single stranded and either naked or associated with protein, carbohydrate, proteoglycan and/or lipid or other molecules. Such vectors may contain modified and/or unmodified nucleotides or ribonucleotides. In the event that the gene to be transfected may be without its native transcriptional regulatory sequences, the vector must provide such sequences to the gene, so that it can be expressed once inside the target cell. Such sequences may direct transcription in a tissue-specific manner, thereby limiting expression of the gene to its target cell population, even if it is taken up by other surrounding cells. Alternatively, such sequences may be general regulators of transcription, such as those that regulate housekeeping genes, which will allow for expression of the transfected gene in more than one cell type; this assumes that the majority of vector molecules will associate preferentially with the cells of the tissue into which they were injected, and that leakage of the vector into other cell types will not be significantly deleterious to the recipient mammal. It is also possible to design a vector that will express the gene of choice in the target cells at a specific time, by using an inducible promoter, which will not direct transcription unless a specific stimulus, such as heat shock, is applied.

A gene encoding a component of the assay system or a candidate modulator of protein-modifying enzyme activity may be transfected into a cell or organism using a viral or non-viral DNA or RNA vector, where non-viral vectors include, but are not limited to, plasmids, linear nucleic acid molecules, artificial chromomosomes and episomal vectors. Expression of heterologous genes in mammals has been observed after injection of plasmid DNA into muscle (Wolff J. A. et al., 1990, Science, 247: 1465-1468; Carson D.A. et al., US Patent No. 5,580,859), thyroid (Sykes et al., 1994, Human Gene Ther., 5: 837-844), melanoma (Vile et al., 1993, Cancer Res., 53: 962-967), skin (Hengge et a]., 1995, Nature Genet., 10: 161-166), liver (Hickman et al., 1994, Human Gene Therapy, 5: 1477-1483) and after exposure of airway epithelium (Meyer et al., 1995, Gene Therapy, 2: 450-460).

In addition to vectors of the broad classes described above and the polypeptide comprising a coiled-coil/fluorescent protein fusion gene expression constructs described above (see "Fluorescent resonance energy transfer"), microbial plasmids, such as those of bacteria and yeast, are of use.

Bacterial plasmids:

Of the frequently used origins of replication, pBR322 is useful, and pUC is preferred. Although not preferred, other plasmids which are useful are those which require the presence of plasmid encoded proteins for replication, for example, those comprising pT181, FII, and FI origins of replication.

Examples of origins of replication which are useful in assays in *E. coli* and *S. typhimurium* include but are not limited to, pHETK (Garapin et al., 1981, Proc. Natl. Acad. Sci. U.S.A., 78: 815-819), p279 (Talmadge et al., 1980, Proc. Natl. Acad. Sci. U.S.A., 77: 3369-3373), p5-3 and p21A-2 (both from Pawalek et al., 1997, Cancer Res., 57: 4537-4544), pMB1 (Bolivar et al., 1977, Gene, 2: 95-113), ColE1 (Kahn et al., 1979, Methods Enzymol., 68: 268-280), p15A (Chang et al., 1978, J. Bacteriol., 134: 1141-1156); pSC101 (Stoker et al., 1982, Gene, 18: 335-341); R6K (Kahn et al., 1979, supra); R1 (temperature dependent origin of replication, Uhlin et al., 1983, Gene, 22: 255-265); lambda dv (Jackson et al., 1972, Proc. Nat. Aca. Sci. U.S.A., 69: 2904-2909); pYA (Nakayama et al., 1988, infra). An example of an origin of replication that is useful in Staphylococcus is pT181 (Scott, 1984, Microbial Reviews 48: 1-23). Of the above-described origins of replication, pMB1, p15A and ColE1 are preferred because these origins do not require plasmid-encoded proteins for replication.

Yeast plasmids:

Three systems are used for recombinant plasmid expression and replication in yeasts:

1. Integrating. An example of such a plasmid is YIp, which is maintained at one copy *per* haploid genome, and is inherited in Mendelian fashion. Such a plasmid, containing a gene of interest, a bacterial origin of replication and a selectable gene (typically an antibiotic-resistance marker), is produced in bacteria. The purified vector is linearized within the selectable gene and used to transform competent yeast cells. Regardless of the type of plasmid used, yeast cells are typically transformed by chemical methods (e.g. as described by Rose et al., 1990, Methods in Yeast Genetics, Cold Spring Harbor Laboratory Press, Cold Spring Harbor, NY). The cells are treated with lithium acetate to achieve transformation efficiencies of approximately $10^4$ colony-forming units (transformed cells)/ig of DNA. Yeast perform homologous recombination such that the cut, selectable marker recombines with the mutated (usually a point mutation or a small deletion) host gene to restore function. Transformed cells are then isolated on selective media.

2. Low copy-number ARS-CEN, of which YCp is an example. Such a plasmid contains the autonomous replicating sequence (ARS1), a sequence of approximately 700 bp which, when carried on a plasmid, permits its replication in yeast, and a centromeric sequence (CEN4), the latter of which allows mitotic stability. These are usually present at 1-2 copies per cell. Removal of the CEN sequence yields a YRp plasmid, which is typically present in 100-200 copes *per* cell; however, this plasmid is both mitotically and meiotically unstable.

3. High-copy-number 2μ circles. These plasmids contain a sequence approximately 1 kb in length, the 2μ sequence, which acts as a yeast replicon giving rise to higher plasmid copy number; however, these plasmids are unstable and require selection for maintenance. Copy number is increased by having on the plasmid a selection gene operatively linked to a crippled promoter. This is usually the LEU2 gene with a truncated promoter (LEU2-d), such that low levels of the Leu2p protein are produced; therefore, selection on a leucine-depleted medium forces an increase in copy number in order to make an amount of Leu2p sufficient for cell growth.

As suggested above, examples of yeast plasmids useful include the YRp plasmids (based on autonomously-replicating sequences, or ARS) and the YEp plasmids (based on the $2\mu$ circle), of which examples are YEp24 and the YEplac series of plasmids (Gietz and Sugino, 1988, Gene, 74: 527-534). (See Sikorski, "Extrachromsomoal cloning vectors of Saccharomyces cerevisiae", in Plasmids, A Practical Approach, Ed. K.G. Hardy, IRL Press, 1993; and Yeast Cloning Vectors and Genes, Current Protocols in Molecular Biology, Section II, Unit 13.4, Eds. , Ausubel et al., 1994).

In addition to a yeast origin of replication, yeast plasmid sequences typically comprise an antibiotic resistance gene, a bacterial origin of replication (for propagation in bacterial cells) and a yeast nutritional gene for maintenance in yeast cells. The nutritional gene (or "auxotrophic marker") is most often one of the following (with the gene product listed in parentheses and the sizes quoted encompassing the coding sequence, together with the promoter and terminator elements required for correct expression):

*TRP1* (PhosphoADP-ribosylanthranilate isomerase, which is a component of the tryptophan biosynthetic pathway).
*URA3* (Orotidine-5'-phosphate decarboxylase, which takes part in the uracil biosynthetic pathway).
*LEU2* (3-Isopropylmalate dehydrogenase, which is involved with the leucine biosynthetic pathway).
*HIS3* (Imidazoleglycerolphosphate dehydratase, or IGP dehydratase).
*LYS2* (á-aminoadipate-semialdehyde dehydrogenase, part of the lysine biosynthetic pathway).

Alternatively, the screening system may operate in an intact, living multicellular organism, such as an insect or a mammal. Methods of generating transgenic *Drosophila,* mice and other organisms, both transiently and stably, are well known in the art; detection of fluorescence resulting from the expression of Green Fluorescent Protein in live *Drosophila* is well known in the art. One or more gene expression constructs encoding one or more of a labeled polypeptide comprising a coiled-coil, a protein-modifiying enzyme and, optionally, a candidate modulator thereof are introduced into the test organism by methods well known in the art (see also below). Sufficient time is allowed to pass after administration of the nucleic acid molecule to allow for gene expression, for dimerization of polypeptides comprising a coiled-coil and for chromophore maturation, if necessary (e.g., Green Fluorescent Protein matures over a period of approximately 2 hours prior to fluorescence) before FRET is measured. A reaction component (particularly a candidate modulator of enzyme function) which is not administered as a nucleic acid molecule may be delivered by a method selected from those described below.

### Alternative fluorescence output - monomer:excimer fluorescence

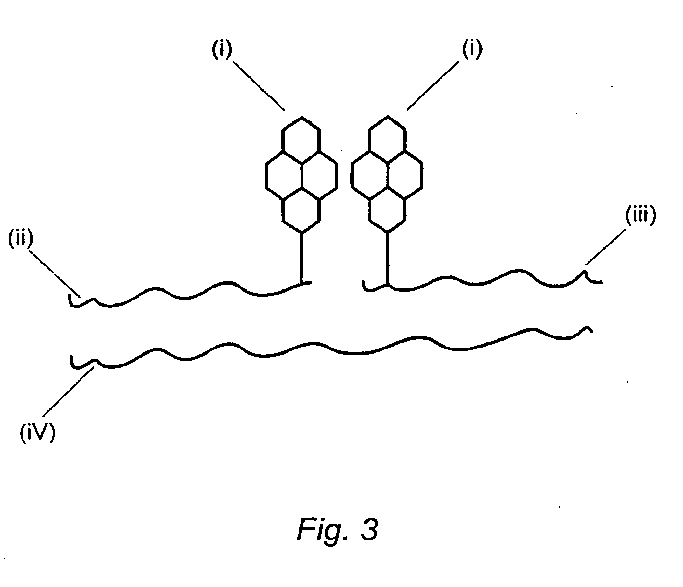

A second embodiment of the technology can utilize monomer:excimer fluorescence as the output. The assembly of polypeptides comprising a coiled-coil motif in this format is shown in Figure 3.

The fluorophore pyrene when present as a single copy displays fluorescent emission of a particular wavelength significantly shorter than when two copies of pyrene form a planar dimer (excimer), as depicted. As above, excitation at a single wavelength (probably 340nm) is used to review the excimer fluorescence (~ 470nm) over monomer fluorescence (~375nm) to quantify assembly:disassembly of the reporter molecule.

### Dosage and administration of a labeled polypeptide comprising a coiled-coil, protein-modifying enzyme or candidate modulator thereof for use in an *in vivo* assay

i. Dosage

When the amount of a protein, nucleic acid or other agent to be administered to a test cell or animal is considered, it will be apparent to those of skill in the art that the effective amount of a composition administered will depend, *inter alia,* upon the efficiency of cellular uptake of a composition, the administration schedule, the unit dose administered, whether the compositions are administered in combination with other agents, the health of the recipient, and the biological activity of the particular composition.

The precise amount of a protein, nucleic acid or agent to be delivered as a component of an assay system (and/or, if an effective modulator of enzymatic activity is uncovered, administered to an organism, such as a human, in which it is desired to modulate the activity of the enzyme influenced by that modulator) depends on the judgment of one of skill in the art and may be peculiar to each subject or cell-based test system, within a limited range of values. For example, the amount of each labeled polypeptide species comprising a coiled-coil must fall within the detection limits of the fluorescence-measuring device employed. The amount of an enzmye or candidate modulator thereof will typically be in the range of about $1\mu g$ - 100 mg/kg body weight. Where the candidate modulator is a peptide or polypeptide, it is typically administered in the range of about 100 - 500 ig/ml per dose. A single dose of a candidate modulator, or multiple doses of such a substance, daily, weekly, or intermittently, is contemplated.

A candidate modulator is tested in a concentration range that depends upon the molecular weight of the molecule and the type of assay. For example, for inhibition of protein/protein or protein/DNA complex formation or transcription initiation (depending upon the level at which the candidate modulator is thought or intended to modulate the activity of a protein-modifying enzyme), small molecules (as defined above) may be tested in a concentration range of 1pg - 100 ig/ml, preferably at about 100 pg - 10 ng/ml; large molecules, e.g., peptides, may be tested in the range of 10 ng - 100 ig/ml, preferably 100 ng - 10 ig/ml.

Generally, nucleic acid molecules are administered in a manner compatible with the dosage formulation, and in such amount as will be effective. In the case of a recombinant nucleic acid comprising a labeled polypeptide comprising a coiled-coil, such an amount should be sufficient to result in production of a detectable amount of the labeled protein or peptide, as discussed above. In the case of a modifying enzyme, the amount produced by expression of a nucleic acid molecule should be sufficient to ensure that at least 10% of coiled-coils will undergo modification if they comprise a target site recognized by the enzyme being assayed. Lastly, the amount of a nucleic acid encoding a candidate modulator of a modifying enzyme must be sufficient to ensure production of an amount of the candidate modulator which can, if effective, produce a change of at least 10% in the effect of the target modifying enzyme on FRET resulting from dimerization of coiled-coils comprised by polypeptides comprising a coiled-coil or, if administered to a patient, an amount which is prophylactically and/or therapeutically effective.

When the end product (e.g. an antisense RNA molecule or ribozyme) is administered directly, the dosage to be administered is directly proportional to the amount needed *per* cell and the number of cells to be transfected, with a correction factor for the efficiency of uptake of the molecules. In cases in which a gene must be expressed from the nucleic acid molecules, the strength of the associated transcriptional regulatory sequences also must be considered in calculating the number of nucleic acid molecules *per* target cell that will result in adequate levels of the encoded product. Suitable dosage ranges are on the order of, where a gene expression construct is administered, 0.5- to lig, or 1- 10ig, or optionally 10- 100 ig of nucleic acid in a single dose. It is conceivable that dosages of up to 1mg may be advantageously used. Note that the number of molar equivalents *per* cell vary with the size of the construct, and that absolute amounts of DNA used should be adjusted accordingly to ensure adequate gene copy number when large constructs are injected.

If no effect (e. g. , of a modifying enzyme or an inhibitor thereof) is seen within four orders of magnitude in either direction of the starting dosage, it is likely that a modifying enzyme does not recognize the target site present on the coiled-coils comprised by polypeptides comprising a coiled-coil, or that the candidate modulator thereof is not of use. It is critical to note that when high dosages are used, the concentration must be kept below harmful levels, which may be known if an enzyme or candidate modulator is a drug that is approved for clinical use. Such a dosage should be one (or, preferably, two or more) orders of magnitude below the $LD_{50}$ value that is known for a laboratory mammal, and preferably below concentrations that are documented as producing serious, if non-lethal, side effects. If it is determined that an enzyme or candidate modulator is optimally useful at levels that are harmful if achieved systemically, that agent should be used for local administration only, and then only at such doses where diffusion of the agent from the target site reduces its concentration to safe levels.

ii. Administration

Components of screening assays may be formulated in a physiologically acceptable diluent such as water, phosphate buffered saline, or saline, and further may include an adjuvant. Adjuvants such as incomplete Freund's adjuvant, aluminum phosphate, aluminum hydroxide, or alum are materials well known in the art. Administration of labeled polypeptides comprising coiled-coils, a protein-modifying enzyme or a candidate modulator as described herein may be either localized or systemic.

Localized adminstration:

It is contemplated that global administration of a component of an assay system to an animal is not needed in order to achieve a highly localized effect. Localized administration of a nucleic acid is preferably by via injection or by means of a drip device, drug pump or drug-saturated solid matrix from which the nucleic acid can diffuse implanted at the target site. When a tissue that is the target of delivery is on a surface of an organism, topical administration of a pharmaceutical composition is possible. For example, antibiotics are commonly applied directly to surface wounds as an alternative to oral or intravenous administration, which methods necessitate a much higher absolute dosage in order to counter the effect of systemic dilution, resulting both in possible side-effects in otherwise unaffected tissues and in increased cost.

Compositions comprising a composition of- or of use in the methods which are suitable for topical administration can take one of several physical forms, as summarized below:

(i) A liquid, such as a tincture or lotion, which may be applied by pouring, dropping or "painting" (i.e. spreading manually or with a brush or other applicator such as a spatula) or injection.

(ii) An ointment or cream, which may be spread either manually or with a brush or other applicator (e.g. a spatula), or may be extruded through a nozzle or other small opening from a container such as a collapsible tube.

(iii) A dry powder, which may be shaken or sifted onto the target tissue or, alternatively, applied as a nebulized spray.

(iv) A liquid-based aerosol, which may be dispensed from a container selected from the group that comprises pressure-driven spray bottles (such as are activated by squeezing), natural atomizers (or "pump-spray" bottles that work without a compressed propellant) or pressurized canisters.

(v) A carbowax or glycerin preparation, such as a suppository, which may be used for rectal or vaginal administration of a therapeutic composition.

In a specialized instance, the tissue to which a candidate modulator of a modifying enzyme is to be delivered for assay (or, if found effective, for therapeutic use) is the lung. In such a case the route of administration is *via* inhalation, either of a liquid aerosol or of a nebulized powder of. Drug delivery by inhalation, whether for topical or systemic distribution, is well known in the art for the treatment of asthma, bronchitis and anaphylaxis. In particular, it has been demonstrated that it is possible to deliver a protein *via* aerosol inhalation such that it retains its native activity *in vivo* (see Hubbard et al., 1989, J. Clin. Invest., 84: 1349-1354).

Systemic administration:

Systemic administration of a protein, nucleic acid or other agent may be performed by methods of whole-body drug delivery are well known in the art. These include, but are not limited to, intravenous drip or injection, subcutaneous, intramuscular, intraperitoneal, intracranial and spinal injection, ingestion *via* the oral route, inhalation, trans-epithelial diffusion (such as *via* a drug-impregnated, adhesive patch) or by the use of an implantable, time-release drug delivery device, which may comprise a reservoir of exogenously-produced protein, nucleic acid or other material or may, instead, comprise cells that produce and secrete a polypeptide comprising a coiled-coil, protein-modifying enzyme or candidate modulator thereof. Note that injection may be performed either by conventional means (i.e. using a hypodermic needle) or by hypospray (see Clarke and Woodland, 1975, Rheumatol. Rehabil. , 14: 47-49).

Systemic administration is advantageous when the components of an assay system must be delivered to a target tissue that is widely-dispersed, inaccessible to direct contact or, while accessible to topical or other localized application, is resident in an environment (such as the digestive tract) wherein the native activity of the protein, nucleic acid or other agent might be compromised, e.g. by digestive enzymes or extremes of pH.

Components of assays, but particularly candidate modulators to be screened according to the methods, can be given in a single- or multiple dose. A multiple dose schedule is one in which a primary course of administration can include 1-10 separate doses, followed by other doses given at subsequent time intervals required to maintain and or reinforce the cellular level of the transfected nucleic acid. Such intervals are dependent on the continued need of the recipient for the candidate modulator; in the case of a nucleic acid molecule, its ability to self-replicate in a test cell if it does not become integrated into the recipient's genome and the half-life of a non-renewable nucleic acid (e.g. a molecule that will not self-replicate) are important factors to consider.

Delivery of a nucleic acid may be performed using a delivery technique selected from the group that includes, but is not limited to, the use of viral vectors and non-viral vectors, such as episomal vectors, artificial chromosomes, liposomes, cationic peptides, tissue-specific cell transfection and transplantation, administration of genes in general vectors with tissue-specific promoters, etc.

<u>EXAMPLE 1</u>

<u>Use of a polypeptide comprising a coiled-coil as a phosphorylation reporter</u>

a. Peptide modified by chemical phosphorylation

Zip 3, a polypeptide which comprises a leucine zipper motif and which has a phosphorylation site in the center of the molecule, has the following amino acid sequence:

R MKQLEDK VEELLSK TYHLENE VACLKKL VGERAAK

This sequence is derived from that of amino acids 249-281 of GCN4 (Genbank Accession No. K02205; the sequence AAK has been added to the C-terminus of that polypeptide sequence, $N_{264}$ has been changed to T and $R_{273}$ has been changed to C. The threonine residue (shown here in bold type) is the residue which is to be phosphorylated. The cysteine residue (also shown in bold type) provides the site for attachment of thiol-directed fluorescent labels. Spaces in the sequence separate the heptad motifs (a, b, c, d, e, f and g, repeated). In other experiments presented below, this sequence was adapted to contain the recognition motif for protein kinase A (PKA), positioned such that the threonine residue could be enzymatically phosphorylated. This was accomplished while still preserving the leucine zipper structure.

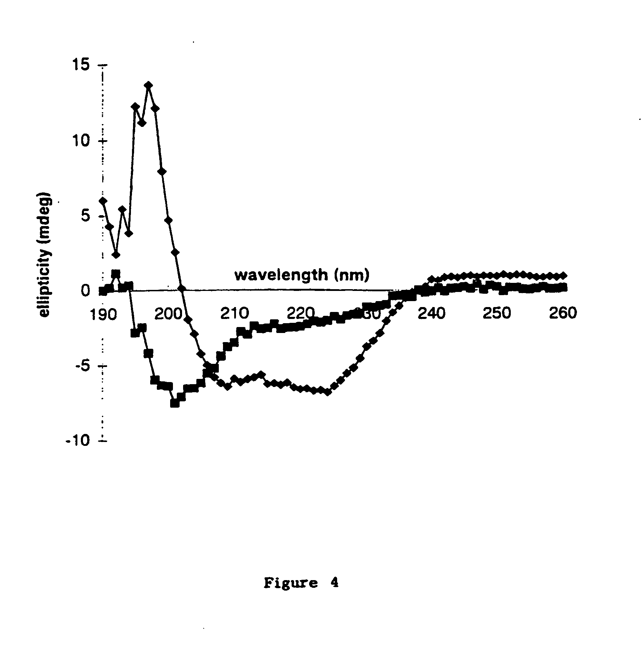

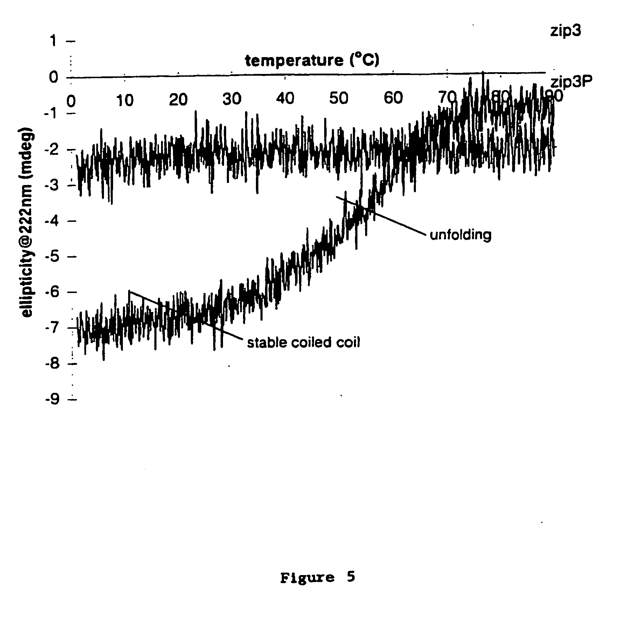

Figures 4 and 5 present data showing that phosphorylation of Zip3 at the central threonine residue destabilized the coiled-coil structure. The experiments may be summarized as follows:

The circular dichroism (measured in units of ellipticity) of proteins at 222 nm provides a measure of the amount of á-helix present in the structure, with a large, negative ellipticity indicting a high level of helicity. The coiled-coil has a distinctive á-helical CD spectrum with minima at 222 nm and 208 nm (O'Shea et al., 1989, Science, 243: 538-542). The CD spectra of the unmodified Zip3 and its phosphorylated form (Zip3P) were determined at a sample concentration of 10 iM in 150 mM KCI, 42.2 mM $K_2HPO_4$ 7.8 mM $KH_2PO_4$, pH 7.0 at 20˚C; spectra were recorded in a 1mm pathlength cell in a Jasco J-715 spectropolarimeter with a Jasco PTC-348W Peltier temperature control unit. In all experiments described herein, peptide concentration was determined by tyrosine absorbance at 280 nm in 6M GuHCl. The results are shown in Fig. 4. The spectrum of Zip3 which was observed was that of a classic coiled-coil (O'Shea et al., 1989, supra), while that of Zip3P produced by was indicative of random coil, unfolded. As shown in Figure 5, the ellipticity of Zip3 and Zip3P at 222 nm with increasing temperature was measured. The steep portion of the sigmoid curve seen for Zip3 indicated the unfolding of the molecule. It is clear that this leucine zipper peptide began to unfold at around 35˚C and was completely unfolded by 70˚C. The ellipticity of Zip3P fluctuated around a small negative value, suggesting that this structure was unfolded even at the starting temperature of 1˚C and remained unfolded at all temperatures considered.

Peptides were then labeled using a method adapted from one known in the art (Hermanson, 1997, Bioconjugate Techniques, Academic Press). 20 mM fluorescein iodoacetamide (FAM) in DMSO and 0.23 mM peptide in 20 mM TES buffer, pH 7.0 were prepared. These were mixed in a molar ratio of 0.9:1 (peptide:label) and incubated at 4˚C in the dark for a minimum of 2 hours. Initially, this method was also applied to labelling with rhodamine maleimide at a ratio of 0.9:2; however, in other experiments, good labelling has been obtained using rhodamine iodoacetamide at a ratio of 0.9:1. Labelling was assessed by reverse phase HPLC (C18 column; solvent A: $H_2O$/0.1% TFA; solvent B: acetonitrile/ 0.1% TFA) and MALDI-TOF mass spectrometry. Zip3 peptides labeled with fluorescein (Zip3F) and rhodamine (Zip3R) were thus generated.

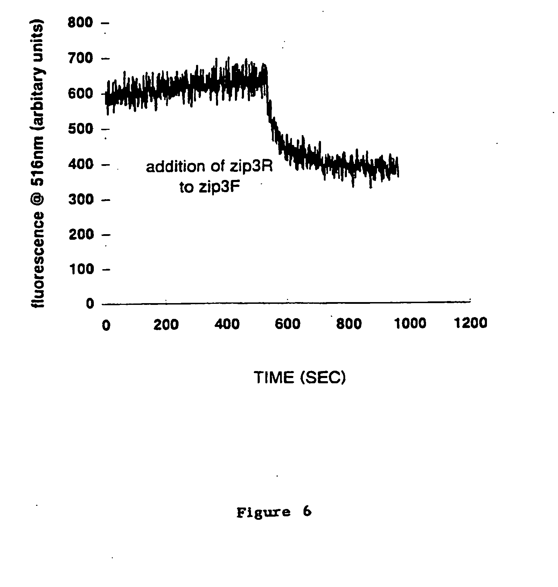

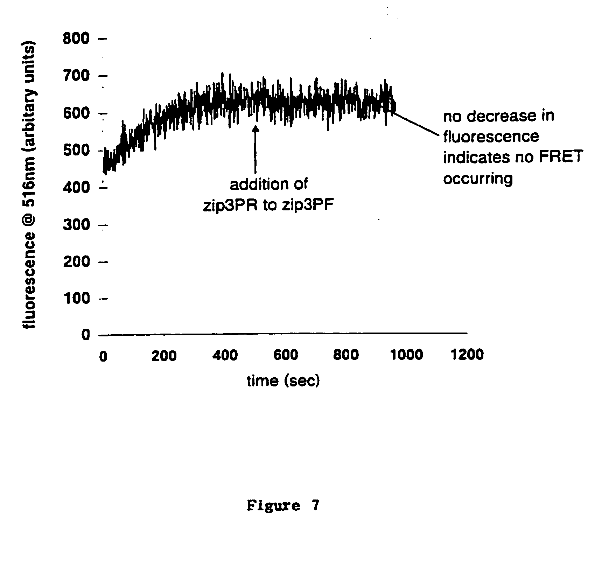

The data presented in Figures 6 and 7 demonstrate that FRET occurs between fluorophores attached to unphosphorylated peptides, which interact with each other, but not between phosphorylated peptides, which do not interact (evidenced by loss of structure in CD experiments; see Figure 4). Only the fluorescence emission quench of the donor flourophore is displayed in these figures.

Fluorescence at 516 nm was measured for labeled Zip3 proteins in a 1cm pathlength cell at peptide concentrations of 0.08 iM Zip3F and 0.48 iM Zip3R in 50 mM KCl, 42.2 mM $K_2HPO_4$, 7.8 mM $KH_2PO_4$, pH 7.0 at 37˚C in a PTI fluorimeter system with temperature controlled by a waterbath. Upon addition of Zip3R to Zip3F, fluorescence in the region of fluorescein emission decreased. This was accompanied by an increase in fluorescence detected in the rhodamine emission region (not shown). These data suggest that energy transfer was taking place and that some of the energy emitted by the fluorescein was directly exciting the rhodamine label on the partner peptide. The fluorescence at 516 nm showed no decrease upon addition of Zip3PR (the phosphorylated form of Zip3R, added at time 500 seconds) to Zip3PF (the phosphorylated form of Zip3F) (Fig. 7). In addition, no FRET was observed when Zip3F was mixed with Zip3PR (not shown), indicating that when even one peptide partner is phosphorylated, formation of the coiled-coil structure and, hence, protein:protein heterodimerization (with respect to fluorophore composition), cannot occur.

Together, these results, that FRET did not occur between the phosphorylated molecules or between a phosphorylated and an unphosphorlated molecule, indicated that FRET using labeled, polypeptides comprising a coiled-coil, may be used successfully in the methods described to report on the phosphorylation state of peptides.

EXAMPLE 2

Assaying the activity of a protein modifying enzyme

In Example 1, the suitability of fluorescently labeled, non-naturally-occurring polypeptides comprising a coiled-coil as reporters on protein phosphorylation using FRET was demonstrated. In the present Example, the tailoring of such peptides to render them functional reporters of enzymatic activity of a protein modifying enzyme (in this case, a protein kinase) is illustrated.

Peptide modified by enzymatic phorphorylation

Two variants of the peptide Zip4 (derived from amino acids 249-281 of GCN4; Genbank Accession No. K02205) were synthesized. The amino acid sequence of these peptides (Zip4S and Zip4T) were as follows:

R MKQLEDQ VRRLRRK SYHLENE VACLKKL VGERAAK (as Zip3, but also $E_{258} \rightarrow R$, $E_{259} \rightarrow R$, $L_{261} \rightarrow R$, $S_{262} \rightarrow R$ and $N_{264} \rightarrow S$), and
R MKQLEDQ VRRLRRK TYHLENE VACLKKL VGERAAK (as Zip4S, but $N_{264} \rightarrow T$).

The italicized arginine residues form the recognition site for PKA (Pearson and Kemp, 1991, Methods Enzymol., 200: 62-81).

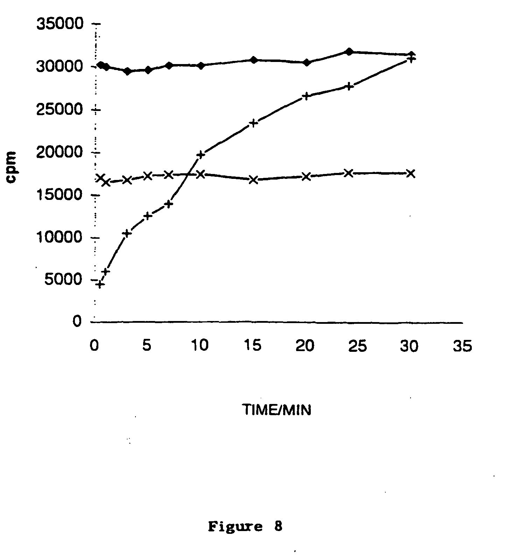

A timecourse of phosphorylation of these peptides by PKA was run in 50 mM histidine/HCl (pH 7.0), 5 mM MgSO$_4$, 5 mM NaF, 0.05 mM EGTA, 120 mM KCl with 0.2 mM ATP (30.9 cpm/pmol $^{32}$P-ATP) and 0.5iM PKA. The results, presented in Figure 8, showed that Zip 4S is a good substrate for this enzyme, with a rate of phosphorylation comparable to that seen with a known substrate (PL919Y, a phospholamban peptide; Drago and Colyer, 1994, J. Biol, Chem.: 269: 25073-25077). Zip4T is also phosphorylated, but at a slower rate, with full phosphorylation achieved in 30 minutes in this assay. In this assay, the difference in cpm recovered between the Zip4 peptides and PL919Y was due to the difference in recovery of these peptides on P81 paper; however, a plateau was taken to indicate full phosphorylation.

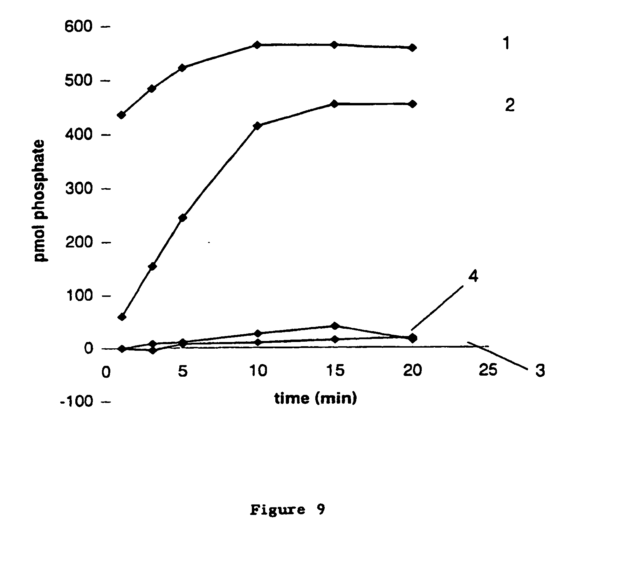

A second set of timecourses of phosphorylation was performed on Zip4S using PKA and Ca$^{2+}$/Calmodulin-dependent Protein Kinase (CaMKII), together with positive and negative controls for CaMKII (used to confirm that the crude preparation of CaMKII had the expected characteristics of that enzyme). PKA phosphorylation was performed in 50 mM histidine/HCl (pH 7.0), 5 mM MgSO$_4$, 5 mM NaF, 0.05 mM EGTA, 120 mM KCl with 0.2 mM ATP (39.72 cpm/pmol $^{32}$P-ATP) and 0.25iM PKA. CaMKII phosphorylation was performed in 50 mM histidine/HCl (pH 7.0), 5 mM MgSO$_4$, 5 mM NaF, 120 mM KCl, 0.5 mM Ca$^{2+}$, 0.037 mg/ml calmodulin with 0.2 mM ATP (39.72 cpm/pmol $^{32}$P-ATP) and 10% crude CaMKII. In the Ca$^{2+}$-free experiment, Ca$^{2+}$ was replaced by 0.05 mM EGTA. As shown in Figure 9, the results indicated that the phosphorylation site in Zip4S was recognized only by C-PKA. CaMKII was unable to phosphorylate Zip4S, while PKA mediated complete phosphorylation of that protein. Such specificity of a modification site included in a polypeptide comprising a coiled-coil is critical for use of the method in an intracellular environment.

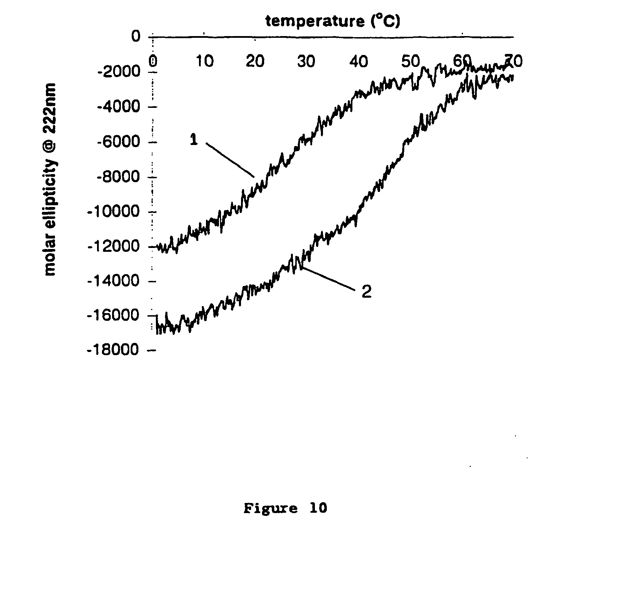

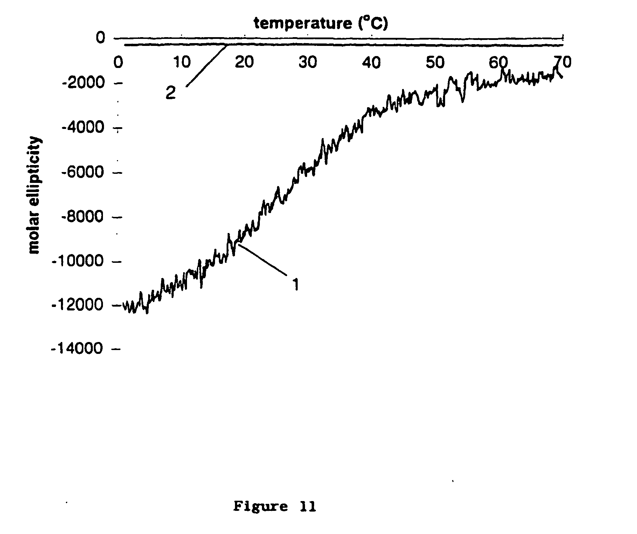

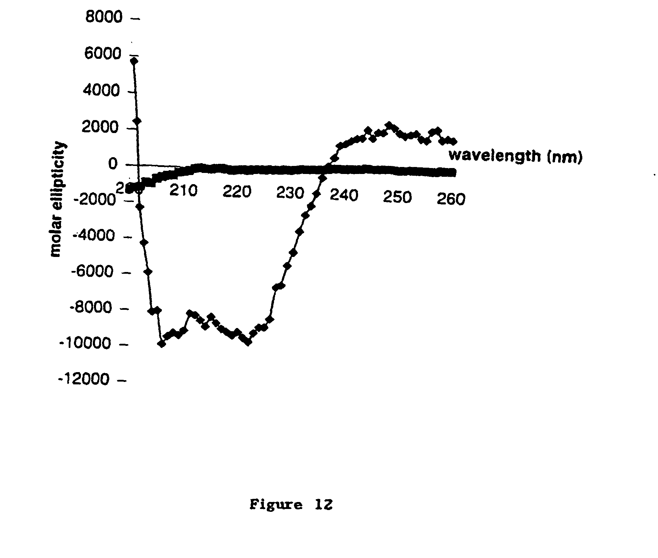

As in Example 1, circular dichroism was used to assess the coiled-coil structure of the Zip4 polypeptides and the disruption of that structure by phosphorylation. Both of the Zip4 peptides were found to be less thermostable than Zip3 (Figure 10) when assayed under the same condition (see above), which is likely due to the introduction of a positively charged region to form the PKA recognition site. Zip4T was more stable than was Zip4S (Fig. 10). Thermal denaturation, again performed as described above, of enzymatically phosphorylated Zip4S showed that this modification disrupted coiled-coil formation and led to an unfolded polypeptide in solution (Fig. 11). This was confirmed by the CD spectra of Zip4S and Zip4SP at 1°C (Fig. 12).

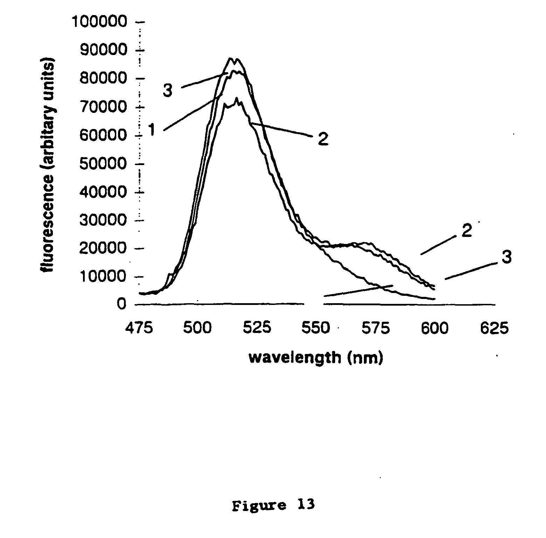

Fluorescence was used to report on the phosphorylation status of these peptides (Fig. 13). A loss of emission at 516 nm was seen on addition of Zip4SR to Zip4SF, as FRET occured when the differentially-labeled polypeptide comprising a coiled-coil partners were allowed to associate. Emission at around 575 nm also increased; while a large portion of this increase may have been attributable to direct excitation of the rhodamine label, the increase was consistently above that seen in the phosphorylated scan, suggesting that a small proportion of the increase was due to excitation by FRET. On addition of PKA, emission at 516 nm returned to above the level produced by Zip4SF alone, while emission at 573 nm decreased slightly; this decrease accounted for the amount of FRET-derived emission which was lost. No loss of FRET was observed on addition of PKA in the absence of ATP (data not shown).

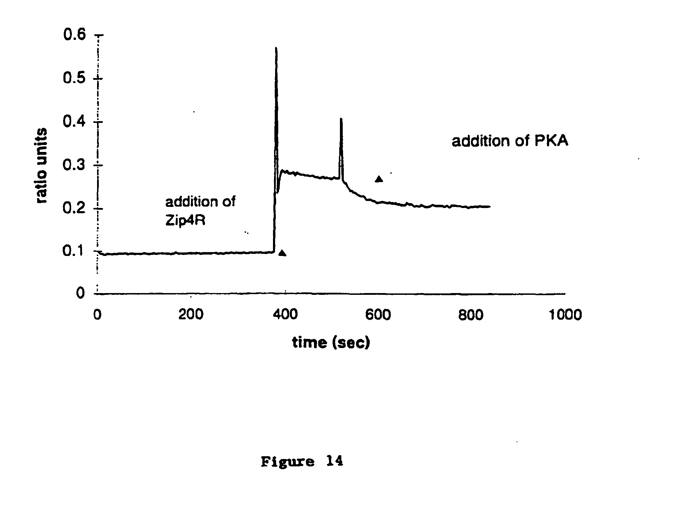

Calculation of the ratio of fluorescence output is important, particularly for *in vivo* applications, thereby avoiding error due to high local concentrations of reporter in the system and enhancing the ability to observe meaningful change. Initial inspection of the data in these experiments led to a calculation of the ratio of fluorescence outputs at 573 nm and 516 nm; as shown in Figure 14, FRET is seen as an increase in the 573/516nm ratio. A decrease was seen in the ratio following phosphorylation by PKA, although the value did not return to the baseline because of the contribution of the 573nm emission caused by direct excitation of the rhodamine label.

These several results demonstrate the applicability of the activity monitoring methods to the assessment of enzymatic activity on a target site present for that enzyme on a coiled-coil of a polypeptide comprising a coiled-coil, and further indicate that Zip4S is a suitable molecule upon which to base an assay system.

Initial unsuccessful trials

Two potential reporter peptides were synthesized. The first, Zip1, had a PKA phosphorylation site at the C-terminus while the second, Zip2s had such a site at the N-terminus (underlined).

Zip 1: HMKQLEDKVEELLSKNYCLENEVRRLRRASFSLQ

Zip 2: RRIRRASIDKVEELKSKNYCLENEVARLKKLVGER

The characterization of these peptides aimed to answer a number of questions regarding their oligomeric state, their suitability as substrates for PKA and a relevant phosphatase and the ability of phosphorylation to disrupt any oligomers formed. A number of techniques were used to assess the oligomeric state of the peptides. MALDI-TOF and electrospray mass spectrometry both yielded no useful results. Analytical ultracentrifugation gave ambiguous data (later shown to be due to the fact that the peptide dimers were unstable at the temperature used for analysis). The most successful technique used was circular dichroism (CD).

The coiled-coil motif has a characteristic CD spectrum. As described above, measurement of circular dichroism is useful to distinguish monomeric polypeptides from oligomers and to assess the stability of the structure present, although the technique cannot give information regarding the number of monomers making up the coiled-coil structure. These peptides (Zip1 and Zip2) were shown to form stable coiled-coils only at low (<20°C) temperatures. Importantly, these peptides shown only have 4.5 leucine zipper heptads, and the incomplete nature of the final heptad is likely to compromise the stability of the coiled-coil structure.

The peptides were both shown to be good substrates for PKA by reverse-phase HPLC and radioactive assay. Phosphorylated peptides were purified by HPLC and analysed by CD. The phosphorylation caused no significant change in the stability of the peptides, suggesting that neither peptide was likely to be useful as a reporter molecule. Attempts to dephosphorylate the peptides using protein phosphatase I were unsuccessful. Both peptides were successfully labeled separately with donor and acceptor fluorophores. FRET was not convincingly seen, most likely due to the unstable nature of dimers formed.

In summary, the positioning of a PKA phosphorylation site in the C-terminal one and a half heptads (target S in the 'a' position) or the N-terminal heptad (target S in the 'd' position) of a GCN4 peptide produces an unstable coiled-coil whose stability is not affected by phosphorylation of the target serine. These findings are consistent with the guidelines provided above for the placement of a protein modification site in a coiled-coil of a polypeptide comprising a coiled-coil used in an assay: Zip 2 has polar residues at 4 of 10 possible 'a' and 'd' positions (which renders it very unstable even before phosphorylation), has the phosphorylation site at a 'd' site (itself, a good design decision), but one which is outside of the central 3 heptads (thereby limiting the contribution to oligomer stability). Zip 1 incorporates two polar residues in the ten possible 'a' and 'd' positions, which destabilizes the dimer and, additionally, has its phosphorylation site outside of the three central heptads, although it is in an 'a' position. While not being bound by any theory, it is likely that the packing of the interface is less tight at these marginal heptads, and thus they are more tolerant of the altered chemistry (addition of a phosphate group in this case) than a would be comparable position in a central heptad.

EXAMPLE 3

A peptide sequence shown below is based upon the p67$^{SRF}$ glycosylation acceptor site S-316 (Reason et al., 1992, supra) and the adaptor of that sequence, which have been modified to improve their compliance with a coiled-coil sequence pattern:

```
abcdefg abcdefg abcdefg abcdefg abcdefg

     SAV SSADGTV LK                    p67SRF(313-324)



  IAALEQK IAALSAV SSDLGTV LKCLQQK IAALEQK p67SRF (313-

324), coiled-coil (Zip5).
```

In this peptide, the site of O-glycosylation is S-316 ('a' position of heptad 3), and the reporter polypeptide (Zip 5) comprising a coiled-coil has had introduced a single change in p67 sequence ($D_{319} \rightarrow L$), plus has undergone an extension of its sequence with sequence which complies with the canonical coiled-coil sequence to complete five heptads. This reporter polypeptide comprising a coiled-coil motif is expected to form stable oligomers in the absence of a glycosylated serine, which is assessed by FRET between appropriate fluorophores attached to the single Cys residue (shown in bold) in each partner peptide. Appropriate chemical fluorophores are attached to the single Cys (e.g., fluorescein and tetramethylrhodamine). O-glycosylation of Zip5 occurs upon exposure of thie peptide to a source of a protein-modifying enzyme which mediates O-GlcNAcylation of peptides (e.g, uridine diphospho-N-acetylglucosamine:peptide β N-acetylglucosaminyltransferase) and the appropriate conditions (defined in Haltiwanger et al., 1990, J. Biol. Chem., 265: 2563-2568) causes modification of the Ser residue shown in bold and, consequently, the dissociation of these polypeptides comprising coiled-coils and loss of FRET.

N-linked glycosylation assay

The N-glycosylation of Asn residues occurs within the consensus sequence NxS/T (where x is any residue other than Pro or Asp; Shakineshleman, 1996, supra) of the target protein post-translationally in the lumen of the endoplasmic reticulum and golgi apparatus, hence limiting the identity of substrate proteins to those destined for secretion and those which are bound for the cell surface or another organelle in the cell.

The coiled-coil structure readily accommodates the sequence NxS or NxT; for example, the N residue to be labeled is placed in the 'a' position of heptad 3. Thus, a candidate coiled-coil sequence modified from that of GCN4 peptide p1 (amino acids 249-280, Genbank Accession No. K02205, plus the C-terminal extension LEQK),

R MKQLEDK VEELLSK NYSLENE VACLKKL VGELEQK

R MKQLEDK VEELLSK NYTLENE VACLKKL VGELEQK

displayed, for clarity showing the heptad repeat pattern and the consensus glycosylation sequence (underlined) in each case, as well as the site of carbohydrate attachment (N) in bold. These sequences are anticipated to form homo-oligomers and also are capable of forming hetero-oligomers if combined.

The assay occurs in the endoplasmic reticulum and golgi apparatus; therefore, the molecule is translated from a nucleic acid template and includes a signal sequence to facilitate transport of the nascent chain into the ER. A proteinaceous fluorophore such as GFP (or other) is required in fusion with the coiled-coil sequence (see above) as either an N- or C-terminal extension of the sequence. A second fluorescent polypeptide comprising a coiled-coil construct is needed (again with leader sequence) to obtain a FRET measurement of coiled-coil heterodimerization. Such a pair of constructs includes:

Leader Sequence: GFP1: coiled-coil sequence, and

Leader Sequence: GFP2: coiled-coil sequence

The assay format is:

$$\text{UDP-CHO}$$
$$\text{GFPI:Coiled-coil} \rightarrow \text{GFP1:Coiled-coil-CHO} + \text{GFP2:Coiled-coil-CHO}$$
$$\text{GFP2:Coiled-coil}$$
$$\text{FRET} \qquad\qquad \text{NO FRET}$$

where CHO represents carbohydrate and UDP-CHO an activated form of the carbohydrate appropriate for enzymatic transfer to the target protein. Alternatively, a tandem fusion construct, in which the sequences of the two labeled polypeptides comprising coiled-coils are connected by a linker amino acid sequence on a single polypeptide molecule, is used.

A leader sequence useful in constructs of the glycosylation assays is provided by residues 1-20 of Folate Receptor β (Genbank Acc. No. X69516). GFP1 and GFP2 sequences are as indicated in WO 97/28261 and WO98/06737 (see SwissProt Accession No. P422I2 for GFP; other Genbank and SwissProt accessions list GFP variants of different excitation/emission wavelengths).

The time required for GFP chromophore maturation (Cubitt et al., 1995, Trends Biol. Sci., 20: 448-455) is somewhat extended (up to 2 hours). In instances in which glycosylation occurs prior to this time, a protein fluorophore with more rapid chromophore maturation properties is employed or a strategy to delay transit or processing of the reporter protein through the ER and golgi is devised.

As an alternative to the use of GFP fusion constructs, polypeptides comprising a coiled-coil motif are labeled *in vitro* by incorporating fluorescent amino acids into the nascent polypeptide chain. In such a case, GFP is not used; rather, the construct is altered to contain at least one (but, preferably, only one) lysine residue. This lysine residue is fluorescently labeled in the *in vitro* translation experiment (see above) using a pool of fluorophore-Lys:tRNA. If possible, two different fluorophore-Lys:tRNA sources are used where the fluorophores are paired for FRET. A single lysine in the reporter molecule precludes the situation of intramolecular FRET and ensures that only intermolecular FRET is observed. FRET-active pairs of fluorophore-Lys:tRNA are produced using fluorophores such as are described above. Alternatively, a non-FRET fluorescence endpoint could be used where only a single source of fluorescent amino acid is available for the *in vitro* translation experiment. In this case, numerous Lys residues are placed within the sequence of one of the partner polypeptides (see below). NBD-Lys:tRNA is a fluorescent Lys derivative used extensively in experiments to define protein synthesis and import into the ER (Crowley et al., 1993, Cell, 73: 1101-1115). This is used in an assay of polypeptide glycosylation in the following manner:

Leader Sequence: large protein: coiled-coil sequence 1, plus

Leader Sequence: coiled-coil sequence 2;

where the coiled-coil sequence 1 contains the site of glycosylation at the 'a' position of heptad 3 (as above), does not homodimerize and the entire construct is devoid of Lys residues (large protein and coiled-coil 1 sequence at least); in addition, the coiled-coil sequence 2 is lysine-rich and will not homodimerize, but will heterodimerize with coiled-coil 1, but does not contain a glycosylation site. In this situation, the incorporation of NBD-Lys occurs in the *in vitro* translation system in coiled-coil 2 only. The heterodimer forms in the absence of glycosylation, and the large size of the complex results in a protein of slow rotational movement, which can be measured by fluorescence anisotrophy techniques and fluorescence correlation spectroscopy. Glycosylation of coiled-coil 1 provokes dissociation of coiled-coil 2 from the large fusion protein, with a concomitant increase in the speed of motion of the fluorescent coiled-coil 2 protein. This is detectable by time-resolved fluorescence anisotrophy measurements and/or fluorescence correlation spectroscopy and forms the basis of an assay for N-glycosylation of proteins.

EXAMPLE 4

Detection of ADP-ribosylation

As discussed above, poly(ADP-ribose) polymerase (PARP from *Drosophila,* Genbank Accession No. D13806) is a nuclear protein capable of poly-ADP-ribosylation of protein targets which performs a control function in DNA repair, replication and other events. The enzyme is an active dimer and contains a putative leucine zipper domain (residues 385-419, Uchida et al., 1993, Proc. Natl. Acad. Sci. USA 90, 3481-3485) which is proposed to mediate protein: protein interactions. The auto-ADP-cibosylation of this enzyme has been noted in a domain containing the leucine zipper motif, which also contains two glutamic acid residues within the leucine zipper which are conserved across PARP from five species. This has raised the possibility that these glutamic acid residues represent the sites of ADP-ribosylation and perhaps control of protein:protein interactions by modulation of coiled-coil partner formation (Uchida et al., 1993).

An assay for ADP-ribosylation of proteins may comprise as the reporter peptide the Drosophilia PARP leucine zipper sequence (385-419), adapted to include a site of chemical label attachment:

abcdefg abcdefg abcdefg abcdefg abcdefg
LYNLKFS IICLKNQ HKELRKR IENLGGK FEVKISE[419]

where the sites of ADP -ribosylation are glutamic acid residues 401, 407, and where the mutation G394C has been introduce to provide an unique site of chemical fluorophore attachment. A coiled- coil dimer is predicted in the absence of ADP-ribosylation, but not in the presence of ADP-ribosylated E-401 or E407. FRET between appropriately labeled peptides of this sequence does not occur prior to ADP-ribosylation and is reduced or eliminated following ADP-ribosylation.

As this protein is normally nuclear located, the reporter peptide is best targeted to the nucleus by inclusion of a nuclear localization sequence (NLS) as an extension to the peptide sequence. Such NLS are well known to those skilled in the art.

Proteinaceous fluorophores are incorporated as extensions of the peptide sequence and serve in combination with or as replacements of chemical fluorophores. Measurements of FRET with and without a modifying enzyme and/or in the presence of a candidate modulator of the modifying enzyme are performed and quantitated as described above.

EXAMPLE 5

A kit for assaying the activity of a protein-modifying enzyme

In order to facilitate convenient and widespread use of the assay methods, a kit is provided which contains the essential components for screening for modulators of the activity of a protein-modifying enzyme, in this case of an enzyme which mediates a change in protein modification, as described above. A pair of differentially-labeled polypeptides comprising a coiled-coil motif, as defined above, is provided, as is a suitable reaction buffer for *in vitro* assay or, alternatively, cells or a cell lysate. A reaction buffer which is "suitable" is one which is permissive of the activity of the enzyme to be assayed and which permits dimerization of unmodified coiled-coil motifs. The labeled coiled-coil components are, provided as peptide/protein or a nucleic acid comprising a gene expression construct encoding the one or more of a peptide/protein, as discussed above. Polypeptides comprising coiled-coils in a kit are supplied either in solution (preferably refrigerated or frozen) in a buffer which inhibits degradation and maintains biological activity, or are provided in dried form, i.e., lyophilized. In the latter case, the components are resuspended prior to use in the reaction buffer or other biocompatible solution (e.g. water, containing one or more of physiological salts, a weak buffer, such as phophate or Tris, and a stabilizing substance such as glycerol, sucrose or polyethylene glycol); in the latter case, the resuspension buffer should not inhibit dimerization of an unmodified polypeptide comprising a coiled-coil when added to the reaction buffer in an amount necessary to deliver sufficient protein for an assay reaction. Polypeptides comprising coiled-coils provided as nucleic acids are supplied- or resuspended in a buffer which permits either transfection/transformation into a cell or organism or *in vitro* transcription/translation, as described above. Each of these components is supplied separately contained or in admixture with one or more of the others in a container selected from the group that includes, but is not limited to, a tube, vial, syringe or bottle.

Optionally, the kit includes cells. Eukaryotic or prokaryotic cells, as described above, are supplied in- or on a liquid or solid physiological buffer or culture medium (e.g. in suspension, in a stab culture or on a culture plate, e.g. a Petri dish). For ease of shipping, the cells are typically refrigerated, frozen or lyophilized in a bottle, tube or vial. Methods of cell preservation are widely known in the art; suitable buffers and media are widely known in the art, and are obtained from commerical suppliers (e.g., Gibco/LifeTechnologies) or made by standard methods (see, for example Sambrook et al. , 1989, Molecular Cloning. A Laboratory Manual., 2nd Edition, Cold Spring Harbor Laboratory Press, Cold Spring Harbor, NY).

An enzyme being assayed is added to the assay system either as a protein (isolated, partially-purified or present in a crude preparation such as a cell extract or even a living cell) or a recombinant nucleic acid. Methods of expressing a nucleic acid comprising an enzyme or other protein are well known in the art (see again above).

An assay is carried out using the kit according to the methods described above in the Examples and elsewhere.

EXAMPLE 6

A kit for screening a candidate modulator of protein-modifying enzyme activity

A candidate modulator of post-translational modification may be assayed using a kit. A kit as described in Example 5 is used for this application, with the assay performed further comprising the addition of a candidate modulator of the modifying enzyme which is present to the reaction system. Optionally, a protein-modifying enzyme is supplied with the kit, either as a protein or nucleic acid as described above.

Assays of protein activity are performed as described above. At a minimum, three detections are performed, one in which the labeled polypeptides comprising coiled-coils are present without the modifying enzyme or candidate modulator thereof (control reaction A), one in which the polypeptides comprising coiled-coils are incubated with the modifying enzyme under conditions which permit the modification reaction to occur (control reaction B) and one in which the modifying enzyme and candidate inhibitor are both incubated with the labeled polypeptides comprising coiled-coils under conditions which permit the modification reaction to occur (Test reaction). The result of the last detection procedure is compared with those of the first two controls; the candidate inhibitor is judged to be efficacious if there is a shift in either of the observed amount of FRET or the rate at which FRET changes of at least 10% away from that observed in control reaction B toward that observed in control reaction A.

USE

The methods described here are useful in monitoring the activity of a protein-modifying enzyme, whether the protein is isolated, partially-purified, present in a crude preparation or present in a living cell. The methods are further useful in assaying a cell or cell extract for the presence- or level of activity of a protein modifying enzyme. The methods are additionally useful in assaying the activity of naturally-occurring (mutant) or synthetic (engineered) isoforms of known protein modifying enzymes or, instead, that of novel (natural or synthetic) enzymes. The methods are of use in assaying the efficacy of candidate modulators of the activity of a protein modifying enzyme in inhibiting or enhancing the activity of that enzyme; moreover, is useful to screen potential therapeutic drugs for activity against cloned and/or purified enzymes that may have important clinical pathogenicities when mutated. The methods are further of use in the screening of candidate bioactive agents (e.g., drugs) for side effects, whereby the ability of such an agent to modulate the activity of a protein modifying enzyme may be indicative a propensity toward provoking unintended side-effects to a therapeutic or other regimen in which that agent might be employed.

## Claims

1. A method of monitoring the activity of an enzyme comprising the steps of:

    (a) providing a polypeptide comprising a coiled-coil and introducing a modification site into the coiled-coil of the polypeptide, the modification site being sufficient for the addition of a moiety at the site selected from the group consisting of a phosphate ($PO_4$), ubiquitin, glycosyl or ADP-ribosyl moiety, such that the polypeptide comprising the introduced modification site is capable of modification dependent binding to a binding partner via said coiled-coil in which:

        (i) addition of a moiety at the site permits association of the modified polypeptide with the binding partner, or
        (ii) addition of a moiety at the site prevents association of the modified polypeptide with the binding partner; and

    (b) detecting association between the polypeptide and the binding partner as an indication of enzyme activity.

2. A method of screening for a candidate modulator of enzymatic activity of a kinase, a phosphatase, a UDP-N-Acetylglucosamine-Dolichyl-phosphate-N-acetylsglucosamine phosphotransferase, an O-GlcNAc transferase, a ubiquitin activating enzyme E1, a ubiquitin conjugating enzyme E2, a ubiquitin protein ligase E3, a poly (ADP-ribose) polymerase and an NAD:Arginine ADP ribosyltransferase, the method comprising:

   (a) providing a polypeptide comprising a coiled-coil and introducing a modification site into the coiled-coil of the polypeptide, the modification site being sufficient for the addition of a moiety at the site selected from the group consisting of: a phosphate ($PO_4$), ubiquitin, glycosyl or ADP-ribosyl moiety, such that the polypeptide comprising the introduced modification site is capable of modification dependent binding to a binding partner via said coiled-coil in which

   (i) addition of a moiety at the site permits association of the modified polypeptide with the binding partner, or
   (ii) addition of a moiety at the site prevents association of the modified polypeptide with the binding partner;

   (b) mixing in an appropriate buffer and an appropriate amount of the polypeptide together with the binding partner; in which each of the polypeptide and the binding partner is suitably labelled with detection means for monitoring association/disassociation between same; and a sample of material whose enzymatic activity is to be tested; and
   (c) monitoring the addition or removal of at least one of the following groups: phosphate, ubiquitin, glycosyl or ADP-ribosyl group to the polypeptide, in which the addition or removal of the phosphate, ubiquitin, glycosyl or ADP-ribosyl group is indicative of modulation by the candidate modulator of the enzymatic activity.

3. A method to monitor the activity of an enzyme comprising the step of monitoring the removal of at least one of the following moieties: phosphate, ubiquitin, glycosyl or ADP-ribosyl moiety from a synthetic polypeptide comprising a coiled-coil, in which the synthetic polypeptide is synthesized such that it comprises at least one amino acid in the coiled-coil which is covalently linked to at least one of the following groups: phosphate, ubiquitin, glycosyl or ADP-ribosyl group, in which the synthetic polypeptide has at least one binding partner to which it is capable of binding via the coiled-coil, in which the binding partner:

   (i) associates with the synthetic polypeptide in the absence of the phosphate, ubiquitin, glycosyl or ADP-ribosyl moiety, or
   (ii) dissociates from the synthetic polypeptide in the absence of the phosphate, ubiquitin, glycosyl or ADP-ribosyl moiety; and

   in which the synthetic polypeptide and the binding partner therefor each comprise at least one light emitting detection means.

4. A method according to Claim 1, 2 or 3, in which the modifying enzyme is selected from the group consisting of a kinase, a phosphatase, a UDP-N-Acetylglucosamine-Dolichyl-phosphate-N-acetylsglucosamine phosphotransferase, an O-GlcNAc transferase, a ubiquitin activating enzyme E1, a ubiquitin conjugating enzyme E2, a ubiquitin protein ligase E3, a poly (ADP-ribose) polymerase and an NAD:Arginine ADP ribosyltransferase.

5. A method according to any preceding claim, in which the method comprises real-time observation of association of the isolated polypeptide and its binding partner.

6. A method according to any preceding claim, in which the polypeptide associates with the binding partner to form a dimer.

7. A method according to any preceding claim, in which the polypeptide and/or the binding partner further comprises a detection means, preferably a light emitting detection means which preferably emits light of at least a fluorescent wavelength emission.

8. A method according to any preceding claim, in which the light emitting detection means comprises two different fluorophores.

9. A method according to Claim 8, in which the method further comprises exciting the fluorophores and monitoring fluorescence emission.

10. A method according to Claim 8 or 9, in which the method comprises measuring a change in energy transfer between the polypeptide and its binding partner.

11. A method according to Claims 8, 9 or 10, in which the measuring is performed by fluorescent resonance energy transfer (FRET).

12. A method according to any of Claims 8 to 11, in which the light emitting detection means comprises a first fluorophore on the polypeptide and a second fluorophore different from the first fluorophore on the binding partner, the first and second fluorophores together being operative to promote fluorescent energy transfer (FRET).

13. A method according to any of Claims 8 to 12, in which the fluorophores comprise fluorescein and tetramethylrhodamine.

14. A method according to any of Claims 8 to 13, in which the fluorophores comprise two different fluorescent proteins.

15. A method according to Claim 14, in which the fluorophores comprise green fluorescent protein and red fluorescent protein or green fluorescent protein and blue fluorescent protein.

16. A method according to any of Claims 8 to 15, in which the polypeptide comprises a cysteine amino acid through which the light emitting means is attached via a covalent bond.

17. A method according to any preceding claim, in which the polypeptide contains two coiled-coils and is capable of self association via the two coiled-coils.

18. A method according to any preceding claim, in which the polypeptide comprises at least one contact site which binds to the binding partner, and the contact site of the polypeptide comprises the site sufficient for the addition of at least one of the following moieties: phosphate, ubiquitin, glycosyl or ADP-ribosyl moiety.

19. A method according to any preceding claim, in which the polypeptide assembles to form a coiled-coil structure and which has in its assembly region a site sufficient for at least one of the following: phosphorylation, ubiquitination, glycosylation or ADP-ribosylation.

20. A method according to any preceding claim, in which the method further comprises, prior to the step of monitoring, a step of mixing in an appropriate buffer an appropriate polypeptide concentration of the polypeptide and its binding partner labelled with an appropriate combination of fluorescence emitting means to monitor the association between the polypeptide and its binding partner.

21. A method according to Claim 20, in which the method further comprises incubating the polypeptide and its binding partner with an appropriate modifying enzyme and measuring the change in energy transfer between the polypeptide and its binding partner.

22. A method according to Claim 20, in which the method further comprises the addition to the buffer of an agent which modulates the activity of the modifying enzyme.

23. A method according to Claim 20 or 21, in which the method further comprises the addition to the buffer of an agent which modulates fluorescence emission of the reporter molecules.

24. A method according to Claim 2 or any of Claims 3 to 23 as dependent thereon, in which the candidate modulator is comprised in a library.

25. Use of a method according to any preceding claim for screening for a candidate modulator of a kinase, a phosphatase, a UDP-N-Acetylglucosamine-Dolichyl-phosphate-N-acetylsglucosamine phosphotransferase, an O-GlcNAc transferase, a ubiquitin activating enzyme E1, a ubiquitin conjugating enzyme E2, a ubiquitin protein ligase E3, a poly (ADP-ribose) polymerase or an NAD:Arginine ADP ribosyltransferase.

**Patentansprüche**

1. Verfahren zum Überwachen der Aktivität eines Enzyms, welches die folgenden Stufen umfaßt:

   (a) Bereitstellen eines Polypeptids, welches eine Coiled Coil umfaßt, und Einbringen einer Modifizierungsstelle in die Coiled Coil des Polypeptids, wobei die Modifizierungsstelle für die Hinzufügung eines Rests, ausgewählt aus der Gruppe, bestehend aus einem Phosphat- ($PO_4$), Ubiquitin-, Glycosyl- oder ADP-Ribosyl-Rest, an der Stelle ausreichend ist, so daß das die eingebrachte Modifizierungsstelle umfassende Polypeptid zu einer modifikationsabhängigen Bindung an einen Bindungspartner über die Coiled Coil in der Lage ist, wobei:

   (i) die Hinzufügung eines Rests an der Stelle die Assoziation des modifizierten Polypeptids mit dem Bindungspartner ermöglicht oder
   (ii) die Hinzufügung eines Rests an der Stelle die Assoziation des modifizierten Polypeptids mit dem Bindungspartner verhindert und

   (b) Detektieren einer Assoziation zwischen dem Polypeptid und dem Bindungspartner als ein Hinweis auf Enzymaktivität.

2. Verfahren zum Screenen hinsichtlich eines Kandidaten-Modulators für die enzymatische Aktivität einer Kinase, einer Phosphatase, einer UDP-N-Acetylglucosamin-dolichylphosphat-N-acetylglucosaminphosphotransferase, einer O-GIcNAc-Transferase, eines Ubiquitin aktivierenden Enzyms E1, eines Ubiquitin konjugierenden Enzyms E2, einer Ubiquitin-Proteinligase E3, einer Poly-(ADP-Ribose)-Polymerase und einer NAD:Arginin-ADP-Ribosyltransferase, wobei das Verfahren folgendes umfaßt:

   (a) Bereitstellen eines Polypeptids, welches eine Coiled Coil umfaßt, und Einbringen einer Modifizierungsstelle in die Coiled Coil des Polypeptids, wobei die Modifizierungsstelle für die Hinzufügung eines Rests, ausgewählt aus der Gruppe, bestehend aus einem Phosphat- ($PO_4$), Ubiquitin-, Glycosyl- oder ADP-Ribosyl-Rest, an der Stelle ausreichend ist, so daß das die eingebrachte Modifizierungsstelle umfassende Polypeptid zu einer modifikationsabhängigen Bindung an einen Bindungspartner über die Coiled Coil in der Lage ist, wobei:

   (i) die Hinzufügung eines Rests an der Stelle die Assoziation des modifizierten Polypeptids mit dem Bindungspartner erlaubt oder
   (ii) die Hinzufügung eines Rests an der Stelle die Assoziation des modifizierten Polypeptids mit dem Bindungspartner verhindert,

   (b) Mischen einer geeigneten Menge des Polypeptids mit dem Bindungspartner in einem geeigneten Puffer, wobei das Polypeptid und der Bindungspartner jeweils in geeigneter Weise mit Detektionsmitteln markiert sind, um die Assoziation/Trennung zwischen ihnen zu überwachen, und einer Probe von Material, dessen enzymatische Aktivität getestet werden soll, und
   (c) Überwachen der Hinzufügung oder der Entfernung wenigstens einer der folgenden Gruppen: der Phosphat-, Ubiquitin-, Glycosyl- oder ADP-Ribosylgruppe zu/von dem Polypeptid, wobei die Hinzufügung oder Entfernung der Phosphat-, Ubiquitin-, Glycosyl- oder ADP-Ribosylgruppe ein Hinweis auf eine Modulierung der enzymatischen Aktivität durch den Kandidaten-Modulator ist.

3. Verfahren zum Überwachen der Aktivität eines Enzyms, welches die Stufe umfaßt, bei der man die Entfernung wenigstens eines der folgenden Reste: eines Phosphat-, Ubiquitin-, Glycosyl- oder ADP-Ribosyl-Rests von einem eine Coiled-Coil umfassenden synthetischen Polypeptid überwacht, wobei das synthetische Polypeptid so synthetisiert wird, daß es wenigstens eine Aminosäure in der Coiled Coil umfaßt, die kovalent an wenigstens eine der folgenden Gruppen: Phosphat-, Ubiquitin-, Glycosyl- oder ADP-Ribosyl-Gruppen, gebunden ist, wobei das synthetische Polypeptid wenigstens einen Bindungspartner hat, an den es über die Coiled Coil binden kann, wobei der Bindungspartner:

   (i) sich in Abwesenheit des Phosphat-, Ubiquitin-, Glycosyl- oder ADP-Ribosyl-Rests mit dem synthetischen Polypeptid verknüpft oder
   (ii) sich in Abwesenheit des Phosphat-, Ubiquitin-, Glycosyl- oder ADP-Ribosyl-Rests von dem synthetischen Polypeptid trennt und

   wobei das synthetische Polypeptid und der Bindungspartner dafür jeweils wenigstens ein Licht emittierendes Detektionsmittel umfassen.

**4.** Verfahren nach Anspruch 1, 2 oder 3, wobei das modifizierende Enzym aus der Gruppe ausgewählt ist, bestehend aus einer Kinase, einer Phosphatase, einer UDP-N-Acetylglucosamin-dolichyl-phosphat-N-acetylglucosaminphosphotransferase, einer O-GlcNAc-Transferase, einem Ubiquitin aktivierenden Enzym E1, einem Ubiquitin konjugie-renden Enzym E2, Ubiquitin-Proteinligase E3, einer Poly- (ADP-Ribose-) Polymerase und einer NAD:Arginin-ADP-Ribosyltransferase.

**5.** Verfahren nach einem der vorangegangenen Ansprüche, wobei das Verfahren die Beobachtung der Assoziation des isolierten Polypeptids und seines Bindungspartners in Echtzeit umfaßt.

**6.** Verfahren nach einem der vorangegangenen Ansprüche, wobei das Polypeptid sich unter Bildung eines Dimers mit dem Bindungspartner assoziiert.

**7.** Verfahren nach einem der vorangegangenen Ansprüche, wobei das Polypeptid und/oder der Bindungspartner weiterhin ein Detektionsmittel, vorzugsweise ein Licht emittierendes Detektionsmittel umfassen, welches bevorzugt Licht mit wenigstens einer Fluoreszenzwellenlänge emittiert.

**8.** Verfahren nach einem der vorangegangenen Ansprüche, wobei das Licht emittierende Detektionsmittel zwei verschiedene Fluorophore umfaßt.

**9.** Verfahren nach Anspruch 8, wobei das Verfahren weiterhin das Anregen der Fluorophore und das Überwachen der Fluoreszenzemission umfaßt.

**10.** Verfahren nach Anspruch 8 oder 9, wobei das Verfahren das Messen einer Veränderung bei der Energieübertragung zwischen dem Polypeptid und seinem Bindungspartner umfaßt.

**11.** Verfahren nach einem der Ansprüche 8, 9 oder 10, wobei die Messung mittels Fluoreszenz-Resonanz-Energieübertragung (FRET) durchgeführt wird.

**12.** Verfahren nach einem der Ansprüche 8 bis 11, wobei das Licht emittierende Detektionsmittel einen ersten Fluorophor auf dem Polypeptid und einen zweiten Fluorophor, der sich von dem ersten Fluorophor unterscheidet, auf dem Bindungspartner umfaßt, wobei der erste und der zweite Fluorophor zusammen so wirken, daß sie die Fluoreszenz-Energieübertragung (FRET) fördern.

**13.** Verfahren nach einem der Ansprüche 8 bis 12, wobei die Fluorophore Fluorescein und Tetramethylrhodamin umfassen.

**14.** Verfahren nach einem der Ansprüche 8 bis 13, wobei die Fluorophore zwei verschiedene fluoreszierende Proteine umfassen.

**15.** Verfahren nach Anspruch 14, wobei die Fluorophore grün fluoreszierendes Protein und rot fluoreszierendes Protein oder grün fluoreszierendes Protein und blau fluoreszierendes Protein umfassen.

**16.** Verfahren nach einem der Ansprüche 8 bis 15, wobei das Polypeptid eine Cysteinaminosäure umfaßt, durch die das Licht emittierende Mittel über eine kovalente Bindung angehängt ist.

**17.** Verfahren nach einem der vorangegangenen Ansprüche, wobei das Polypeptid zwei Superhelices umfaßt und zu einer Eigenassoziation über die zwei Superhelices in der Lage ist.

**18.** Verfahren nach einem der vorangegangenen Ansprüche, wobei das Polypeptid wenigstens eine Kontaktstelle umfaßt, die an den Bindungspartner bindet, und die Kontaktstelle des Polypeptids die Stelle umfaßt, die für die Hinzufügung wenigstens eines der folgenden Reste: eines Phosphat-, Ubiquitin-, Glycosyl- oder ADP-Ribosyl-Rests, ausreichend ist.

**19.** Verfahren nach einem der vorangegangenen Ansprüche, wobei das Polypeptid sich unter Bildung einer Coiled-Coil-Struktur zusammenfügt und in der Region seiner Zusammenfügung eine Stelle aufweist, die für wenigstens eines der folgenden ausreichend ist: Phosphorylierung, Ubiquitinierung, Glycosylierung oder ADP-Ribosylierung.

**20.** Verfahren nach einem der vorangegangenen Ansprüche, wobei das Verfahren vor der Stufe des Überwachens weiterhin eine Stufe umfaßt, bei der man in einem geeigneten Puffer eine geeignete Polypeptidkonzentration des Polypeptids und seines Bindungspartners, markiert mit einer geeigneten Kombination aus Fluoreszenz emittierenden Mitteln, mischt, um die Assoziation zwischen dem Polypeptid und seinem Bindungspartner zu überwachen.

**21.** Verfahren nach Anspruch 20, wobei das Verfahren weiterhin das Inkubieren des Polypeptids und seines Bindungspartners mit einem geeigneten modifizierenden Enzym und das Messen der Veränderung der Energieübertragung zwischen dem Polypeptid und seinem Bindungspartner umfaßt.

**22.** Verfahren nach Anspruch 20, wobei das Verfahren weiterhin das Hinzufügen eines Mittels, welches die Aktivität des modifizierenden Enzyms moduliert, zu dem Puffer umfaßt.

**23.** Verfahren nach Anspruch 20 oder 21, wobei das Verfahren weiterhin das Hinzufügen eines Mittels, welches die Fluoreszenzemission der Reportermoleküle moduliert, zu dem Puffer umfaßt.

**24.** Verfahren nach Anspruch 2 oder irgendeinem der Ansprüche 3 bis 23, soweit davon abhängig, wobei der Kandidaten-Modulator in einer Bibliothek enthalten ist.

**25.** Verwendung eines Verfahrens nach einem der vorangegangenen Ansprüche zum Screenen einer Kinase, einer Phosphatase, einer UDP-N-Acetylglucosamin-dolichylphosphat-N-acetylglucosaminphosphotransferase, einer O-GlcNAc-Transferase, eines Ubiquitin aktivierenden Enzyms E1, eines Ubiquitin konjugierenden Enzyms E2, einer Ubiquitin-Proteinligase E3, einer Poly-(ADP-Ribose)-Polymerase oder einer NAD:Arginin-ADP-Ribosyltransferase hinsichtlich eines Kandidaten-Modulators.

**Revendications**

**1.** Procédé de surveillance de l'activité d'une enzyme, comprenant les étapes de :

(a) fourniture d'un polypeptide comprenant une structure bispiralée et introduction d'un site de modification dans la structure bispiralée du polypeptide, le site de modification étant suffisant pour l'addition d'un fragment au niveau du site choisi dans le groupe constitué par : un fragment de phosphate ($PO_4$), d'ubiquitine, de glycosyle ou d'ADP-ribosyle, de sorte que le polypeptide comprenant le site de modification introduit est capable de liaison dépendante de la modification à un partenaire de liaison via ladite structure bispiralée, dans lequel :

(i) l'addition d'un fragment au niveau du site permet l'association du polypeptide modifié avec le partenaire de liaison, ou
(ii) l'addition d'un fragment au niveau du site empêche l'association du polypeptide modifié avec le partenaire de liaison ; et

(b) détection de l'association entre le polypeptide et le partenaire de liaison comme indication de l'activité enzymatique.

**2.** Procédé de criblage d'un modulateur candidat de l'activité enzymatique d'une kinase, d'une phosphatase, d'une UDP-N-Acétylglucosamine-Dolichyl-phosphate-N-acétyl-glucosamine phosphotransférase, d'une O-GlcNAc transférase, d'une enzyme d'activation de l'ubiquitine E1, d'une enzyme de conjugaison de l'ubiquitine E2, d'une ubiquitine protéine ligase E3, d'une poly(ADP-ribose) polymérase et d'une NAD:Arginine ADP-ribosyltransférase, le procédé comprenant :

(a) la fourniture d'un polypeptide comprenant une structure bispiralée et l'introduction d'un site de modification dans la structure bispiralée du polypeptide, le site de modification étant suffisant pour l'addition d'un fragment au niveau du site choisi dans le groupe constitué par : un fragment de phosphate ($PO_4$), d'ubiquitine, de glycosyle ou d'ADP-ribosyle, de sorte que le polypeptide comprenant le site de modification introduit est capable de liaison dépendante de la modification à un partenaire de liaison via ladite structure bispiralée, dans lequel:

(i) l'addition d'un fragment au niveau du site permet l'association du polypeptide modifié avec le partenaire de liaison, ou
(ii) l'addition d'un fragment au niveau du site empêche l'association du polypeptide modifié avec le partenaire de liaison ;

(b) le mélange dans un tampon approprié et une quantité appropriée du polypeptide conjointement avec le partenaire de liaison ; dans lequel chacun du polypeptide et du partenaire de liaison est correctement marqué avec des moyens de détection pour surveiller l'association/la dissociation entre ceux-ci ; et un échantillon de matière dont l'activité enzymatique doit être testée ; et
(c) la surveillance de l'addition ou de l'élimination d'au moins l'un des groupes suivants: groupe phosphate, ubiquitine, glycosyle ou ADP-ribosyle par rapport au polypeptide, dans lequel l'addition ou l'élimination du groupe phosphate, ubiquitine, glycosyle ou ADP-ribosyle indique une modulation par le modulateur candidat de l'activité enzymatique.

3. Procédé de surveillance de l'activité d'une enzyme comprenant l'étape de surveillance de l'élimination d'au moins l'un des fragments suivants : fragment de phosphate, d'ubiquitine, de glycosyle ou d'ADP-ribosyle d'un polypeptide synthétique comprenant une structure bispiralée, dans lequel le polypeptide synthétique est synthétisé de telle sorte qu'il comprend au moins un acide aminé dans la structure bispiralée, qui est lié de manière covalente à au moins l'un des groupes suivants: groupe phosphate, ubiquitine, glycosyle ou ADP-ribosyle, dans lequel le polypeptide synthétique possède au moins un partenaire de liaison auquel il est capable de se lier via la structure bispiralée, dans lequel le partenaire de liaison:

(i) s'associe avec le polypeptide synthétique en l'absence du fragment phosphate, ubiquitine, glycosyle ou ADP-ribosyle, ou
(ii) se dissocie du polypeptide synthétique en l'absence du fragment phosphate, ubiquitine, glycosyle ou ADP-ribosyle ; et

dans lequel le polypeptide synthétique et le partenaire de liaison correspondant comprennent par conséquent chacun au moins un moyen de détection électroluminescent.

4. Procédé selon la revendication 1, 2 ou 3, dans lequel l'enzyme de modification est choisie dans le groupe constitué par une kinase, une phosphatase, une UDP-N-Acétylglucosamine-Dolichyl-phosphate-N-acétylglucosamine phosphotransférase, une O-GlcNAc transférase, une enzyme d'activation de l'ubiquitine E1, une enzyme de conjugaison de l'ubiquitine E2, une ubiquitine protéine ligase E3, une poly(ADP-ribose) polymérase et une NAD:Arginine ADP-ribosyltransférase.

5. Procédé selon l'une quelconque des revendications précédentes, lequel procédé comprend l'observation en temps réel de l'association du polypeptide isolé et de son partenaire de liaison.

6. Procédé selon l'une quelconque des revendications précédentes, dans lequel le polypeptide s'associe avec le partenaire de liaison pour former un dimère.

7. Procédé selon l'une quelconque des revendications précédentes, dans lequel le polypeptide et/ou le partenaire de liaison comprennent en outre un moyen de détection, de préférence un moyen de détection électroluminescent qui émet de préférence de la lumière d'au moins une émission de longueur d'onde de fluorescence.

8. Procédé selon l'une quelconque des revendications précédentes, dans lequel le moyen de détection électroluminescent comprend deux différents fluorophores.

9. Procédé selon la revendication 8, lequel procédé comprend en outre l'excitation des fluorophores et la surveillance de l'émission de fluorescence.

10. Procédé selon la revendication 8 ou 9, lequel procédé comprend la mesure d'un changement de transfert d'énergie entre le polypeptide et son partenaire de liaison.

11. Procédé selon la revendication 8, 9 ou 10, dans lequel la mesure est effectuée par transfert d'énergie de fluorescence par résonance (FRET).

12. Procédé selon l'une quelconque des revendications 8 à 11, dans lequel le moyen de détection électroluminescent comprend un premier fluorophore sur le polypeptide et un deuxième fluorophore différent du premier fluorophore sur le partenaire de liaison, les premier et deuxième fluorophores ensemble étant opérationnels pour promouvoir le transfert d'énergie de fluorescence (FRET).

13. Procédé selon l'une quelconque des revendications 8 à 12, dans lequel les fluorophores comprennent de la fluorescéine et de la tétraméthylrhodamine.

14. Procédé selon l'une quelconque des revendications 8 à 13, dans lequel les fluorophores comprennent deux différentes protéines fluorescentes.

15. Procédé selon la revendication 14, dans lequel les fluorophores comprennent la protéine fluorescente verte et la protéine fluorescente rouge ou la protéine fluorescente verte et la protéine fluorescente bleue.

16. Procédé selon l'une quelconque des revendications 8 à 15, dans lequel le polypeptide comprend un acide aminé cystéine à travers lequel le moyen électroluminescent est attaché via une liaison covalente.

17. Procédé selon l'une quelconque des revendications précédentes, dans lequel le polypeptide contient deux structures bispiralées et est capable d'auto-association via les deux structures bispiralées.

18. Procédé selon l'une quelconque des revendications précédentes, dans lequel le polypeptide comprend au moins un site de contact qui se lie au partenaire de liaison, et le site de contact du polypeptide comprend le site suffisant pour l'addition d'au moins l'un des fragments suivants : fragment phosphate, ubiquitine, glycosyle ou ADP-ribosyle.

19. Procédé selon l'une quelconque des revendications précédentes, dans lequel le polypeptide s'assemble pour former une structure bispiralée et qui possède dans sa région d'assemblage un site suffisant pour au moins l'une des réactions suivantes : phosphorylation, ubiquitination, glycosylation ou ADP-ribosylation.

20. Procédé selon l'une quelconque des revendications précédentes, lequel procédé comprend en outre, avant l'étape de surveillance, une étape de mélange dans un tampon approprié d'une concentration polypeptidique appropriée du polypeptide et de son partenaire de liaison marqué avec une combinaison appropriée de moyens d'émission de fluorescence pour surveiller l'association entre le polypeptide et son partenaire de liaison.

21. Procédé selon la revendication 20, lequel procédé comprend en outre l'incubation du polypeptide et de son partenaire de liaison avec une enzyme de modification appropriée et la mesure du changement de transfert d'énergie entre le polypeptide et son partenaire de liaison.

22. Procédé selon la revendication 20, lequel procédé comprend en outre l'addition dans le tampon d'un agent qui module l'activité de l'enzyme de modification.

23. Procédé selon la revendication 20 ou 21, lequel procédé comprend en outre l'addition dans le tampon d'un agent qui module l'émission de fluorescence des molécules rapporteuses.

24. Procédé selon la revendication 2 ou l'une quelconque des revendications 3 à 23 lorsqu'elles en dépendent, dans lequel le modulateur candidat est compris dans une banque.

25. Utilisation d'un procédé selon l'une quelconque des revendications précédentes pour cribler un modulateur candidat d'une kinase, d'une phosphatase, d'une UDP-N-Acétylglucosamine-Dolichyl-phosphate-N-acétylglucosamine phosphotransférase, d'une O-GlcNAc transférase, d'une enzyme d'activation de l'ubiquitine E1, d'une enzyme de conjugaison de l'ubiquitine E2, d'une ubiquitine protéine ligase E3, d'une poly(ADP-ribose) polymérase ou d'une NAD:Arginine ADP-ribosyltransférase.

Fig. 1a

Fig. 1b

Fig. 1c

Fig. 2

(i)          (i)

(ii)          (iii)

(iV)

*Fig. 3*

Figure 4

Figure 5

Figure 6

Figure 7

Figure 8

Figure 9

Figure 10

Figure 11

Figure 12

Figure 13

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9200388 A
- WO 9728261 A, Tsien
- WO 9806737 A
- US 9514692 W
- US 5580859 A, Carson D.A

### Non-patent literature cited in the description

- **SPATOLA.** Chemistry and Biochemistry of Amino Acids, Peptides and Proteins. Marcel Dekker, 1983, 267
- **HICKS et al.** *Folding and Design,* 1997, vol. 2, 149-158
- **CRICK.** *Acta. Crystallogr,* 1953, vol. 6, 689-697
- **GONZALEZ et al.** *Nature Structural Biology,* 1996, vol. 3, 1011-1018
- *J.BIOL.CHEM.,* 1978, vol. 253, 1137-1148
- *Trends Biol. Sci.,* 1995, vol. 20, 133-135
- *Proc. Natl. Acad. Sci. U.S.A.,* 1982, vol. 81, 6442-6446
- *Proc. Natl. Acad. Sci. U.S.A.,* vol. 80, 3183-3187
- *Proc. Natl. Acad. Sci. U.S.A.,* 1988, vol. 85, 9148-9152
- *NUCLEIC ACIDS RES.,* 1987, vol. 15, 1005-1017
- *Science,* vol. 263, 1404-141
- *Nature,* 1995, vol. 373, 636-640
- *Nature,* vol. 371, 80-83
- *Science,* vol. 259, 1288-1293
- *Proc. Natl. Acad. Sci. U.S.A.,* 1982, vol. 79, 6313-6317
- *JBC,* 1996, vol. 271, 5941-5946
- **HICKS et al.** *Folding & Design,* 1997, vol. 2, 149-158
- **WOOLFSON ; ALBER.** *Protein Sci.,* 1995, vol. 4, 1596-1607
- **HARBURY, P.B ; ZHANG, T ; KIM, P.S ; ALTER, T.** *Science,* 1993, vol. 262, 1401-1407
- **SAMBROOK et al.** Molecular Cloning: A Laboratory Manual. Cold Spring Harbor Laboratory Press, 1989
- **MATYUS.** *J. Photochem. Photobiol. B: Biol.,* 1992, vol. 12, 323-337
- **LAKOWICZ.** Principles of Fluorescence Spectroscopy. Plenum Press, 1983
- **JOVIN ; JOVIN.** Cell Structure and Function by Microspectrofluorometry. Academic Press, 1989
- **WARD et al.** *Photochem. Photobiol,* 1982, vol. 35, 803-808
- **LEVINE et al.** *Comp. Biochem. Physiol.,* 1982, vol. 72B, 77-85
- **PRASHER et al.** *Gene,* 1992, vol. 111, 229-233
- **HEIM et al.** *Proc. Natl. Acad. Sci. U.S.A.,* 1994, vol. 91, 12501-12504
- **ZOLKIEWSKA et al.** *Proc Natl. Acad. Sci. U.S.A.,* 1992, vol. 89, 11352-11356
- **DE MURCIA et al.** *Trends Cell Biol.,* 1995, vol. 5, 78-81
- **WEIGERT et al.** *J. Biol. Chem.,* 1997, vol. 272, 14200-14207
- **CERVANTES-LAUREAN et al.** *Methods Enzymol.,* 1997, vol. 280, 275-287
- **UCHIDA et al.** *Proc. Natl. Acad. Sci. U.S.A.,* 1993, vol. 90, 3481-3485
- **MENDOZA-ALVAREZ et al.** *J. Biol, Chem.,* 1993, vol. 268, 22575-22580
- **COGGINS et al.** *J. Neurochem,* 1993, vol. 60, 368-371
- **BONDARENKO et al.** *J. Biol. Chem.,* 1997, vol. 272, 15856-15864
- **VARSHAVSKY.** *Proc. Natl. Acad. Sci. U.S.A.,* 1996, vol. 93, 12142-12149
- **DAI et al.** *J. Biol. Chem,* 1998, vol. 273, 3562-3573
- **JOHNSON et al.** *Nature,* 1990, vol. 346, 287-291
- **SHAKINESHLEMAN.** *Trends Glycosci. Glycotech,* 1996, vol. 8, 115-130
- **HANSEN et al.** *Biochem. J.,* 1995, vol. 308, 801-813
- **HART.** *Ann. Rev. Biochem.,* 1997, vol. 66, 315-335
- **REASON et al.** *J. Biol. Chem.,* 1992, vol. 267, 16911-16921
- **CHOU et al.** *J. Biol. Chem.,* 1995, vol. 270, 18961-18965
- **KREPPEL et al.** *J. Biol. Chem.,* 1997, vol. 272, 9308-9315

- **PETERS et al.** *Biochemistry,* 1977, vol. 16, 5691-5697
- **SAMBROOK et al.** Molecular Cloning. A Laboratory Manual. Cold Spring Harbor Laboratory Press, 1989
- **AUSUBEL et al.** Current Protocols in Molecular Biology. John Wiley & Sons, Inc, 1987
- **ATHERTON et al.** *J. Chem. Soc. Perkin,* 1981, vol. I (2), 538-546
- **MERRIFIELD.** *J. Am. Chem. Soc.,* 1963, vol. 85, 2149-2154
- **HERMANSON G.T.** Bioconjugate Techniques. Academic Press, 1995
- **CASNELLIE.** *Methods Enzymol.,* 1991, vol. 200, 115-120
- **LAEMMLI.** *Nature,* 1970, vol. 227, 680-685
- **SULLIVAN.** *J. Invest. Dermatol,* 1994, vol. 103, 85S-98S
- **USMAN et al.** *Curr. Opin. Struct. Biol,* 1996, vol. 6, 527-533
- **DYMECKI et al.** *J. Biol. Chem.,* 1992, vol. 267, 4815-4823
- **BOERSMA ; VAN LEEUWEN.** *J. Neurosci. Methods,* 1994, vol. 51, 317
- **GREEN et al.** *Cell,* 1982, vol. 28, 477-487
- **ARNHEITER et al.** *Nature,* 1981, vol. 294, 278-280
- **RIABOWOL et al.** *Cold Spring Harbor Symposia on Quantitative Biology,* 1988, vol. 53, 85-90
- **PARKER et al.** *Proc. Soc. Exp. Biol. Med,* 1947, vol. 66, 461-465
- **FOX et al.** *Gene Therapy,* 1996, vol. 3, 173-178
- **MINTON et al.** *FEMS Microbiol. Rev,* 1995, vol. 17, 357-364
- **PAWELEK et al.** *Cancer Res.,* 1997, vol. 57, 4537-4544
- **SALTZMAN et al.** *Cancer Biother. Radiopharm.,* 1996, vol. 11, 145-153
- **CARRIER et al.** *J. Immunol.,* 1992, vol. 148, 1176-1181
- **SU et al.** *Microbiol. Pathol.,* 1992, vol. 13, 465-476
- **CHABALGOITY et al.** *Infect. Immunol,* 1996, vol. 65, 2402-2412
- **SCHAFER et al.** *J. Immunol,* 1992, vol. 149, 53-59
- **PAN et al.** *Nature Med,* 1995, vol. 1, 471-477
- **SIZEMORE et al.** *Science,* 1995, vol. 270, 299-302
- **TURPEN et al.** *J. Virol. Methods,* 1993, vol. 42, 227-239
- **MACCARRONE et al.** *Biochem. Biophys. Res. Commun,* 1992, vol. 186, 1417-1422
- **JOHNSTON ; TANG.** *Genet. Eng. (NY),* 1993, vol. 15, 225-236
- **SAALBACH et al.** *Mol. Gen. Genet,* 1994, vol. 242, 226-236
- **PEREZ et al.** *Plant Mol. Biol,* 1989, vol. 13, 365-373
- **OLSZEWSKI et al.** *Nucleic Acids Res.,* 1988, vol. 16, 10765-10782
- **ROUSSELL et al.** *Mol. Gen. Genet.,* 1988, vol. 211, 202-209
- **BALAZS et al.** *Gene,* 1985, vol. 40, 343-348
- **MANNION-HENDERSON et al.** *Exp. Hematol,* 1995, vol. 23, 1628
- **SCHIFFMANN et al.** *Blood,* 1995, vol. 86, 1218
- **WILLIAMS.** *Bone Marrow Transplant,* 1990, vol. 5, 141
- **BOGGS.** *Int. J. Cell Cloning,* 1990, vol. 8, 80
- **MARTENSSON et al.** *Eur. J. Immunol.,* 1987, vol. 17, 1499
- **OKABE et al.** *Eur. J. Immunol.,* 1992, vol. 22, 37-43
- **BANERJI et al.** *Cell,* 1983, vol. 33, 729
- **WOLFF J. A et al.** *Science,* 1990, vol. 247, 1465-1468
- **SYKES et al.** *Human Gene Ther.,* 1994, vol. 5, 837-844
- **VILE et al.** *Cancer Res.,* 1993, vol. 53, 962-967
- **HENGGE.** *Nature Genet.,* 1995, vol. 10, 161-166
- **HICKMAN et al.** *Human Gene Therapy,* 1994, vol. 5, 1477-1483
- **MEYER et al.** *Gene Therapy,* 1995, vol. 2, 450-460
- **GARAPIN et al.** *Proc. Natl. Acad. Sci. U.S.A.,* 1981, vol. 78, 815-819
- **TALMADGE et al.** *Proc. Natl. Acad. Sci. U.S.A.,* 1980, vol. 77, 3369-3373
- **PAWALEK et al.** *Cancer Res.,* 1997, vol. 57, 4537-4544
- **BOLIVAR et al.** *Gene,* 1977, vol. 2, 95-113
- **KAHN et al.** *Methods Enzymol.,* 1979, vol. 68, 268-280
- **CHANG et al.** *J. Bacteriol,* 1978, vol. 134, 1141-1156
- **STOKER et al.** *Gene,* 1982, vol. 18, 335-341
- **UHLIN et al.** *Gene,* 1983, vol. 22, 255-265
- **JACKSON et al.** *Proc. Nat. Aca. Sci. U.S.A.,* 1972, vol. 69, 2904-2909
- **SCOTT.** *Microbial Reviews,* 1984, vol. 48, 1-23
- **ROSE et al.** Methods in Yeast Genetics. Cold Spring Harbor Laboratory Press, 1990
- **GIETZ ; SUGINO.** *Gene,* 1988, vol. 74, 527-534
- Extrachromsomoal cloning vectors of Saccharomyces cerevisiae. **SIKORSKI.** Plasmids, A Practical Approach. IRL Press, 1993
- Yeast Cloning Vectors and Genes. Current Protocols in Molecular Biology. 1994

- **HUBBARD et al.** *J. Clin. Invest,* 1989, vol. 84, 1349-1354
- **CLARKE ; WOODLAND.** *Rheumatol. Rehabil,* 1975, vol. 14, 47-49
- **O'SHEA et al.** *Science,* 1989, vol. 243, 538-542
- **HERMANSON.** Bioconjugate Techniques. Academic Press, 1997
- **PEARSON ; KEMP.** *Methods Enzymol.,* 1991, vol. 200, 62-81
- **DRAGO ; COLYER.** *J. Biol, Chem,* 1994, vol. 269, 25073-25077
- **HALTIWANGER et al.** *J. Biol. Chem.,* 1990, vol. 265, 2563-2568
- **CUBITT et al.** *Trends Biol. Sci.,* 1995, vol. 20, 448-455
- **CROWLEY et al.** *Cell,* 1993, vol. 73, 1101-1115
- **UCHIDA et al.** *Proc. Natl. Acad. Sci. USA,* 1993, vol. 90, 3481-3485